(19) Europäisches Patentamt
European Patent Office
Office européen des brevets

(11) EP 3 755 305 B1

(12) **EUROPEAN PATENT SPECIFICATION**

(45) Date of publication and mention of the grant of the patent:
**17.07.2024 Bulletin 2024/29**

(21) Application number: **19758048.3**

(22) Date of filing: **22.02.2019**

(51) International Patent Classification (IPC):
***A61K 9/00*** *(2006.01)* ***A61K 38/18*** *(2006.01)*
***A61K 38/36*** *(2006.01)* ***A61K 38/38*** *(2006.01)*
***A61K 38/39*** *(2006.01)* ***A61K 49/00*** *(2006.01)*
***A61L 27/38*** *(2006.01)*

(52) Cooperative Patent Classification (CPC):
**A61L 27/383; A61L 27/3834; B33Y 10/00; B33Y 70/10; C12N 5/0623; C12N 5/0696;**
C12N 2501/13; C12N 2513/00; C12N 2531/00; C12N 2533/40; C12N 2533/72; C12N 2537/10

(86) International application number:
**PCT/US2019/019283**

(87) International publication number:
**WO 2019/165300 (29.08.2019 Gazette 2019/35)**

(54) **MORPHOGENIC COMPOUND-RELEASING MICROSPHERES AND USE IN BIOINK**

EINE MORPHOGENE VERBINDUNG FREIGEBENDE MIKROKUGELN UND VERWENDUNG IN BIOTINTE

MICROSPHÈRES LIBÉRANT UN COMPOSÉ MORPHOGÉNIQUE ET UTILISATION DANS UNE BIOENCRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.02.2018 US 201862634029 P**

(43) Date of publication of application:
**30.12.2020 Bulletin 2020/53**

(73) Proprietor: **UVIC Industry Partnerships Inc.**
**Victoria, British Columbia V8W 2Y2 (CA)**

(72) Inventors:
- **AGBAY, Andrew**
  **Victoria, BC V8W 2Y2 (CA)**
- **DE LA VEGA, Laura**
  **Victoria, BC V8W 2Y2 (CA)**
- **WILLERTH, Stephanie, Michelle**
  **Victoria, BC V8W 2Y2 (CA)**

(74) Representative: **Symbiosis IP Limited**
**The Innovation Centre**
**217 Portobello**
**Sheffield, South Yorkshire S1 4DP (GB)**

(56) References cited:
**AU-A1- 2013 204 780 AU-A1- 2013 204 780**
**US-A1- 2003 175 410 US-A1- 2003 175 410**
**US-B2- 6 582 391**

- **QI GU ET AL: "3D Bioprinting Human Induced Pluripotent Stem Cell Constructs for In Situ Cell Proliferation and Successive Multilineage Differentiation", ADVANCED HEALTHCARE MATERIALS, vol. 6, no. 17, 24 May 2017 (2017-05-24), DE, XP055632123, ISSN: 2192-2640, DOI: 10.1002/adhm.201700175**
- **YONG BOK KIM ET AL: "Strategy to Achieve Highly Porous/Biocompatible Macroscale Cell Blocks, Using a Collagen/Genipin-bioink and an Optimal 3D Printing Process", APPLIED MATERIALS & INTERFACES, vol. 8, no. 47, 17 November 2016 (2016-11-17), US, pages 32230 - 32240, XP055632137, ISSN: 1944-8244, DOI: 10.1021/acsami.6b11669**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **GU ET AL.: "3D Bioprinting Human Induced Pluripotent Stem Cell Constructs for In Situ Cell Proliferation and Successive Multilineage Differentiation", ADVANCED HEALTHCARE MATERIALS, vol. 6, no. 17, 24 May 2017 (2017-05-24), pages 1 - 27, XP055632123**
- **KIM ET AL.: "Strategy to Achieve Highly Porous/Biocompatible Macroscale Cell Blocks, Using a Collagen/Genipin-bioink and an Optimal 3D Printing Process", ACS APPLIED MATERIALS & INTERFACES, vol. 8, no. 47, 30 November 2016 (2016-11-30), pages 32230 - 32240, XP055632137**
- **LAURA DE LA VEGA ET AL.: "3D Bioprinting Human Induced Pluripotent Stem Cell -Derived Neural Tissues Using a Novel Lab-on-a-Printer Technology", APPLIED SCIENCE, vol. 8, no. 12, 28 November 2018 (2018-11-28), pages 1 - 13, XP055632144**

## Description

### CROSS-REFERENCE TO RELATED APPLICATION

This application claims priority to U.S. provisional 62/634,029 filed on February 22, 2018.

### FIELD

This application provides bioink, methods of three-dimensional bioprinting, and methods of producing a functional tissue.

### BACKGROUND

The goal of tissue engineering (such as neural tissue) is to produce functional tissues that mimic the architecture and physiology of tissues on the body (*e.g.,* brain and spinal cord(SC)). These functional tissues can be used for disease modeling (*e.g.,* Alzheimer's or Parkinson's disease) and injuries (*e.g.,* SC injury or traumatic brain injury). Cell lines (*e.g.,* human induced pluripotent stem cells, hiPSCs) can be derived from patients suffering from diseases (*e.g.,* neurodegenerative diseases). These lines can be used to generate neural tissues in an *in vitro* setting, which can be used as a tool for drug screening. Currently, pre-clinical models consist of *in vivo* animal studies and cadaveric bodies; however, these approaches often do not often replicate and translate into clinical trials. Therefore, compositions and methods with long-lasting characteristics that can support differentiation from stem cells would be useful.

US 2003/175410 A1 provides methods and apparatuses for selectively depositing bio-ink solutions to build up a 3-D biomimetic scaffold structure. The therapeutic bioinks may be cells and may be formulated so as to provide controlled release over time. This may be accomplished by co-deposition with one or more biodegradable structural bio-inks and/or more functional bio-inks. Structural bio-inks include hydrogel solutions, PLA, PLGA.The therapeutic bio-inks (i.e. polypeptides, polynucleotides, small molecules, drugs, cells, neurotrophic factors may be mixed with or encapsulated into microspheres or nanospheres.

### SUMMARY

The invention is defined by the claims.

The bioink disclosed herein includes one or more cells, a carrier material, and microspheres.

The microspheres include one or more biodegradable polymers and one or more morphogenic compounds.

The one or more compounds release with degradation of the one or more biodegradable polymers.

In some examples, the cells of the bioink can include cell aggregates. Exemplary cells include stem cells (*e.g.,* induced pluripotent stem cells (iPSCs), such as human iPSCs, hiPSCs, or neural stem cells) or progenitor cells (*e.g.,* neural progenitor cells). In further examples, the carrier material of the bioink can include one or more polymers, crosslinkers, hydrogels, or any combination thereof. In some specific examples, the carrier material is a hydrogel, such as methacrylamide chitosan (MAC), fibrin, alginate, or any combination thereof.

Exemplary morphogenic compounds include small-molecule morphogen mimetics (*e.g.,* purmorphamine, puro, and guggulsterone (GS)), morphogens (*e.g.,* neurotrophic factors, NTFs, and insulin-like growth factor 1, IGF-1), or any combination thereof. In specific examples, the morphogenic compounds include one or more NTFs, such as a glial cell line-derived neurotrophic factor (GDNF), brain-derived neurotrophic factor (BDNF), or both. In additional examples, the biodegradable polymer of the microspheres is polylactic acid (PLA), polyglycolic acid (PLGA), polycaprolactone (PCL), or any combination thereof.

The methods disclosed herein can include ex-vivo three-dimensional bioprinting. In some examples, the methods include bioprinting (*e.g.,* using an inkjet bioprinter) at least one layer of the bioink disclosed herein onto a surface (such as a solid support). Additional embodiments of the methods disclosed herein include ex-vivo functional tissue (*e.g.,* neural tissue). In some embodiments, the methods include bioprinting (*e.g.,* using an inkjet bioprinter) at least one layer of bioink disclosed herein.

The ex-vivo methods of producing functional tissue include detecting a level of expression of one or more markers of differentiation or maturation expected in a functional tissue. In particular examples, the methods include detecting increased expression of one or more of ChAT, O4, NeuN, beta-tubulin III, HB9, synaptophysin, tyrosine hydroxylase, and neurites, compared with expression expected in a pluripotent stem cell. In other particular examples, the methods include detecting decreased expression of one or more of SSEA-4, Sox-2, Olig2, Isl-1, synapsin I, Oct3/4, c-myc, nestin, PAX6, and KLF4, compared with expression expected in a pluripotent stem cell.

Additional methods of bioprinting are further disclosed herein. In some examples, the methods include contacting at least one cell type (e.g., a cell type that forms aggregates; in specific examples, the cell type includes neuronal cells,

stem cells, stem cell-derived cells, human induced pluripotent stem cells (hiPSCs), and/or cancer cells) with a microsphere solution and a bioink solution (e.g., a bioink solution that includes fibrinogen, alginate, and/or genipin), thereby producing a bioink-cell-microsphere composition. The methods also include contacting the bioink-cell composition with a crosslinker (e.g., $CaCl_2$, chitosan, and/or thrombin), thereby producing a crosslinked bioink-cell composition. The methods can include bioprinting at least one layer of the crosslinked bioink-cell composition using a bioprinter. 26.

The foregoing and other objects and features of the disclosure will become more apparent from the following detailed description, which proceeds with reference to the accompanying figures.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

**FIGS. 1A-1D:** Characterization of the properties of purmorphamine loaded microspheres. **FIG. 1A:** Scanning electron microscopy images showing shape and size of puro-loaded microspheres which have an average size of 3.4 ± 1.17 μm, and **(FIG. 1B)** blank microspheres with an average size of 2.24 ± 2.04 μm. Scale bars are 10 μm. **FIG. 1C:** Density histogram showing the distribution of the measured microsphere diameters for purmorphamine-loaded microspheres. **FIG. 1D:** Quantification of purmorphamine release over 46 days - 91 % of the drug was released at the end of the time period.

**FIGS. 2A-2D:** Neurite growth extension after 35 days *in vitro* for neural aggregates (NAs). All groups were stained for β-tubulin III (βT-III neuronal marker, green. **(FIG. 2A)** puro/RA-loaded-microspheres, **(FIG. 2B)** NAs with unloaded microspheres, **(FIG. 2C)** negative control and **(FIG. 2D)** positive control. Images are made out of montages with Image J software from 36 images taken by an IncuCyte automated imaging machine. Scale bar represents 300 μm. Tracking culture morphologies over time for neurite extension and branching.

**FIGS. 3A-3C:** Quantitative analysis of neurite extension and branching after **(FIG. 3A)** 15, **(FIG. 3B)** 28 and **(FIG. 3C)** 35 days of differentiation *in vitro* for all groups. **FIGS. 3A-3C** show neurite length and neurite branch per cluster area. All groups (n=6) were normalized against the negative control group with the data being reported as the average with the error bars representing the standard deviation. * for $p<0.05$.

**FIGS. 4A-4S:** Analysis of neural cell cultures and engineered neural tissues after 15 days of differentiation *in vitro.* All groups were stained for β-tubulin III (βT-III neuronal marker, green), SSEA-4 (pluripotency marker, red), and DAPI (nuclear stain, blue). **FIGS. 4A-4D:** Cell aggregates combined with unloaded microspheres, **(FIGS. 4E-4H)** negative (untreated) control, **(FIGS. 4I-4L)** positive control (soluble puro/RA), and **(FIGS. 4M-4P)** engineered neural tissues containing puro/RA loaded-microspheres. **FIGS. 4Q-4S:** Quantification of cell marker expression with flow cytometry after 15 days of differentiation *in vitro.* Each condition was represented by a letter; Positive (P), Negative (N), aggregates combined with unloaded microspheres(B), and aggregates combined with puro/RA loaded microspheres (M). All groups were stained for Sox-2 (pluripotent cells and undifferentiated cells in the neural epithelium), SSEA-4, and β-t III. n=3 for all conditions, * for $p<0.05$ and ** for $p<0.001$.

**FIGS. 5A-5S:** Analysis of neural cell cultures and engineered neural tissues after 28 days of differentiation *in vitro.* All groups were stained for βT-III (green), NeuN (neuronal nuclei marker, red), and DAPI (blue). **FIGS. 5A-5D:** Cell aggregates combined with unloaded microspheres, **(FIGS. 5E-5H)** negative (untreated) control, **(FIGS. 5I-5L)** positive control (soluble puro/RA), and **(FIGS. 5M-5P)** engineered neural tissues containing puro/RA loaded-microspheres. **FIGS. 5Q-5S:** Quantification of cell marker expression with flow cytometry after 28 days of differentiation *in vitro.* All groups were stained for SSEA-4, βT-III, and Olig2 (oligodendrocyte transcription factor 2). n=3 for all conditions, * for $p<0.05$ and ** for $p<0.001$.

**FIGS. 6A-6S:** Analysis of neural cell cultures and engineered neural tissues after 35 days of differentiation *in vitro.* All groups were stained for βT-III (green), Syn1 (synapsin, red), and Olig2 (blue). **FIGS. 6A-6D:** Cell aggregates combined with unloaded microspheres, **(FIGS. 6E-6H)** negative (untreated) control, **(FIGS. 6I-6L)** positive control (soluble puro/RA), and **(FIGS. 6M-6P)** engineered neural tissues containing puro/RA loaded-microspheres. **FIGS. 6Q-6S:** Quantification of cell marker expression with flow cytometry after 35 days of differentiation *in vitro.* All groups were stained for Olig2, HB-9 (motor neuron) and ISL-1 (motor neuron). n=3 for all conditions, * for $p<0.05$ and ** for $p<0.001$.

**FIGS. 7A-7C:** Quantification of cell marker expression with flow cytometry after 60 days of differentiation *in vitro.* All groups were stained for **(FIG. 7A)** O4 (oligodendrocyte marker O4), **(FIG. 7B)** Sy38 (synaptophysin) and **(FIG. 7C)** ChAT (choline acetyltransferase). Data is reported as the mean with the error bars representing the standard deviation. n=3 for all condition. * for $p<0.05$ and ** for $p<0.001$.

**FIGS. 8A-8D:** characterization of the spontaneous activity of derived motor neurons on day 64. **FIG. 8A:** temporal raster plot of recorded spontaneous activity. **FIG. 8B:** representative raw voltage of spontaneous neuronal spikes and representative successive wave forms of spikes on electrode 23, overlaid over time. **FIG. 8C:** identification of

cellular network activity after electrical stimulation. **FIG. 8D:** neuronal mean firing rate over 10 days.

**FIGS. 9A-9P:** Phase contrast imaging (10x magnification) of embryoid bodies formation during 7 days in Aggrewell plates. **FIGS. 9A-9D:** Cell aggregates combined with unloaded microspheres, **(FIGS. 9E-9H)** negative (untreated) control, **(FIGS. 9I-9L)** positive control (soluble puro/RA), and **(FIGS. 9M-9P)** engineered neural tissues containing puro/RA loaded-microspheres. Images of day 7 (**FIGS. 9D, 9H, 9L,** and **9P**) were captured just after transferring to PLO-laminin plate.

**FIGS. 10A-10P:** Analysis of neural cell cultures and engineered neural tissues after 28 days of differentiation *in vitro.* All groups were stained for Olig2 (oligodendrocyte transcription factor 2, blue), β-tubulin III (βT-III neuronal marker, green), and HB9 (Motor neuron marker, red). **FIGS. 10A-10D:** Cell aggregates combined with unloaded microspheres, **(FIGS. 10E-10H)** negative (untreated) control, **(FIGS. 10I-10L)** positive control (soluble puro/RA), and **(FIGS. 10M-10P)** engineered neural tissues containing puro/RA loaded-microspheres.

**FIGS. 11A-11B:** Formation of cellular aggregates from pluripotent stem cells. (**FIG. 11A**) A cell suspension of pluripotent stem cells is centrifuged into aggregate-forming microwells. **(FIG. 11B)** A mixture of pluripotent stem cells and microspheres is centrifuged into aggregate-forming microwells. Aggregates are referred to as embryoid bodies (EBs) if they contain the ability to differentiate into all three germ layers and as NAs if they are specified to a neural fate. For example, NA formation is complete after 5 days of growth in NIM. Aggregates are then harvested and plated on cell culture-treated surfaces for subsequent differentiation and growth.

**FIGS. 12A-12E:** Characterization of PCL-based guggulsterone-encapsulated microspheres and unloaded microspheres. SEM images showing morphology of **(FIG. 12A)** dried unloaded microspheres at 2000X and **(FIG. 12B)** dried guggulsterone microspheres at 2200X. An accelerating voltage of 1.0 kV and working distances of 8.0 mm and 8.2 mm were used to capture the images. Probability density of microsphere diameters produced from the histogram of measured diameters for **(FIG. 12C)** unloaded and **(FIG. 12D)** guggulsterone microspheres with a sample size of n=2351 and n=1666, respectively. **FIG. 12E:** *In vitro* cumulative amount released (ng) and percent cumulative guggulsterone release from PCL-based microspheres over 44 days during the release study. The total amount of guggulsterone encapsulated in 10 mg of microspheres was ~ 2550 ng. Percent cumulative release was calculated from the total theoretical encapsulated guggulsterone per mg of microspheres in each sample replicate using the determined encapsulation efficiency value and the amount of guggulsterone leftover at each time point. Standard deviations are shown with a sample size of n=3. Diameter measurements were taken with ImageJ processing software and a kernel density curve was fitted to the probability density plot using the R statistical programming language.

**FIG. 13:** Guggulsterone remaining inside of PCL-based microspheres during the in vitro release study after predetermined time points over 44 days. Data points shown are guggulsterone amounts in μg for each sample vial (~10 mg of microspheres). Standard deviations are shown with a sample size n=3.

**FIGS. 14A-14P:** Bright field images at 10X magnification of hiPS cell aggregate formation with Aggrewell plates, attachment, and growth on PLO/laminin culture plates over 20 days. Aggregate formation of hiPSCs grown with **(FIG. 14A)** negative control media, **(FIG. 14B)** incorporated unloaded microspheres, **(FIG. 14C)** positive control soluble 2.5 μM guggulsterone added to the media and incorporated guggulsterone microspheres taken at **(FIGS. 14A-14D)** day 0 and **(FIGS. 14E-14H)** day 5. **FIGS. 14I-14L:** Growth and spreading of **(FIG. 14I)** a negative control aggregate, **(FIG. 14J)** an unloaded microsphere-incorporated aggregate, **(FIG. 14K)** a positive control aggregate, and **(FIG. 14L)** a guggulsterone microsphere-incorporated cell aggregate after 15 days. **FIGS. 14M-14P:** Growth and spreading of **(FIG. 14M)** a negative control aggregate, **(FIG. 14N)** an unloaded microsphere-incorporated aggregate, **(FIG. 14O)** a positive control aggregate, and **(FIG. 14P)** a guggulsterone microsphere-incorporated cell aggregate after 20 days. Cell aggregates were harvested and attached onto PLO/laminin culture plates on day 5. Microspheres are viewed as dark spheroids within the cell aggregates. Scale bar represents 300 μm.

**FIGS. 15A-15D:** Fluorescence images of cell aggregates for four separate treatments after 12 days *in vitro.* The aggregates were stained with βIII-tubulin (TUJ1) for immature neurons and display the extent of neurite branching from **(FIG. 15A)** a negative control aggregate, **(FIG. 15B)** an unloaded microsphere-incorporated aggregate, **(FIG. 15C)** guggulsterone microsphere-incorporated aggregate, and **(FIG. 15D)** a positive control aggregate. The montage images were compiled from 36 separate images taken by an IncuCyte automatic imaging machine and stitched together and cropped with ImageJ software. Scale bar represents 300 μm.

**FIGS. 16A-16D:** Fluorescence images of cell aggregates for four separate treatments after 20 days *in vitro.* The aggregates were stained with βIII-tubulin (TUJ1) for immature neurons and display the extent of neurite branching from **(FIG. 16A)** a negative control aggregate, **(FIG. 16B)** an unloaded microsphere-incorporated aggregate, **(FIG. 16C)** guggulsterone microsphere-incorporated aggregate, and **(FIG. 16D)** a positive control aggregate. The montage images were compiled from 36 separate images taken by an IncuCyte automatic imaging machine and stitched together and cropped with ImageJ software. Scale bar represents 300 μm.

**FIG. 17:** Fluorescence image of an NA containing guggulsterone microspheres after 12 days in vitro. The aggregate was stained with βIII-tubulin for immature neurons and shows neurite branching extending out from the aggregate.

Incorporated microspheres are viewed as dark spheroids inside of the aggregate. The image was compiled from 36 separate images taken by an IncuCyte automatic imaging machine and stitched together and cropped with ImageJ software. Scale bar represents 300 μm.

**FIG. 18:** Fluorescence image of a positive control NA after 12 days in vitro with soluble guggulsterone added to the media. The aggregate was stained with βIII-tubulin for immature neurons and shows neurite branching extending out from the aggregate. The image was compiled from 36 separate images taken by an IncuCyte automatic imaging machine and stitched together and cropped with ImageJ software. Scale bar represents 300 μm.

**FIG. 19:** Fluorescence image of a negative control NA after 12 days in vitro. The aggregate was stained with βIII-tubulin for immature neurons and shows neurite branching extending out from the aggregate. The image was compiled from 36 separate images taken by an IncuCyte automatic imaging machine and stitched together and cropped with ImageJ software. Scale bar represents 300 μm.

**FIG. 20:** Fluorescence image of unloaded microspheres-incorporated NA after 12 days in vitro. The aggregate was stained with βIII-tubulin for immature neurons and not showing extended neurite branching extending out from the aggregate. The image was compiled from 36 separate images taken by an IncuCyte automatic imaging machine and stitched together and cropped with ImageJ software. Scale bar represents 300 μm.

**FIG. 21:** Fluorescence image of an NAcontaining guggulsterone microspheres after 20 days in vitro. The aggregate was stained with βIII-tubulin for immature neurons and shows neurite branching extending out from the aggregate. Incorporated microspheres are viewed as dark spheroids inside of the aggregate. The image was compiled from 36 separate images taken by an IncuCyte automatic imaging machine and stitched together and cropped with ImageJ software. Scale bar represents 300 μm.

**FIG. 22:** Fluorescence image of a positive control NA after 20 days in vitro with soluble guggulsterone added to the media. The aggregate was stained with βIII-tubulin for immature neurons and shows neurite branching out from the aggregate. The image was compiled from 36 separate images taken by an IncuCyte automatic imaging machine and stitched together and cropped with ImageJ software. Scale bar represents 300 μm.

**FIG. 23:** Fluorescence image of a negative control NA after 20 days in vitro. The aggregate was stained with βIII-tubulin for immature neurons and shows neurite branching out from the aggregate. The image was compiled from 36 separate images taken by an IncuCyte automatic imaging machine and stitched together and cropped with ImageJ software. Scale bar represents 300 μm.

**FIG. 24:** Fluorescence image of unloaded microspheres- incorporated NA after 20 days in vitro. The aggregate was stained with βIII-tubulin for immature neurons and shows neurite branching out from the aggregate. The image was compiled from 36 separate images taken by an IncuCyte automatic imaging machine and stitched together and cropped with ImageJ software. Scale bar represents 300 μm.

**FIGS. 25A-25B:** Quantitative analysis of cell aggregate morphology for neurite length and branching after **(FIG. 25A)** 12 and **(FIG. 25B)** 20 days *in vitro.* The negative control cell aggregates (n=6) were used to normalize the data against the positive control (n=6), unloaded microsphere-incorporated (n=3), and guggulsterone microsphere-incorporated (n=5) aggregates. The positive control condition included soluble guggulsterone added to the media at 2.5 μM. The guggulsterone microsphere and unloaded microsphere conditions include incorporated microspheres (guggulsterone-loaded and unloaded without drug) within the cellular aggregates. The negative control conditions consisted of media without drug or microspheres added. Each replicate consisted of one aggregate per well. Neurite length and neurite branching were both calculated per cell cluster area. Cell morphology metrics were identified and quantified by an IncuCyte ZOOM® live-cell imaging system using the Neurotrack and Basic Analyzer cell masking software. Statistical analysis consisted of one-way analysis of variance with Tukey's post-hoc test. Standard deviations are shown with * indicating statistical significance at $p < 0.05$ for neurite length between conditions.

**FIGS. 26A-26C:** Flow cytometry analysis of βIII-tubulin (TUJ1), Olig2, and GFAP expression for human induced pluripotent stem cell aggregates after 20 days of culture. Differences in expression are shown among five different treatment conditions: negative control with no microspheres incorporated or soluble drug added, unloaded microspheres without drug encapsulated incorporated into aggregates, guggulsterone-encapsulated microspheres incorporated into aggregates, and two positive controls with soluble drug added at different concentrations (0.25 μM and 2.5μM). **FIG. 26A:** Percentage of cells expressing βIII-tubulin (TUJ1) (n=3 for each condition). **FIG. 26B:** Percentage of cells expressing Olig2 (n=3 for each condition). **FIG. 26C:** Percentage of cells expressing GFAP (n=3 for each condition). Each replicate consisted of one aggregate per well. One-way analysis of variance with Tukey's post-hoc test was performed for statistical comparisons. Standard deviations are shown with * indicating statistical significance at $p < 0.05$ from all conditions, # indicating statistical significance at $p < 0.05$ from the negative control, ¢ indicating statistical significance at $p < 0.05$ from the unloaded microsphere condition, ¥ indicating statistical significance at $p < 0.05$ from the guggulsterone microsphere condition, and Ø indicating statistical significance at $p < 0.05$ from the 2.5μM condition.

**FIGS. 27A-27B:** Day 14 samples with **(FIG. 27A)** 3D Fibrin cultures (not bioprinted) and **(FIG. 27B)** Laminin stained for Nestin (red), βT-III (green) and DAPI (blue).

**FIGS. 28A-28D:** Day 14 phase contrast images of neural progenitor cells 3D bioprinted in thinner scaffolds. Live/dead assay shown below **(FIGS. 28C-28D).** Scale bar represents 100 μm.

**FIGS. 29A-29C:** Day 21 phase contrast images of 3D Fibrin samples (not bioprinted) with **(FIG. 29A)** no compound **(FIG. 29B)** benzene, and **(FIG. 29C)** cadmium.

**FIGS. 30A-30D:** Scanning electron microscopy of puro-loaded microspheres **(FIG. 30A)** which have an average size of 3.4 ± 1.17 μm, Guggulsterone loaded microspheres **(FIG. 30B)** with an average size of 6.14± 9.09 μm, and blank microspheres with an average size of 2.24 ± 2.04 μm **(FIG. 30C),** RA loaded microspheres with an average diameter of 3.41± 1.60 μm **(FIG. 30D).** Scale bars on A and C represent 10 μm.

**FIGS. 31A-31H:** Immunostaining of hiPSC aggregates cultured in 3D fibrin scaffolds (not bioprinted) treated with soluble Puro/RA **(FIGS. 31A-31D),** and aggregates combined with Puro/RA microspheres **(FIGS. 31E-31H).** On day 15 samples were stained for SSEA-4 (green), βT-III (red) and DAPI (**FIGS. 31A** and **31E**). Day 28 (**FIGS. 31B** and **31F**) OLIG-2 (blue), HB9 (red), and NeuN (green). Day 35 differentiated cells were stained with HB9 (red), βT-III (green), and OLIG2 (blue) (**FIGS. 31C** and **31G**). Day 56 samples were stained for βT-III (green) and ChAT (blue) and synapsin I (red) (**FIGS. 31D** and **31H**).

**FIGS. 32A-32D:** Immunostaining of hiPSC aggregates cultured in 3D fibrin scaffolds (not bioprinted) treated with soluble Puro/GS **(FIGS. 32A-32B),** and aggregates combined with Puro/GS microspheres **(FIGS. 32C-32D).** On day 15 samples were stained for SSEA-4 (green), βT-III (red) and DAPI (**FIGS. 32A** and **32C**). Day 28 (**FIGS. 32B** and **32D**) OLIG-2 (blue), HB9 (red), and NeuN (green).

## DETAILED DESCRIPTION

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. For example, the term "comprising a cell" includes single or plural cells and is considered equivalent to the phrase "comprising at least one cell." The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements. Dates of GENBANK® Accession Nos. referred to herein are the sequences available at least as early as February 22, 2019.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

In order to facilitate review of the various embodiments of the disclosure, the following explanations of specific terms are provided.

**Aggregate:** Agglomerates of cells (such as viable cells) that can mature into functionality. The term aggregate includes set(s) of multiple aggregates that are in patterns (also known as an array), such as defined patterns, for example, to enable analyses (such as high-throughput analyses).

**Alginate:** Also known as alginic acid or algin, alginate is an anionic polysaccharide distributed in the cell walls of brown algae that forms a viscous gum upon binding with water. Alginate can be used in a hydrogel with the disclosed microparticles or in bulk gels, for example in combination with nerve growth factors. Alginate composites have been used in bone reconstruction and exhibit properties that encourage regeneration, such as improved porosity, cell proliferation, and mechanical strength.

**Beta-tubulin III (β3-tubulin):** Also known as tubulin beta-3, tubb3, tubb4, tubulin, and beta class III (*e.g.,* OMIM 602661), beta-tubulin III is predominantly expressed in neurons and testis cells and regulates ligand interactions as well as microtubule formation.

β3-tubulin sequences are publicly available. For example, GENBANK® Accession Nos. U47634.1, NM_139254.2, andNM_023279.2 disclose exemplary human, rat, and mouse β3-tubulin nucleotide sequences, respectively, and GENBANK® Accession Nos. AAC52035.1, AAM28438.1, andNP_075768.1 disclose exemplary human, rat, and mouse β3-tubulin protein sequences, respectively. One of ordinary skill in the art can identify additional β3-tubulin nucleic acid and protein sequences, including β3-tubulin variants that retain β3-tubulin biological activity (such as regulating ligand interactions and microtubule formation in neurons and testis cells).

**Biodegradable polymers:** Stable and durable polymers for use in a particular application, such as in a microsphere used bioink for bioprinting and/or generation of functional tissue. In some examples, they easily break down into natural, non-toxic byproducts such as gases ($CO_2$, $N_2$), water, biomass, and inorganic salts. Examples include polylactic acid (PLA), polyglycolic acid (PLGA), polycaprolactone (PCL), or any combination thereof.

**Bioink:** A material that can be combined with living cells as an "ink" in a bioprinting process. In some embodiments, the bioink (e.g., bioink solution) can include a hydrogel (*e.g.,* methacrylamide chitosan (MAC), fibrin, or alginate), extracellular matrix components, microcarriers, or combinations thereof. In particular embodiments, the bioink can include a hydrogel with crosslinking (*e.g.,* chemical, physical, or enzymatic crosslinking); exemplary crosslinking includes ionic crosslinking (*e.g.,* alginate in the presence of calcium chloride or calcium sulfate solutions), fibrin-based enzymatic crosslinking (*e.g.,* an enzymatic reaction between thrombin and fibrinogen), pH-mediated crosslinking (*e.g.,* chitosan), photo-mediated crosslinking (*e.g.,* methacrylamide), or any other type of crosslinking reaction (*e.g.,* genipin, Kwon et al., J Endod., 41(4):501-7,2015).

**Bioprinter:** A three dimensional (3D) printer that used 3D printing-like techniques (e.g., joining or solidifying material under computer control to create a three-dimensional object, such as adding material together, for example, layer-by-layer) to combine cells, growth factors, and/or biomaterials, such as to fabricate biomedical parts, for example, maximally imitating natural tissue. In some examples, a bioprinter uses a layer-by-layer method to deposit materials (e.g., bioink) to create tissue-like structures. Any type of bioprinter can be used. Non-exclusive examples of bioprinters include extrusion-based, a droplet-based (*e.g.,* inkjet), or a laser-based bioprinter.

**Bioprinting:** In bioprinting, cells and other biologics are deposited by a bioprinter in a spatially controlled pattern to fabricate living tissues and organs. In specific, non-limiting examples, the bioprinting can be extrusion-based, droplet-based (*e.g.,* inkjet), or laser-based.

**Brain-derived neurotrophic factor (BDNF):** BDNF (*e.g.,* OMIM 113505) aids in supporting survival of existing neurons and encouraging the growth and differentiation of new neurons and synapses. BDNF sequences are publicly available. For example, GENBANK® Accession Nos. X91251.1, M61175.1, and NM_007540.4 disclose exemplary human, rat, and mouse BDNF nucleotide sequences, respectively, and GENBANK® Accession Nos. CAA62632.1, NP_001257559.1, and AAH34862.1 disclose exemplary human, rat, and mouse BDNF protein sequences, respectively. One of ordinary skill in the art can identify additional BDNF nucleic acid and protein sequences, including BDNF variants that retain BDNF biological activity (such as encouraging the growth and differentiation of new neurons and synapses).

**Cell or cell type:** Morphologically and/or phenotypically distinct cells (*e.g.,* from the same species, such as cells with the same genotype). Any type of cell from any species is included (*e.g.*, cells from humans, animals, plants, fungi, bacteria, and/or archaea). Exemplary cell types (*e.g.,* from humans and/or animals) include neuronal cells, tumorigenic and/or cancer cells, stem cells (*e.g.,* induced pluripotent stem cells (iPSCs), such as human iPSCs, hiPSCs, or neural stem cells), progenitor cells (*e.g.,* neural progenitor cells), kidney cells, lung cells, liver cells, muscle cells, pancreatic cells, gastrointestinal cells, breast cells, ovarian cells, and/or testicular cells. In specific examples, cell type includes hiPSCs, neural stem cells, and/or neural progenitor cells.

In one example the cell is a cancer cell, such as one from a solid tumor, such as a cell from a breast carcinoma (*e.g.* lobular and duct carcinoma), sarcoma, carcinoma of the lung (*e.g.,* non-small cell carcinoma, large cell carcinoma, squamous carcinoma, and adenocarcinoma), mesothelioma of the lung, colorectal adenocarcinoma, stomach carcinoma, prostatic adenocarcinoma, ovarian carcinoma (such as serous cystadenocarcinoma and mucinous cystadenocarcinoma), ovarian germ cell tumor, testicular carcinoma and germ cell tumor, pancreatic adenocarcinoma, biliary adenocarcinoma, hepatocellular carcinoma, bladder carcinoma (including, for instance, transitional cell carcinoma, adenocarcinoma, and squamous carcinoma), renal cell adenocarcinoma, endometrial carcinomas (including, *e.g.*, adenocarcinoma and mixed Mullerian tumor (carcinosarcoma)), carcinoma of the endocervix, ectocervix, and vagina (such as adenocarcinoma and squamous carcinoma of each of same), tumors of the skin (*e.g.,* squamous cell carcinoma, basal cell carcinoma, malignant melanoma, skin appendage tumors, Kaposi sarcoma, cutaneous lymphoma, skin adnexal tumors and various types of sarcomas and Merkel cell carcinoma), esophageal carcinoma, carcinomas of the nasopharynx and oropharynx (including squamous carcinoma and adenocarcinomas of same), salivary gland carcinoma, brain and central nervous system tumors (including, for example, tumors of glial, neuronal, and meningeal origin), tumors of peripheral nerve, soft tissue sarcomas and sarcomas of bone and cartilage, and lymphatic tumors (including B-cell and T- cell malignant lymphoma).

In one example the cell is a cancer cell is from a liquid tumor, such as a lymphatic, white blood cell, or other type of leukemia. In a specific example, the cell is a cancer cell of the blood, such as a leukemia (for example, acute lymphoblastic leukemia (ALL), chronic lymphocytic leukemia (CLL), acute myelogenous leukemia (AML), chronic myelogenous leukemia (CML), hairy cell leukemia (HCL), T-cell prolymphocytic leukemia (T-PLL), large granular lymphocytic leukemia, and adult T-cell leukemia), lymphomas (such as Hodgkin's lymphoma and non-Hodgkin's lymphoma), and myeloma).

**Choline acetyltransferase (ChAT, CAT):** ChAT is found in in cholinergic neurons and is responsible for the synthesis of the neurotransmitter acetylcholine (*e.g.,* OMIM 118490). ChAT sequences are publicly available. For example, GENBANK® Accession Nos. S56138.1, NM_001170593.1, and NM_009891.2 disclose exemplary human, rat, and mouse ChAT nucleotide sequences, respectively, and GENBANK® Accession Nos. AAA14245.1, NP _001164064.1, and NP_034021.1 disclose exemplary human, rat, and mouse ChAT protein sequences, respectively. One of ordinary skill in the art can identify additional ChAT nucleic acid and protein sequences, including ChAT variants that retain ChAT biological activity (such as synthesizing acetylcholine).

**C-myc:** Also known as V-myc avian myelocytomatosis viral oncogene homolog (myc), oncogene myc, avian myelocytomatosis viral oncogene homolog, and protooncogene homologous to myelocytomatosis virus (*e.g.*, OMIM 190080), c-myc plays a role in cell cycle progression, apoptosis and cellular transformation.

C-myc sequences are publicly available. For example, GENBANK® Accession Nos. AH004538.1, Y00396.1, and AF076523.1 disclose exemplary human, rat, and mouse c-myc nucleotide sequences, respectively, and GENBANK® Accession Nos. AAB30748.1, AAT92512.1, and AAB59728.1 disclose exemplary human, rat, and mouse c-myc protein sequences, respectively. One of ordinary skill in the art can identify additional c-myc nucleic acid and protein sequences, including c-myc variants that retain c-myc biological activity (such as playing a role in cell cycle progression, apoptosis and cellular transformation).

**Crosslinked:** A composition containing intermolecular crosslinks and/or intramolecular crosslinks from covalent bond formation. Covalent bonding between two components capable of crosslinking may be direct, wherein an atom in one component is directly bound to an atom in the other component, or indirect, through a linking group. A crosslinked gel or polymer (such as a hydrogel) may also include intermolecular and/or intramolecular noncovalent bonds, such as hydrogen bonds and electrostatic (ionic) bonds. Exemplary reagents for crosslinking include genipin (*e.g.*, Kwon et al., J Endod., 41(4):501-7,2015),

**Differentiation:** The process whereby unspecialized cells (*e.g.,* embryonic cells) acquire specialized structural and/or functional features characteristic of mature cells. Similarly, "differentiate" refers to this process. Typically, during differentiation, cell structure alters and tissue-specific proteins and properties appear.

**Encapsulation efficiency (EE):** A measure of encapsulating (*i.e.,* containing or enclosing) something, such as a compound, compared with an initial amount. For example, encapsulation efficiency can include the percent of a compound encapsulated, such as in a microsphere, compared with an initial amount provided for encapsulation, such as the initial amount of compound provided for encapsulation in a microsphere. In some examples, encapsulation efficiency can be measured as follows.

$$\text{Encapsulation efficiency} = (\text{Compound}_{\text{encapsulated}}/\text{Compound}_{\text{provided}}) \times 100\%$$

**Fibrin:** Fibrin gel is a biodegradable polymer formed from fibrinogen and mimics the blood coagulation cascade, producing a fibrin clot. Upon exposure of polymerization sites, fibrin monomers self-assemble into insoluble fibrin gel, which is eventually degraded through plasmin-mediated fibrinolysis. Fibrin gel may facilitate degradation at a rate that can be controlled to correspond to tissue regeneration, yields nontoxic degradation products, and exhibits biocompatibility. A fibrin hydrogel morphology, mechanical properties, and stability may also be controlled by precursor concentration and ionic strength.

**Functional tissue:** Functional tissues can mimic the architecture and physiology of a natural tissue (*e.g.,* brain and spinal cord (SC)). Functional tissues can be used for disease modeling (*e.g.,* Alzheimer and Parkinson's disease) and injuries, such as spinal cord injury or traumatic brain injury. Tissue is a cellular organizational level between cells and a complete organ and is an ensemble of similar cells and extracellular matrix from the same origin that perform a specific function. Organs are formed by functional grouping of multiple tissues. Animal tissues are grouped into four types: connective, muscle, nervous, and epithelial. Collections of tissues joined in structural units to serve a common function compose organs. While all animals contain the four tissue types, the manifestation of these tissues can differ depending on the type of organism. For example, the origin of the cells comprising a particular tissue type may differ developmentally for different classifications of animals.

**Genipin:** A compound that can serve as a crosslinker (*e.g.,* in a hydrogel) and plays a role in cell differentiation (*e.g.,* Kwon et al., J Endod., 41(4):501-7, 2015).

**Glial cell line-derived neurotrophic factor (GDNF):** GDNF (*e.g.*, 600837) promotes the survival of neurons and differentiation of dopaminergic neurons (DNs). Includes GDNF nucleic acid molecules and proteins. GDNF sequences are publicly available. For example, GENBANK® Accession Nos. NM_000514.3, NM_019139.1, and U37459.1 disclose exemplary human, rat, and mouse GDNF nucleotide sequences, respectively, and GENBANK® Accession Nos. AAI28109.1, AAM18096.1, and AAB52953.1 disclose exemplary human, rat, and mouse GDNF protein sequences, respectively. One of ordinary skill in the art can identify additional GDNF nucleic acid and protein sequences, including GDNF variants that retain GDNF biological activity (such as promotes the survival of neurons and differentiation of DNs).

**Guggulsterone (GS):** A phytosteroid found in the resin of the guggul plant, *Commiphora mukul.* Guggulsterone effectively induces differentiation into dopaminergic neurons (DNs), which replace a complex series of molecules used for late stage differentiation.

**Hydrogel:** A solid, jelly-like material with a controlled cross-linked structure that does not exhibit flow in steady state. A hydrogel can be a water-swellable polymeric matrix that can absorb a substantial amount of water to form an elastic gel, wherein "matrices" are three-dimensional networks of macromolecules held together by covalent or noncovalent crosslinks. Upon placement in an aqueous environment, dry hydrogels swell to the extent allowed by the degree

of crosslinking (*e.g.,* using crosslinker reagents, such as genipin, *e.g.,* Kwon et al., J Endod., 41(4):501-7, 2015).

**Homeobox HB9 (HB9, HLXB9):** Also known as motor neuron and pancreas homeobox 1 (MNX1; *e.g.*, OMIM 142994), HB9 triggers motor neuron differentiation. Includes HB9 nucleic acid molecules and proteins. HB9 sequences are publicly available. For example, GENBANK® Accession Nos. NM_005515.3, NM_001271274.1, and NM_019944.2 disclose exemplary human, rat, and mouse HB9 nucleotide sequences, respectively, and GENBANK® Accession Nos. NP_001158727.1, NP_001258203.1, and NP_064328.2 disclose exemplary human, rat, and mouse HB9 protein sequences, respectively. One of ordinary skill in the art can identify additional HB9 nucleic acid and protein sequences, including HB9 variants that retain HB9 biological activity (such as triggering motor neuron differentiation).

**Insulin gene enhancer protein (Isl-1):** Also known as ISL lim homeobox 1 and ISL1 transcription factor, LIM/homeodomain (ISLET1; *e.g.,* OMIM 600366), Isl-1 plays a role in embryogenesis of the islets of Langerhans and is essential for neural tube motor neuron differentiation. Isl-1 sequences are publicly available. For example, GENBANK® Accession Nos. U07559.1, NM_017339.3, and NM_021459.4 disclose exemplary human, rat, and mouse Isl-1 nucleotide sequences, respectively, and GENBANK® Accession Nos. AAA81946.1, NP_059035.3, andNP_067434.3 disclose exemplary human, rat, and mouse Isl-1 protein sequences, respectively. One of ordinary skill in the art can identify additional Isl-1 nucleic acid and protein sequences, including Isl-1 variants that retain Isl-1 biological activity (such as playing a role in neural tube differentiation).

**Insulin-like growth factor 1 (IGF-1):** An endocrine hormone that regulates cell growth and development, especially in nerve cells, as well as DNA synthesis. IGF-1 sequences are publicly available. For example, GENBANK® Accession Nos. NM_001111283.2, X06043.1, and NM_010512.5 disclose exemplary human, rat, and mouse IGF-1 nucleotide sequences, respectively, and GENBANK® Accession Nos. CAA40092.1, P08025.3, andNP_034642.2 disclose exemplary human, rat, and mouse IGF-1 protein sequences, respectively. One of ordinary skill in the art can identify additional IGF-1 nucleic acid and protein sequences, including IGF-1 variants that retain IGF-1 biological activity (such as regulating cell growth and development).

**Kruppel-like factor 4 (KLF4):** Also known as gut-enriched Krüppel-like factor (GKLF) and endothelial kruppel-like zinc finger protein (EZF) (*e.g.,* OMIM 602253), KLF4 is involved in regulating proliferation, differentiation, apoptosis, and somatic cell reprogramming and is considered a good indicator of stem-like capacity.

KLF4 sequences are publicly available. For example, GENBANK® Accession Nos. NM_001314052.1, NM_053713.1, andNM_010637.3 disclose exemplary human, rat, and mouse KLF4 nucleotide sequences, respectively, and GENBANK® Accession Nos. AAH30811.1, NP_446165.1, and NP_034767.2 disclose exemplary human, rat, and mouse KLF4 protein sequences, respectively. One of ordinary skill in the art can identify additional KLF4 nucleic acid and protein sequences, including KLF4 variants that retain KLF4 biological activity (such as regulating proliferation, differentiation, apoptosis, and somatic cell reprogramming and acting as an indicator of stem-like capacity).

**Markers of differentiation/maturation:** Markers of differentiation and/or maturation include physical indicia of a growth or development stage. The markers can include expression levels of small molecules, lipids, proteins, peptides, or nucleic acids, such as expression levels of nestin, beta-tubulin III, stage-specific embryonic antigen-4 (SSEA-4), NeuN, oligodendrocyte transcription factor (OLIG2), homeobox HB9 (HB9), paired box protein (PAX6), synapsin I, SRY-box 2 (SOX-2), insulin gene enhancer protein (ISL-1), anti-oligodendrocyte O4 (O4), choline acetyltransferase (ChAT), synaptophysin, Oct3/4, c-Myc, kruppel-like factor 4 (KLF4), or tyrosine hydroxylase. Markers can also include physiological characteristics, such as cell function, localization, or morphology (*e.g.,* the presence of action potentials and neurites in neural cells).

**Microspheres:** Micro-scale particles (*e.g.,* particles with a diameter of about 1 μm to about 1000 μm) that can serve as drug delivery vehicles, releasing a compound (*e.g.,* a drug) encapsulated therein over extended periods of time. Altering the size, density, and polymer of the microsphere can influence the release rate of a drug. Many types of biodegradable polymers can be used due to their biocompatibility in biological systems. Examples of such biodegradable polymers include polylactic acid (PLA), polyglycolic acid (PLGA), and poly-ε-caprolactone (PCL). In some examples, the microspheres can be combined into a microsphere solution, such as a single or multiphase solution (e.g., a biphasic solution, such as an oil-in-water emulsion).

**Morphogen/Morphogenic compound:** A substance with a non-uniform distribution that regulates the pattern of tissue development in the process of morphogenesis or pattern formation in developmental biology, establishing positions of specialized cell types within a tissue. A morphogen is a molecule, such as a signaling molecule (*e.g.,* a molecule involved in transmitting information between cells), that acts directly on cells to produce specific cellular responses depending on its local concentration. Exemplary morphogens found in animals (*e.g.,* mammals) include retinoic acid (RA) and proteins or peptides, such as neurotrophic factors (NTFs; *e.g.,* glial cell line-derived neurotrophic factor, GDNF; brain-derived neurotrophic factor, BDNF; fibroblast growth factor 8, FGF8; and/or bone morphogenetic protein, BMP9), insulin-like growth factor 1 (IGF-1), sonic hedgehog (SHH), transforming growth factor beta (TGF-β)/bone morphogenic protein (BMP), and Wnt/beta-catenin. A **morphogenic compound** is an agent that is a morphogen or exhibits a morphogen-like response (*e.g.,,* a morphogen mimetic, such as a small-molecule morphogen mimetic). Exemplary small-molecule morphogen mimetics include purmorphamine (puro), guggulsterone (GS), and genipin.

**Nestin:** Also known as NES, nestin is predominantly expressed in central nervous system (CNS) stem cells in the neural tube. Nestin plays a role in regulating cell morphology of mitotically active cells and is downregulated upon terminal neural differentiation.

Includes nestin nucleic acid molecules and proteins. Nestin sequences are publicly available. For example, GENBANK® Accession Nos. NM_006617.1, AF004334.1, and NM_016701.3 disclose exemplary human, rat, and mouse nestin nucleotide sequences, respectively, and GENBANK® Accession Nos. NP_006608.1, AAN33053.1, and NP_057910.3 disclose exemplary human, rat, and mouse nestin protein sequences, respectively. One of ordinary skill in the art can identify additional nestin nucleic acid and protein sequences, including nestin variants that retain nestin biological activity (such as regulating cell morphology of mitotically active cells).

**Neuronal nuclei antigen (NeuN):** Also known as RNA-binding protein FOX1, C. elegans, homolog of, 3; RBFOX3 FOX3; hexaribonucleotide-binding protein 3; HRNBP3 (e.g., OMIM 616999), NeuN is involved in neuron-specific alternative splicing of pre-mRNAs and, thus can be used to detect postmitotic neurons.

Includes NeuN nucleic acid molecules and proteins. NeuN sequences are publicly available. For example, GENBANK® Accession Nos. NM_001134498.2, NM_001039167.1, and NM_001039167.1 disclose exemplary human, rat, and mouse NeuN nucleotide sequences, respectively, and GENBANK® Accession Nos. NP_001337380.1, NP_001127970.1, and NP_001034256.1 disclose exemplary human, rat, and mouse NeuN protein sequences, respectively. One of ordinary skill in the art can identify additional NeuN nucleic acid and protein sequences, including NeuN variants that retain NeuN biological activity (such as playing a role in neuron-specific alternative splicing of pre-mRNAs and, thus use in detecting postmitotic neurons).

**Neurotrophic factors (NTFs):** A biomolecule (*e.g.*, peptides or small proteins) that supports developing and mature neuronal growth, survival, and differentiation. Exemplary neurotrophic factors include glial cell line-derived neurotrophic factor (GDNF) and brain-derived neurotrophic factor (BDNF).

**Octamer-binding transcription factor 3/4 (OCT3/4):** Also known as POU domain, class 5, transcription factor 1 (POU5F1; *e.g.,* OMIM 164177), Oct3/4 is involved in self-renewal of undifferentiated embryonic stem cells. Oct3/4 can be both a marker for undifferentiated cells and used to create induced pluripotent stem cells.

Oct3/4 sequences are publicly available. For example, GENBANK® Accession Nos. KT584545.1, NM_001009178.2, and NM_013633.3 disclose exemplary human, rat, and mouse Oct3/4 nucleotide sequences, respectively, and GENBANK® Accession Nos. AQY77032.1, CAE84020.1, and NP_038661.2 disclose exemplary human, rat, and mouse Oct3/4 protein sequences, respectively. One of ordinary skill in the art can identify additional Oct3/4 nucleic acid and protein sequences, including Oct3/4 variants that retain Oct3/4 biological activity (such as playing a role in stem cell self-renewal and inducing pluripotent stem cells).

**Oligodendrocyte O4 (O4):** Also known as forkhead box O4 (FOXO4), myeloid/lymphoid or mixed lineage leukemia translocated to 7 (MLLT7), mixed lineage leukemia translocated to 7, ALL1-fused gene from x chromosome (AFX1; *e.g.*, OMIM 300033), O4 is involved in regulating oxidative stress signaling, longevity, insulin signaling, cell cycle progression, and apoptosis and is necessary for differentiation of neural cells.

O4 sequences are publicly available. For example, GENBANK® Accession Nos. NM_005938.3, NM_001106943.1, and NM_018789.2 disclose exemplary human, rat, and mouse O4 nucleotide sequences, respectively, and GENBANK® Accession Nos. AAI06762.1, NP_001100413.1, and NP_061259.1 disclose exemplary human, rat, and mouse O4 protein sequences, respectively. One of ordinary skill in the art can identify additional O4 nucleic acid and protein sequences, including O4 variants that retain O4 biological activity (such as aiding in differentiation of neural cells).

**Oligodendrocyte transcription factor (Olig2):** Also known as protein kinase C binding protein 2 (PRKCBP2) and basic helix-loop-helix protein, class B, 1 (BHLHB1; *e.g.,* OMIM 606386), Olig2 acts as an anti-neurigenic and a neurigenic factor depending on the stage of development, determines motor neuron and oligodendrocyte differentiation, and plays a role in sustaining replication during early development.

Olig2 sequences are publicly available. For example, GENBANK® Accession Nos. NM_005806.3, NM_001100557.1, and NM_016967.2 disclose exemplary human, rat, and mouse Olig2 nucleotide sequences, respectively, and GENBANK® Accession Nos. AAH36245.1, NP_001094027.1, andNP_058663.2 disclose exemplary human, rat, and mouse Olig2 protein sequences, respectively. One of ordinary skill in the art can identify additional Olig2 nucleic acid and protein sequences, including Olig2 variants that retain Olig2 biological activity (such as regulating differentiation and replication).

**Paired box gene 6 (Pax6):** Pax6 aids in regulating differentiation and proliferation during neurogenesis and oculogenesis (*e.g.,* OMIM 607108). Pax6 sequences are publicly available. For example, GENBANK® Accession Nos. NM_000280.4, NM_013001.2, and AJ292077.1 disclose exemplary human, rat, and mouse Pax6 nucleotide sequences, respectively, and GENBANK® Accession Nos. NP_001245394.1, NP_037133.1, and NP_001231129.1 disclose exemplary human, rat, and mouse Pax6 protein sequences, respectively. One of ordinary skill in the art can identify additional Pax6 nucleic acid and protein sequences, including Pax6 variants that retain Pax6 biological activity (such as regulating differentiation and proliferation).

**Purmorphamine (Puro):** A small molecule morphogen that activates the hedgehog pathway, which is involved in stem cell renewal. Purmorphamine can promote differentiation of ventral spinal progenitors and motor neurons from human pluripotent stem cells and of osteoblasts from human and mouse mesenchymal cells but can also inhibit differentiation and maturation of adipocytes from human mesenchymal cells.

**Retinoic acid (RA):** A vitamin A metabolite necessary for growth and development that can act as a morphogen and plays a role in cell differentiation. For example, RA induces neural differentiation.

**Scaffold:** A structure that typically includes a biocompatible material, which provides a surface suitable for adherence and proliferation of cells, and also provides stability and support. A scaffold can be in a particular shape or form so as to influence or delimit a three-dimensional shape or form assumed by a population of proliferating cells. Such shapes or forms include, but are not limited to, films (*e.g.* a form with two-dimensions substantially greater than the third dimension), ribbons, cords, sheets, flat discs, cylinders, spheres, and amorphous shapes.

**Stage-specific embryonic antigen-4 (SSEA-4):** SSEA-4 is a glycosphingolipid that can indicate the presence of immature human stem cells during differentiation (*e.g.*, neuronal differentiation; *see* Isacson et al., Stem Cells, 25(9):2257-2268,2007).

**SRY-box 2 (Sox2):** Also known as Sry-related HMG-box gene 2 (*e.g.,* OMIM 184429), Sox2 is necessary for maintaining self-renewal of undifferentiated embryonic stem cells. Sox2 sequences are publicly available. For example, GENBANK® Accession Nos. NM_003106.3, NM_001109181.1, and NM_011443.4 disclose exemplary human, rat, and mouse Sox2 nucleotide sequences, respectively, and GENBANK® Accession Nos. NP_003097.1, NP_003097.1, and AAH57574.1 disclose exemplary human, rat, and mouse Sox2 protein sequences, respectively. One of ordinary skill in the art can identify additional Sox2 nucleic acid and protein sequences, including Sox2 variants that retain Sox2 biological activity (such as maintaining pluripotency in undifferentiated stem cells).

**Stem cell:** An undifferentiated cell of a multicellular organism that can indefinitely produce more cells of the same type (*i.e.*, self-renewal) and from which other types of cells arise by differentiation. Mammalian stem cells include embryonic and somatic stem cells. Exemplary stem cells include neural stem cells and induced pluripotent stem cells (iPSCs; *e.g.*, human iPSCs, hiPSCs).

**Synapsin I (Syn1):** Also known as brain protein 4.1 (*e.g.,* OMIM 313440), Syn1 aids in regulating neuronal development, axonogenesis, and synaptogenesis. Syn1 sequences are publicly available. For example, GENBANK® Accession Nos. M55301.1, X04655.1, and BC022954.1 disclose exemplary human, rat, and mouse Syn1 nucleotide sequences, respectively, and GENBANK® Accession Nos. AAC41930.1, NP_062006.1, and AAH22954.1 disclose exemplary human, rat, and mouse Syn1 protein sequences, respectively. One of ordinary skill in the art can identify additional Syn1 nucleic acid and protein sequences, including Syn1 variants that retain Syn1 biological activity (such as regulating neuronal development).

**Synaptophysin (SYP):** Also known as major synaptic vesicle protein p38 (*e.g.,* OMIM 313475), SYP interacts with synaptic vesicle proteins and is present in neuroendocrine cells and neurons involved in synaptic transmission. Thus, SYP can be used as an immunostain for quantifying synapses.

SYP sequences are publicly available. For example, GENBANK® Accession Nos. NM_003179.2, NM_012664.3, and NM_009305.2 disclose exemplary human, rat, and mouse SYP nucleotide sequences, respectively, and GENBANK® Accession Nos. NP_003170.1, AAH99798.1, andNP_033331.2 disclose exemplary human, rat, and mouse SYP protein sequences, respectively. One of ordinary skill in the art can identify additional SYP nucleic acid and protein sequences, including SYP variants that retain SYP biological activity (such as interacting with synaptic vesicle proteins).

**Thrombin:** Also known as coagulation factor II (F2) and factor II (*e.g.,* OMIM No. 176930), thrombin is a serine protease that converts fibrinogen into fibrin for blood clot formation. Thrombin sequences are publicly available. For example, GENBANK® Accession Nos. NM_000506.4, NM_022924.2, and NM_010168.3 disclose exemplary human, rat, and mouse thrombin nucleotide sequences, respectively, and GENBANK® Accession Nos. NP_000497.1, NP_075213.2, and NP_034298.1 disclose exemplary human, rat, and mouse thrombin protein sequences, respectively. One of ordinary skill in the art can identify additional thrombin nucleic acid and protein sequences, including thrombin variants that retain thrombin biological activity (such as converting fibrinogen into fibrin).

**Tyrosine hydroxylase (TH):** Also known as tyrosine 3-monooxygenase, TH catalyzes the conversion of L-tyrosine to L-3,4-dihydroxyphenylalanine (L-DOPA), TH is present in the central nervous system (CNS), peripheral sympathetic neurons and the adrenal medulla (*e.g.*, OMIM 191290).

TH sequences are publicly available. For example, GENBANK® Accession Nos. BC104967.1, M10244.1, and M69200.1 disclose exemplary human, rat, and mouse TH nucleotide sequences, respectively, and GENBANK® Accession Nos. AAI43612.1, AAA42258.1, and AAA40434.1 disclose exemplary human, rat, and mouse TH protein sequences, respectively. One of ordinary skill in the art can identify additional TH nucleic acid and protein sequences, including TH variants that retain TH biological activity (such as catalyzing the conversion of L-tyrosine to L-DOPA).

## Overview

Three-dimensional (3D) bioprinting can provide more accurate and detailed neural tissues than those presently available. In combination with stem cells, scaffold architecture and geometry can provide an ideal support for such tissues to mature. Additionally, when stem cells are in a favorable environment, similar to the conditions in the extracellular matrix, and are exposed to specific morphogenic compounds, such as small-molecule morphogen mimetics (*e.g.,* purmorphamine, puro, and guggulsterone (GS)) and/or morphogens (*e.g.,* neurotrophic factors (NTFs) and insulin-like growth factor 1 (IGF-1)), these cells tend to differentiate and mature into the desired tissues.

## Bioink

The bioink of the present disclosure includes one or more cells, one or more carrier materials, and discrete microspheres. In the microspheres disclosed herein, one or more morphogenic compounds and biodegradable polymers are included.

### *A. Cells*

The cells of the bioink disclosed herein can be any type of cell in any configuration. In some examples, the cells are single, individual, separated cells. In some examples, the cells are aggregates, such as clusters or clumps of two or more cells attached to, adhered to, or otherwise in contact with one another, such as an aggregate often or more cells, 100 or more cells, or 1000 or more cells. In some examples, the cell aggregate is spheroid, strand, or in pellet form.

In some embodiments, cells of the bioink can be undifferentiated cells, such as stem cells or progenitor cells. Stem cells can self-renew through mitotic cell division and have the potential to differentiate into a tissue or an organ. Self-renewing cells can divide and generate at least one daughter cell with the self-renewing characteristics of the parent cell. The second daughter cell may commit to a particular differentiation pathway. The stem cells can be from any type of organism (*e.g.,* mammalian stem cells, such as human or mouse stem cells).

The stem cells can further exhibit any type of potency. For example, stem cells can be pluripotent (*i.e.*, pluripotent stem cells, PCSs). PSCs are characterized by the ability to become any somatic cell-type (*i.e.*, pluripotency) and the ability to continuously self-renew (*i.e.,* immortality). Pluripotent stem cells can differentiate into cell types of all three germ layers (*e.g.,* ectodermal, mesodermal, and endodermal cell types) and express one or more markers of embryonic stem cells (*e.g.,* SSEA-4, SOX-2, OLIG2, ISL-1, synapsin I, Oct3/4, c-Myc, nestin, PAX6, or KLF4) but cannot form an embryo or extraembryonic membranes (*i.e.,* pluripotent stem cells are not totipotent).

Exemplary pluripotent stem cells include induced pluripotent stem cells (iPSCs; *e.g.,* human iPSCs, hiPSCs) generated by reprogramming a somatic cell through expressing or inducing expression of a combination of factors (*e.g.,* morphogens). iPSCs can be generated using fetal, postnatal, newborn, juvenile, or adult somatic cells, and iPSCs exhibit distinctive morphologies as well as similar properties as embryonic stem cells, such as pluripotency and self-renewal. Through iPSCs, patient-specific treatments can be developed, as somatic cells from patients can be reprogrammed into PSCs, which can be used replace a desired cell type (*e.g.,* dopaminergic neurons, DNs).

In some examples, the PCSs include induced pluripotent stem cells (iPSCs). In particular examples, the stem cells are hiPSCs. hiPSCs can generate any type of cell found in the body. Adult human somatic cells can be reprogrammed back to a pluripotent state (*i.e.,* hiPSCs) by expressing a specific set of transcription factors. hiPSCs can self-renew and differentiate into all three germ layers, similar to human embryonic stem cells (hESCs).

In other examples, the cells are non-pluripotent, such as multipotent stem cells (*e.g.,* neural stem cells, NSCs) or progenitor cells (*e.g.,* neural progenitor cells, NPCs; oligodendrocyte progenitor cells, OPCs). A non-pluripotent cell can be a cell of lesser potency to self-renew and differentiate than a pluripotent stem cell. Cells of lesser potency can be, but are not limited to adult stem cells, tissue specific progenitor cells, primary or secondary cells.

In some examples, the non-pluripotent cells are NSCs. Neural stem cells are self-renewing, multipotent cells that generate the neurons and glia of the nervous system of all animals during embryonic development. In some examples, NSCs are derived from iPSCs (*e.g.,* hiPSCs), such as hiPSC-derived NSCs. Neural stem cells can persist in the adult vertebrate brain and continue to produce neurons throughout life and primarily differentiate into neurons, astrocytes, and oligodendrocytes.

In still further examples, the bioink can include one or more cells in any configuration. Exemplary configurations include single cell suspension and cell aggregates (*e.g.,* embryoid bodies, such as using iPSCs). Other reagents can be included in the bioink, such as protease inhibitors, for example, that support cell differentiation (e.g., aprotinin, bestatin, e-64, leupeptin, pepstatin A; in specific examples, aprotonin can be included).

*B. Carrier material*

In further embodiments, the bioink includes a carrier material, such as one or more polymers, crosslinkers, hydrogels, or any combination thereof. In specific, non-limiting embodiments, the carrier material is a hydrogel. In some examples, the hydrogel also includes crosslinkers. Exemplary materials include methacrylamide chitosan (MAC), fibrin, alginate, collagen, and laminin. In some embodiments, the carrier material includes methacrylamide chitosan (MAC). For example, MAC can deliver adult NSC/progenitor cells to engineer neural tissues with improved neuronal regeneration. In other embodiments, the carrier material can include collagen, laminin, or a combination thereof. For example, the carrier material can include a collagen and laminin hydrogel.

*C. Microspheres*

In some embodiments, the bioink includes discrete microspheres that include one or more morphogenic compounds and biodegradable polymers. Any micro-scale particle can be used that provides a drug delivery vehicle by releasing encapsulated drug over extended periods of time. In some examples, the microspheres disclosed herein can facilitate temporally controlled, localized drug delivery. In some embodiments, the microspheres have a diameter of at least 1 μm, at least 10 μm, or at least 100 μm, such as 1 μm - 1000 μm. In some embodiments, the microsphere diameter is about 1-2 μm, 2-3 μm, 3-4 μm, 4-5 μm, 5-6 μm, 6-7 μm, 7-8 μm, 9-10 μm, 1-5 μm, 5-10 μm, 10-15 μm, 15-20 μm, 20-30 μm, 30-40 μm, 40-50 μm, 50-60 μm, 60-70 μm, 70-80 μm, 80-90 μm, 90-100 μm, 100-150 μm, 150-200 μm, 200-300 μm, 300-400 μm, 400-600 μm, 600-800 μm, or 800-1000 μm, or about 1-5 μm, 5-10 μm, 10-15 μm, or 400-600 μm. In other embodiments, the microsphere diameter is at least about 1 μm, 2 μm, 3 μm, 4 μm, 5 μm, 6 μm, 7 μm, 8 μm, 9 μm, 10 μm, 15 μm, 20 μm, 30 μm, 40 μm, 50 μm, 60 μm, 70 μm, 80 μm, 90 μm, 100 μm, 150 μm, 200 μm, 300 μm, 400 μm, 600 μm, 800 μm, or 1000 μm, or at least about 2 μm, 4 μm, 6 μm, 9 μm, 14 μm, or 400 μm.

i. Biodegradable polymers

In further embodiments, the discrete microspheres can include more biodegradable polymers. Any polymer can be used that breaks down into non-toxic, natural, non-toxic byproducts, such as gases ($CO_2$ and $N_2$), water, biomass, and inorganic salts. Exemplary biodegradable polymers include polylactic acid (PLA), polyglycolic acid (PLGA), polycaprolactone (PCL), or any combination thereof.

In some examples, the biodegradable polymer includes PCL. Polycaprolactone is degraded by hydrolysis of its ester linkages in physiological conditions (such as in the human body). PCL is a biodegradable polyester with the following chemical formula and structure: ($C_6H_{10}O_2$).

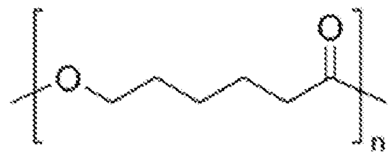

In other examples, the biodegradable polymer includes PLA. PLA can be by hydrolysis of its ester linkages. PLA is a biodegradable and bioactive thermoplastic aliphatic polyester with the following chemical formula and structure: $(C_3H_4O_2)_n$

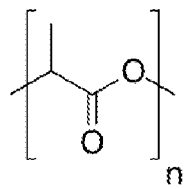

In still further examples, the biodegradable polymer includes PLGA. PLGA is degraded through hydrolysis of its ester linkages in an aqueous environment. PLGA is a biodegradable, thermoplastic polymer with the following chemical formula and structure: $(C_2H_2O_2)_n$

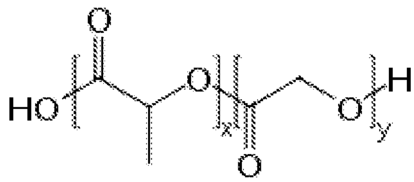

where x denotes the number of lactic acid units, and y denotes the number of glycolic acid units.

ii. Microsphere compounds

The disclosed microsphere release one or more morphogenic compounds as the one or more biodegradable polymers break down. Morphogenic compounds include morphogens and/or small-molecule morphogen mimetics.

In some embodiments, one or more compounds can regulate cell development or movement (e.g., morphogenic compounds, such as small-molecule morphogen mimetics and/or morphogens). In other embodiments, the amount of compound in the microsphere can be at least 0.05 μg compound/mg microsphere, at least 0.15 μg compound/mg microsphere, at least 0.5 μg compound/mg microsphere, at least 1 μg compound/mg microsphere, at least 2 μg compound/mg microsphere at least 3 μg compound/mg microsphere, at least 4 μg compound/mg microsphere, at least 5 μg compound/mg microsphere, at least 10 μg compound/mg microsphere, at least 15 μg compound/mg microsphere, at least 20 μg compound/mg microsphere, at least 25 μg compound/mg microsphere, at least 30 μg compound/mg microsphere, at least 40 μg compound/mg microsphere, or at least 50 μg compound/mg microsphere, such as about 0.05-0.1 μg compound/mg microsphere, 0.1-0.15 μg compound/mg microsphere, 0.15-0.2 μg compound/mg microsphere, 0.2-0.25 μg compound/mg microsphere, 0.25-0.3 μg compound/mg microsphere, 0.3-0.35 μg compound/mg microsphere, 0.35-0.4 μg compound/mg microsphere, 0.4-0.45 μg compound/mg microsphere, 0.45-0.5 μg compound/mg microsphere, 0.5-0.55 μg compound/mg microsphere, 0.55-0.6 μg compound/mg microsphere, 0.6-0.65 μg compound/mg microsphere, 0.65-0.7 μg compound/mg microsphere, 0.7-0.75 μg compound/mg microsphere, 0.75-0.8 μg compound/mg microsphere 0.8-0.85 μg compound/mg microsphere, 0.85-0.9 μg compound/mg microsphere, 0.9-0.95 μg compound/mg microsphere, 0.95-1 μg compound/mg microsphere, 1-2 μg compound/mg microsphere, 2-3 μg compound/mg microsphere,3-4 μg compound/mg microsphere, 4-5 μg compound/mg microsphere, 5-10 μg compound/mg microsphere, 10-15 μg compound/mg microsphere, 15-20 μg compound/mg microsphere, 20-30 μg compound/mg microsphere, 30-40 μg compound/mg microsphere, 40-50 μg compound/mg microsphere, or 50-75 μg compound/mg microsphere, or about 0.25, 0.27, 0.78, 2.4, or 18 μg compound/mg microsphere.

The microspheres described herein can release any amount of a morphogenic compound, such as a small-molecule morphogen mimetic and/or a morphogen. In some embodiments, the microspheres can release at least about 1%, 5%, 10%, 15%, 20%, 25%, 30%, 35%, 40%, 45%, 50%, 55%, 60%, 65%, 70%, 75%, 80%, 85%, 90%, 95%, or 100%, or about 1-5%, 5-10%, 10-15%, 15-20%, 20-25%, 25-30%, 30-35%, 35-40%, 40-45%, 45-50%, 50-55%, 55-60%, 60-65%, 65-70%, 70-75%, 75-80%, 80-85%, 85-90%, 90-95%, or 95-100% of a morphogenic compound, such as over at least about 1 day, 2 days, 5 days, 10 days, 15 days, 20 days, 25 days, 30 days, 35 days, 40 days, 45 days, 50 days, 55 days, 60 days, or 90 days. In some examples, the microspheres can release at least about 1%, 5%, 10%, 15%, 20%, 25%, 30%, 35%, 40%, 45%, 50%, 55%, 60%, 65%, 70%, or 75% of a morphogenic compound, such as over at least about 5 days, 10 days, 20 days, 25 days, 30 days, or 45 days.

In specific, non-limiting examples, the compounds of the microspheres disclosed herein can include small-molecule morphogen mimetics. In some examples, the small-molecule morphogen mimetic(s) can include guggulsterone (GS), purmorphamine (puro), and/or genipin. In particular examples, the small-molecule morphogen mimetic(s) include purmorphamine (puro), such as at least about 0.05 μg purmorphamine /mg microsphere, at least 0.15 μg purmorphamine /mg microsphere, at least 0.5 μg purmorphamine /mg microsphere, or at least 1 μg purmorphamine /mg microsphere, such as about 0.1-0.2, 0.2-0.3, 0.3-0.4, 0.4-0.5, 0.5-0.6, 0.6-0.7, 0.7-0.8, 0.8-0.9, or 0.9-1 μg purmorphamine /mg purmorphamine or about 0.78 μg purmorphamine /mg microsphere. In other examples, the small-molecule morphogen mimetic(s) can include puro, and the microspheres release puro at least at about 5%, 10%, 20%, 40%, 60%, 80%, or 100%, or about 5-10%, 10-20%, 20-40%, 40-60%, 60-80%, or 80-100% or about 60%-70% or 68%, such as over at least about 5 days, 10 days, 20 days, 25 days, or 30 days or about 28 days. Puro activates the hedgehog pathway, which is involved in stem cell renewal, and includes the following chemical formula and structure: $C_{31}H_{32}N_6O_2$

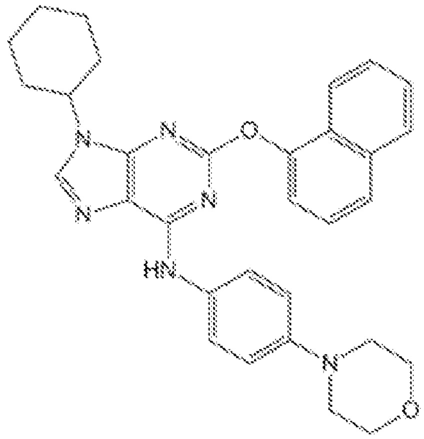

In other examples, the small-molecule morphogen mimetic(s) of the microspheres disclosed herein include guggulsterone (GS), such as at least about 0.05 μg GS /mg microsphere, 0.15 μg GS /mg microsphere, 0.5 μg GS /mg microsphere, or 1 μg GS /mg microsphere, such as about 0.1-0.2, 0.2-0.3, 0.3-0.4, 0.4-0.5, 0.5-0.6, 0.6-0.7, 0.7-0.8, 0.8-0.9, or 0.9-1 μg GS /mg microsphere or about 0.25 μg GS /mg microsphere. In other examples, the small-molecule morphogen mimetic(s) can include GS, and the microspheres release GS at least at about 5%, 10%, 20%, 40%, 60%, 80%, or 100%, or about 5-10%, 10-20%, 20-40%, 40-60%, 60-80%, or 80-100% or about 40%-50% or 45%, such as over at least about 5 days, 10 days, 20 days, 25 days, or 30 days or about 28 days. GS induces differentiation into dopaminergic neurons (DNs) and includes the following chemical formula and structures (*i.e.,* the E and Z conformations): $C_{21}H_{28}O_2$

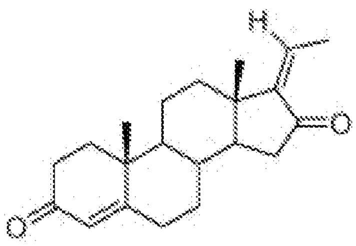

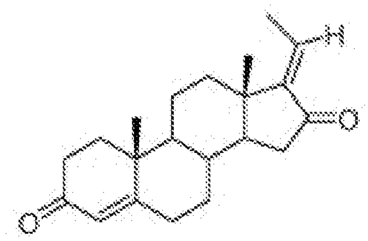

In further examples, the small-molecule morphogen mimetic(s) of the microspheres disclosed herein include genipin, such as at least about 0.05 μg genipin /mg microsphere, 0.15 μg genipin /mg microsphere, 0.5 μg genipin /mg microsphere, or 1 μg genipin /mg microsphere, such as about 0.1-0.2, 0.2-0.3, 0.3-0.4, 0.4-0.5, 0.5-0.6, 0.6-0.7, 0.7-0.8, 0.8-0.9, or 0.9-1 μg genipin /mg microsphere. In other examples, the small-molecule morphogen mimetic(s) can include genipin, and the microspheres release genipin at least at about 5%, 10%, 20%, 40%, 60%, 80%, or 100%, or about 5-10%, 10-20%, 20-40%, 40-60%, 60-80%, or 80-100%, such as over at least about 5 days, 10 days, 20 days, 25 days, or 30 days. Genipin can serve as a crosslinker (*e.g.,* in a hydrogel) and plays a role in cell differentiation (*e.g.,* Kwon et al., J Endod., 41(4):501-7,2015). Genipin also includes the following chemical formula and structure: $C_{11}H_{14}O_5$.

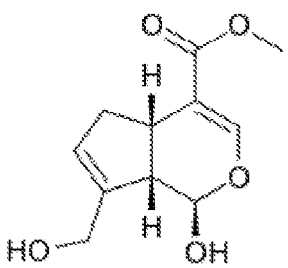

In particular examples, the compounds of the microspheres disclosed herein can include one or more morphogens. In some examples, the morphogens(s) can include retinoic acid (RA) and/or proteins or peptides, such as neurotrophic factors (NTFs, *e.g.,* glial cell line-derived neurotrophic factor, GDNF, and brain-derived neurotrophic factor, BDNF) or growth factors (*e.g.,* insulin growth factor, IGF-1). In some examples, the morphogen(s) can include RA, such

as at least about 0.5 μg RA/mg microsphere, 1 μg RA /mg microsphere, 2 μg RA /mg microsphere, 2.5 μg RA/mg microsphere, 3 μg RA /mg microsphere, 4 μg RA /mg microsphere, 5 μg RA /mg microsphere, 10 μg RA /mg microsphere, 15 μg RA/mg microsphere, 20 μg RA /mg microsphere, 30 μg RA /mg microsphere, 40 μg RA /mg microsphere, 50 μg RA /mg microsphere, or 75 μg RA /mg microsphere or about 2.4 or 18 μg RA /mg microsphere. In other examples, the morphogen(s) can include RA, and the microspheres release RA at least at about 5%, 10%, 20%, 40%, 60%, 80%, or 100%, or about 5-10%, 10-20%, 20-40%, 40-60%, 60-80%, or 80-100% or about 20%-30%, 50%-60%, 28%, or 53%, such as over at least about 5 days, 10 days, 20 days, 25 days, or 30 days or about 28 days. RA plays a role in cell differentiation and includes the following chemical formula and structure: $C_{20}H_{28}O_2$.

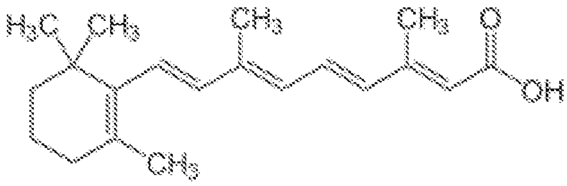

Other compounds and combinations of compounds can be present in the microspheres. Non-limiting examples of compounds (*e.g.,* morphogens), such as for differentiating stem cells to produce neural cells, include protein or peptide morphogens, such as neurotrophic factors (NTFs; *e.g.,* glial cell line-derived neurotrophic factor, GDNF, and brain-derived neurotrophic factor, BDNF) and insulin-like growth factor 1, (IGF-1). For example, other compounds may include morphogens, such as at least about 0.05 μg morphogen /mg microsphere, 0.15 μg morphogen /mg microsphere, 0.5 μg morphogen /mg microsphere, or 1 μg morphogen /mg microsphere, such as about 0.1-0.2, 0.2-0.3, 0.3-0.4, 0.4-0.5, 0.5-0.6, 0.6-0.7, 0.7-0.8, 0.8-0.9, or 0.9-1μg morphogen /mg microsphere or about 0.25 or 0.27 μg morphogen /mg microsphere). In other examples, the protein or peptide morphogen(s) can include NTFs and/or IGF-1, and the microspheres release protein or peptide morphogen(s) at least at about 1%, 5%, 10%, 15%, 20%, 30%, 40%, 50%, or 60%, or about 1-5%, 5-10%, 10-15%, 15-20%, 20-25%, 25-30%, 30-40%, 40-50%, 50-60%, or about 5%-10%, 10%-15%, 5.3%, or 11.3%, such as over at least about 5 days, 10 days, 20 days, 25 days, or 30 days or about 25 days.

In additional examples, the microspheres can include combined compounds, such as a combination of small-molecule mimetics, a combination of morphogens, or a combination of both small-molecule morphogen mimetics and morphogens. In specific, non-limiting examples, the combinations can include puro and RA (puro/RA); puro and GS (puro/GS); RA and GS (RA/GS); or RA, GS, and puro (RA/GS/puro). In specific examples, the microspheres include puro and RA, for example, at a ratio of at least about 0.1:1, 0.2:1, 0.5:1, 0.7:1, 1:1, 1.1:1, 1.2:1, 1.5:1, 1.7:1, 2:1, 2.5:1, 3:1, 3.5:1, 4:1, 4.5:1, 5:1, 7:1, 10:1, or 20:1, or about 0.1:1 - 0.5:1, 0.1:1 - 5:1, 1:1 - 3:1, 1:1 - 5:1, 1:1 - 10:1, or 1:1-20:1, or about 2:1 puro:RA. Other compounds, such as protein and/or peptide morphogens (e.g., NTFs and/or IGF-1) can be included. In some examples, other compounds, such as protein or peptide morphogens, can be included with other morphogens (*e.g.,* RA) and/or small-molecule morphogen mimetics (*e.g.,* puro and/or GS) in any combination.

## Methods of bioprinting and producing functional tissue

### *A. Methods of bioprinting*

The methods herein include bioprinting, such as using bioink (e.g., bioink as disclosed herein). The methods can include fabricating bioink. In some examples, fabricating bioink includes fabricating microspheres. The methods include incorporating microspheres, cells, and/or a carrier material into a bioink.

In some embodiments, the methods herein can further include fabricating bioink. Any type of bioink can be fabricated, such as the bioink disclosed herein. In some embodiments, the methods of fabricating bioink include fabricating microspheres for use in bioink. Any of the microspheres described herein can be fabricated. In some examples, microspheres are fabricated using an emulsion-based method (*e.g.,* a Pickering or an oil-in-water emulsion, including single or double emulsions; see, *e.g.,* Yuan et al., Front Pharmacol, 8:287, 2017). In some specific, non-limiting examples, the emulsion-based method can be a single emulsion method, such as emulsifying any of the compounds and biodegradable polymers of the bioink disclosed herein with an organic solvent evaporation step (*e.g.,* using polyvinyl alcohol, PVA; dichloromethane; acetonitrile; ethanol; and trifluoroacetic acid).

The methods can include fabricating microspheres using any amount of one or more compounds or biodegradable polymer suitable for the bioink application. For example, the methods can include using at least about 0.1 μg compound/mg biodegradable polymer, at least about 0.2 μg compound/mg biodegradable polymer, at least about 0.3 μg compound/mg biodegradable polymer, at least about 0.4 μg compound/mg biodegradable polymer, at least about 0.5 μg compound/mg biodegradable polymer, at least about 0.6 μg compound/mg biodegradable polymer, at least about 0.7 μg compound/mg biodegradable polymer, at least about 0.8 μg compound/mg biodegradable polymer, at least about

0.9 μg compound/mg biodegradable polymer, at least about 1 μg compound/mg biodegradable polymer, at least about 1.1 μg compound/mg biodegradable polymer, at least about 1.2 μg compound/mg biodegradable polymer, at least about 1.3 μg compound/mg biodegradable polymer, at least about 1.4 μg compound/mg biodegradable polymer, at least about 1.5 μg compound/mg biodegradable polymer, at least about 2 μg compound/mg biodegradable polymer, at least about 3 μg compound/mg biodegradable polymer, at least about 4 μg compound/mg biodegradable polymer, at least about 5 μg compound/mg biodegradable polymer, at least about 10 μg compound/mg biodegradable polymer, at least about 15 μg compound/mg biodegradable polymer, at least about 20 μg compound/mg biodegradable polymer, at least about 30 μg compound/mg biodegradable polymer, or at least about 40 μg compound/mg biodegradable polymer, or about 0.1-0.2, 0.2-0.3, 0.4-0.5, 0.6-0.7, 0.7-0.8, 0.8-0.9, 0.9-1, 1-2, 2-3, 3-4, 4-5, 5-10, 10-15, 15-20, 20-30, or 30-40 or about or about 0.5, 0.6, 0.93, 1.5, 2.5, 3, 4, or 30 μg compound/mg biodegradable polymer.

The compound(s) include morphogenic compounds, such as small-molecule morphogen mimetic compounds, or morphogens, such as retinoic acid RA) or protein or peptide morphogens (*e.g.,* neurotrophic factors, NTFs, or IGF-1). In some examples, the compound(s) used in fabrication include small-molecule morphogen mimetics, such as puro, for example at least at about 0.8, 0.9, or 1 μg compound/mg biodegradable polymer (*e.g.,* PCL), or about 0.8-0.9 or 0.9-1 or about 0.93 μg compound/mg biodegradable polymer. In other examples of small-molecule morphogen mimetic compounds, the compound can include GS, and the amount of GS used in fabrication can be at least about 0.5, 0.6, or 0.7 μg compound/mg biodegradable polymer (*e.g.,* PCL), or about 0.5-0.6 or 0.6-0.7 or about 0.6 μg compound/mg biodegradable polymer. In further non-limiting examples, the compound can include morphognes, such as RA, and the amount of RA used in fabrication can be at least about 1, 2, 3, 4, 5, 10, 20, or 30 μg compound/mg biodegradable polymer (*e.g.,* PCL), or about 1-2, 2-3, 3-4, 4-5, 5-10, 10-20, 20-30, or 30-40 or about 2.5, 3, 4, or 30 μg compound/mg biodegradable polymer. In further examples, the compound(s) can include protein or peptide mophogens, such as neurotrophic factors (NTFs; *e.g.,* glial cell line-derived neurotrophic factor, GDNF, and brain-derived neurotrophic factor, BDNF) and insulin-like growth factor 1, (IGF-1), and the amount of protein or peptide mophogens used in fabrication can be at least about 0.4, 0.5, 0.6, 0.7, 0.8, 0.9, 1, 1.5, or 2 μg compound/mg biodegradable polymer (*e.g.,* PCL), or about 0.4-0.5, 0.5-0.6, 0.6-0.7, 0.7-0.8, 0.8-0.9, 0.9-1, 1-1.5, or 1.5-2 or about 0.5 or 1.5 μg compound/mg biodegradable polymer.

In further embodiments, the methods can include fabricating microspheres using any encapsulation efficiency for a compound encapsulated therein. In some examples, the encapsulation effciency can be at least about 5%, 10%, 15%, 20%, 25%, 30%, 35%, 40%, 45%, 50%, 55%, 60%, 65%, 70%, 75%, 80%, 85%, 90%, 95%, or 100% or about 5-10%, 10-15%, 15-20%, 20-25%, 25-30%, 30-35%, 40-45%, 45-50%, 55-60%, 60-65%, 65-70%, 70-75%, 75-80%, 80-85%, 85-90%, 90-95%, or 95-100%, or about 19% 35%, 42%, 47%, 58%, 60%, or 85%. The compound(s) include a morphogenic compound, such as small molecule morphogen mimetic(s) (*e.g.,* GS and/or puro) and/or morphogen(s) (*e.g.,* neurotrophic factors, NTFs, for example glial cell line-derived neurotrophic factor, GDNF, and brain-derived neurotrophic factor, BDNF, as well as insulin-like growth factor 1, IGF-1). In some examples, the compound(s) include small-molecule morphogen mimetic(s), such as GS, puro, and/or genipin. For example, the compound(s) can include GS using an encapsulation efficiency of at least about 35%, 40%, 45%, or 50%, or about 35%-40%, 40-45%, or 45-50%, or about 42%; the compound(s) can include puro using an encapsulation efficiency of at least about 75%, 80%, or 85%, or about 75%-80%, 80-85%, or 85-90%, or about 84%; and/or the compound(s) can include genipin using an encapsulation efficeincy of at least about 5%, 10%, 15%, 20%, 25%, 30%, 35%, 40%, 45%, 50%, 55%, 60%, 65%, 70%, 75%, 80%, 85%, 90%, 95%, or 100% or about 5-10%, 10-15%, 15-20%, 20-25%, 25-30%, 30-35%, 40-45%, 45-50%, 55-60%, 60-65%, 65-70%, 70-75%, 75-80%, 80-85%, 85-90%, 90-95%, or 95-100. In other examples, the compound(s) can include morphogen(s), such as RA and/or peptide or protein morphogen(s) (e.g., NTFs and IGF-1). For example, the compound(s) can include RA using an encapsulation efficiency of at least about 50%, 55%, 60%, or 65%, or about 50%-55%, 55-60%, or 60-65%, or about 58%, or 60%, and/or the compounds can include protein or peptide morphogens (*e.g.,* NTFs and/or IGF-1) using an encapsulation efficiency of at least about 10%, 15%, 20%, 25%, 30%, 35%, 40%, 45%, 50%, or 55%, or about 10%-15%, 15%-20%, 20%-25%, 25%-30%, 30%-35%, 35%-40%, 40%-45%, 45%-50%, or 50%-55%, or about 19% or 47%.

In further embodiments, the methods can include fabricating microspheres using any encapsulation efficiency for a compound encapsulated therein. In some examples, the encapsulation effciency can be at least about 5%, 10%, 15%, 20%, 25%, 30%, 35%, 40%, 45%, 50%, 55%, 60%, 65%, 70%, 75%, 80%, 85%, 90%, 95%, or 100% or about 5-10%, 10-15%, 15-20%, 20-25%, 25-30%, 30-35%, 40-45%, 45-50%, 55-60%, 60-65%, 65-70%, 70-75%, 75-80%, 80-85%, 85-90%, 90-95%, or 95-100%, or about 19% 35%, 42%, 47%, 58%, 60%, or 85%. The compound(s) include a morphogenic compound, such as small molecule morphogen mimetic(s) (e.g., GS and/or puro) and/or morphogen(s) (*e.g.,* neurotrophic factors, NTFs, for example glial cell line-derived neurotrophic factor, GDNF, and brain-derived neurotrophic factor, BDNF, as well as insulin-like growth factor 1, IGF-1). In some examples, the compound(s) include small-molecule morphogen mimetic(s), such as GS and/or puro. For example, the compound(s) can include GS using an encapsulation efficiency of at least about 35%, 40%, 45%, or 50%, or about 35%-40%, 40-45%, or 45-50%, or about 42%, and/or the compound(s) can include puro using an encapsulation efficiency of at least about 75%, 80%, or 85%,

or about 75%-80%, 80-85%, or 85-90%, or about 84%. In other examples, the compound(s) can include morphogen(s), such as RA and/or peptide or protein morphogen(s) (*e.g.,* NTFs and IGF-1). For example, the compound(s) can include RA using an encapsulation efficiency of at least about 50%, 55%, 60%, or 65%, or about 50%-55%, 55-60%, or 60-65%, or about 58%, or 60%, and/or the compounds can include protein or peptide morphogens (*e.g.,* NTFs and/or IGF-1) using an encapsulation efficiency of at least about 10%, 15%, 20%, 25%, 30%, 35%, 40%, 45%, 50%, or 55%, or about 10%-15%, 15%-20%, 20%-25%, 25%-30%, 30%-35%, 35%-40%, 40%-45%, 45%-50%, or 50%-55%, or about 19% or 47%.

In some embodiments, the methods can further include fabricating bioink by incorporating cells (*e.g.,* stem cells or progenitor cells, such as in as a single cell suspension or cell aggregate) and microspheres (*e.g.,* including one or more compounds and biodegradable polymers) into a carrier material (*e.g.,* hydrogel, such as methacrylamide chitosan (MAC), fibrin, alginate, or any combination thereof).

The methods disclosed herein can further include bioprinting at least one layer of the bioink disclosed herein. Any of the bioink disclosed herein can be used. The methods can include bioprinting using any type of bioprinter. Exemplary methods of bioprinting can include using droplet- (*e.g.,* inkjet bioprinting), laser- (*e.g.,* selective laser sintering or stereolithography), and extrusion-based (*e.g.,* fused-deposition modeling, bioplotting, or microfluidic extrusion) bioprinter methods (see, *e.g.*, Thomas and Willerth, Front Bioeng Biotechnol, 5:69,2017).

In specific, non-limiting examples, the methods include using an inkjet-based bioprinter methods, such as methods using a conventional or modified piezo-based bioprinter (see, *e.g., id.* and Walus et al., Lab Chip, 16:3351,2016).

*β. Methods of producing functional tissue*

The methods herein include producing functional tissue (*e.g.,* bone or soft tissue, such as epithelial, connective, muscular, or nervous tissue, for example, neural tissue). In some examples, the methods can include bioprinting at least one layer of bioink, such as at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 70, at least 80, at least 90, or at least 100 layers, or about 5-10 layers, 10-20 layers, 20-30 layers, 30-40 layers, 40-60 layers, 60-80 layers, or 80-100 layers, or about 8 layers (*e.g.,* for bioprinting neural tissue) or 40-60 layers (*e.g.,* for bioprinting on a tubular shape) of bioink (*e.g.,* as disclosed herein), for example on a solid support. One skilled in the art can determine a number of layers, which can depend, for example, on the application as well as layer geometry and thickness.

In some examples, the methods include incubating the bioink. In certain examples, the bioink can be incubated at about at least 15°C, 18°C, 20°C, 22°C, 25°C, 28°C, 30°C, 37°C, or 42°C or about 15-20°C, 20-25°C, 25-30°C, 30-37°C, or 37-42°C, or about 37°C. In other examples, the bioink can be incubated under $CO_2$, such as about at least 0.25%, 0.5%, 1%, 2%, 3%, 4%, 5%, 6%, 8%, or 10%, or 0.25-1%, 1-5%, or 5-10% or about 5% $CO_2$. In further examples, the bioink can incubated in media. Any media that supports the growth of cells or tissue can be used. In some examples, the media is formulated to support the growth of a tissue or cell type of interest, such as media that supports neural tissue growth (e.g., a serum-free neuronal basal medium, such as BRAINPHYS™ products, STEMCELL™ TECHNOLOGIES, for example BRAINPHYS™ Neuronal Medium; Du, Z. W., et al., Nature communications, 2015. 6: p.6626). Other agents can be added to the media to support the growth of a tissue or cell type of interest, such as agents that support neural tissue growth, for example, IGF-1, BDNF, ciliary neurotrophic factor (CNTF), and other neural growth supplements (e.g., serum-free supplements, such as N-2™ and/or B-27™, THERMOFISHER SCIENTIFIC™; Du, Z. W., et al., Nature communications, 2015. 6: p.6626). In some examples, the media can include In certain examples, the media can include antimicrobial agents, such as antibiotics and/or antimycotics (*e.g.,* to reduce contamination), for example, penicillin, streptomycin, actinomycin D, kanamycin, ampicillin, neomycin, carbenicillin, cefotaxime, polymyxin B, fosmidomycin, gentamicin, or any combination thereof (*e.g.,* streptomycin and penicillin, such as in a Pen Strep formulation, THERMOFISHER SCIENTIFIC™). In some examples, the media is changed during incubation, such as at least about once every week, once every three days, once every two days, once a day, or twice a day. In additional examples, all or part of the media can be changed, such as at least about 5%, 10%, 20%, 30%, 40%, 50%, 60%, 75%, 90%, or 100% or about 5-10%, 10-25%, 25-50%, 50-75%, or 75-100 of the media is changed.

In other examples, the methods can include bioprinting at least one additional layer of bioink hours, days, weeks, and/or months after at least one layer has been bioprinted. For example, the methods can include bioprinting at least one additional layer of bioink about at least 2 hours, at least 4 hours, at least 6 hours, at least 12 hours, at least 18 hours, at least 24 hours, at least 48 hours, at least 3 days, at least 4 days, at least 5 days, at least one week, at least two weeks, at least three weeks, at least one month, at least two months, or at least three months, or about 1-7 days, 1-3 weeks, or 1-3 months, or about 2-4 weeks after at least one layer has been bioprinted.

The methods include measuring one or more markers of differentiation or maturation. Measuring markers of differentiation or maturation can include measuring small molecules, proteins, peptides, nucleic acids, or physiological characteristics of cells in the at least one layer of bioink. Exemplary markers of differentiation or maturation include

nestin, beta-tubulin III, stage-specific embryonic antigen-4 (SSEA-4), NeuN, oligodendrocyte transcription factor (OLIG2), homeobox HB9 (HLXB9), paired box protein (PAX6), synapsin I, SRY-box 2 (SOX-2), insulin gene enhancer protein (ISL-1), anti-oligodendrocyte O4 (O4), choline acetyltransferase (ChAT), synaptophysin, Oct3/4, c-Myc, kruppel-like factor 4 (KLF4), microtubule-associated protein 2 (MAP2), calbindin, acetylcholinesterase (ACHE), tyrosine kinase A receptor (TrkA), or tyrosine hydroxylase. In other examples, the methods can include measuring markers of neural progenitor cells. Exemplary markers of neural progenitor cells include Sox1, Sox2, Sox3, Nkx6, Pax6, Olig2, Ngn2, Sox21, NeuroM, MNR2, Lim3, Isl1/2, Hb9, N1x2.2, Sox8, Sox9, and/or Sox10 (see, *e.g.*, Briscoe and Novitch, Phil. Trans. R. Soc. B, 363:57-70, 2008).

In other examples, the markers can include cell function (*e.g.,* functional action potentials in neuronal cells) or cell morphology (*e.g.,* neurite extension or branching in neuronal cells, *i.e.,* neurite length and branch points). Other assays for cell or tissue growth can be performed, such as cell survival assays and/or $Ca^{2+}$ imaging.

The methods include detecting the level of expression of one or more markers of differentiation or maturation expected in a functional tissue. In some examples, the methods can include measuring increased expression of one or more of ChAT, O4, NeuN, beta-tubulin III, HLXB9, synaptophysin, or tyrosine hydroxylase compared with expression expected in a pluripotent stem cell. In other examples, the methods can include measuring decreased expression of one or more of SSEA-4, SOX-2, OLIG2, ISL-1, synapsin I, Oct3/4, c-Myc, nestin, PAX6, or KLF4 compared with expression expected in a pluripotent stem cell. In still further examples, the methods can include detecting cells with neurite extension and/or branch points.

## Examples

### Example 1: Engineering neural tissue from human pluripotent stem cells using small molecule releasing microspheres

In this example, a biomaterial-based system that generates controlled release of the small molecule purmorphamine (puro) was developed and characterized. A mixture of puro- and retinoic acid (RA)-loaded microspheres was incorporated into hiPSC aggregates that differentiated into mature neural tissue as demonstrated by ChAT and Sy38 expression and electrophysiological activity. Thus, puro-releasing microspheres can be used to engineer neural tissue *in vitro.* Such microspheres can be used as an alternative to soluble puro when performing extended neural differentiation protocols *in vitro.*

<u>Materials</u>

Puro was from Cayman Chemical (Purity ≥98%, 10009634). PCL (Mn ~45,000), polyvinyl alcohol (PVA) (Mw - 13,000-2,3000, 87-89% hydrolyzed), laminin from Engelbreth-Holm-Swarm murine sarcoma basement membrane, poly-L-omithine (PLO) 0.01 solution, normal goat serum (NGS), triton X-100, N2, B27, cyclic-AMP (c-AMP), ascorbic acid (AA) and penicillin streptomycin (PenStrep) were from Sigma-Aldrich. Brain-derived neurotrophic factor (BDNF), glial cell-derived neurotrophic factor (GDNF) and insulin-like growth factor 1 (IGF-1) were from Peprotech.

Dichloromethane (DCM) and trypsin-EDTA were from Fisher Scientific. Trypsin neutralization solution (TNS) (0113) was from ScienCell. Anhydrous ethyl alcohol was from Commercial Alcohols. Acetonitrile (ACN) (HPLC 190) was from Caledon Laboratory Chemicals. Phosphate buffer solution (PBS) was from Invitrogen. TeSR™-E8™ media, Vitronectin XF™, STEMdiff™ neural induction medium (NIM), AggreWell™800 plates, and ReLeSR™ were from STEM CELL Technologies (Vancouver, BC, Canada). Undifferentiated hiPSCs (iPS(Foreskin)-1, Lot 1-DL-01) were purchased from WiCell (Madison, WI, USA). 4,6-diamidino- 2-phenylindole, dihydrochloride (DAPI) nucleic acid stain and 0.2 μm polytetrafluoroethylene (PTFE) syringe filters were from Thermo Fisher Scientific.

For flow cytometry, human/mouse SOX-2 ((sex determining region Y)-box 2) PE-conjugated antibody (IC2018P), human/mouse SSEA-4 (Stage-specific embryonic antigen 4) PE-conjugated antibody (FAB1435), neuron-specific beta-III tubulin (βT-III) PerCP-conjugated antibody (IC1195C), human olig1, 2, 3 PE-conjugated antibody (IC2230P), oligodendrocyte marker (O4) PE-conjugated antibody (FAB1326P), isotype control mouse IgG1 fluorescein-conjugated antibody (IC002F), mouse IgG2A PerCP-conjugated isotype control (IC003C), normal mouse IgM PE-conjugated control (IC015P) were from R&D Systems. Anti-ChaT antibody [HR2] (ab2803), mouse IgG2b-Isotype Control (ab91366), anti-synaptophysin antibody [SY38] (ab8049), mouse IgG1 [B11/6]-isotype control (ab91353) were from Abcam.

For immunocytochemistry (ICC), goat anti-mouse IgG (H+L) highly cross-adsorbed secondary antibody, Alexa Fluor 488 (A-11029) was purchased from Thermo Fisher Scientific. vHB9/HLXB9 polyclonal antibody, FITC-conjugated (bs-11320R-FITC) was purchased from Bioss antibodies. PE mouse anti-islet-1 (562547) was from BD Pharmingen™.

Anti-βT-III antibody (Rabbit polyclonal) (ab18207), anti-nestin antibody [SP103] (ab105389), anti-synapsin I (Syn1) antibody-synaptic marker (ab64581), anti- ChaT antibody (ab2803), donkey anti-goat IgG H&L (Alexa Fluor®

405) (ab175664), and goat anti-rabbit IgG H&L (Alexa Fluor® 488) (ab150077) were from Abcam. Anti-human SSEA-4 antibody (MC 813-70), anti-βT-III antibody, clone TUJ1(60052) were from Stem cell Technologies. Anti-βT-III antibody isoform (MAB1637), anti-MNX1 (HB9) antibody (ABN174) were purchased from Millipore. Goat anti-mouse IgG (H+L) cross-adsorbed secondary antibody, Alexa Fluor 568 (A11OO4), goat anti-rabbit IgG (H+L) cross-adsorbed secondary antibody, Alexa Fluor 568 (A-11011), Goat anti-Mouse IgG (H+L) highly cross-adsorbed secondary antibody, Alexa Fluor 488 (A-11029) were from Thermo Fisher Scientific. Human Olig2 antibody (AF2418) was from R&D. Neurite growth and branching was analyzed using the IncuCyte® ZOOM automatic live-cell imaging system from Essen BioScience. The electrophysiological analysis was performed using the Axion Muse MEA System with single-well MEA microelectrodes (M64-GL1-30Pt200, Axion Biosystems).

Methods

*Fabrication of small molecule releasing microspheres*

Microspheres were fabricated using a single emulsion oil-in-water (o/w) protocol followed by the evaporation of the organic solvent as previously described [40]. A 2% PVA solution was generated as previously described [42]. Briefly, PVA was dissolved in de-ionized water at 50°C with constant stirring using a magnetic stir bar. Next, 321 mg of PCL was dissolved in 3 ml of DCM in a 25 ml Erlenmeyer flask on a Corning PC-420D magnetic mixer for 15 minutes at 900 rpm. An adapted stopper covered with TaegalSeal PTFE tape was placed on top of the flask to avoid evaporation. A puro stock solution was prepared by diluting the drug in 100% ethanol to produce a stock concentration of 190.2 μM. Before adding the drug, the stock solution was warmed to room temperature, 0.3 mg of the drug was added to the oil phase to generate a final concentration of 0.93 μg/mg microspheres (w/w, puro/PCL). After the drug was added, 3 ml of 2% PVA was added to the oil phase, and the solution was then emulsified on a vortex mixer for 30 seconds at maximum speed. Once emulsified, the solution was delivered into the water phase at 35°C and stirred at 500 rpm for 4 hours (or more). The solution was centrifuged at 3000 rpm (Eppendorf 5810R) and washed with de-ionized water to collect the microspheres, for example, until the supernatant appears clear. In some examples, the solution is filtered before centrifugation, such as sterile filtering (e.g., Steriflip® filter units, Millipore®, or reversible strainers, STEMCELL™ TECHNOLOGIES). The pellet was and frozen at -20°C to - 80°C and then lyophilized. RA-loaded and unloaded microspheres were prepared as previously described [42, 43].

*Microsphere characterization using scanning electron microscopy*

Microsphere characterization of size and shape was performed using the Hitachi S-4800 FE scanning electron microscope (SEM) as described previously [42-44]. Approximately 0.1 mg of microspheres were placed into a micro tube with 50 μl of 100% ethanol and mixed vigorously with the vortex mixer. Next, 2 μl of the solution was placed on the SEM stub mount. The sample was coated using a gold-palladium sputter coater (Anatech Hummer VI sputter coater). Images were obtained with an accelerated voltage of 1.0kV and a working distance of 8 mm. The microsphere size was determined using ImageJ software (version 1.48). A minimum of 600 microspheres were analyzed for each batch of microspheres characterized. Probability and frequency plots of microsphere data were produced using R studio program. Encapsulation efficiency was determined, and controlled release of purmorphamine was characterized.

A 46-day release study was performed to determine the release kinetics of purmorphamine. Each sample contained 10 mg of microspheres and 1ml of PBS placed on 1.5 ml micro tubes (Axygen®). Samples for each day of collection (n=3) were placed on a VWR® mini shaker at 330 rpm at 37°C and were collected every 4 days. After collection, samples were washed with de-ionized water and freeze-dried for further analysis. The drug was extracted from the microspheres and then quantified with high-performance liquid chromatography (HPLC) to estimate the puro concentration in the microspheres. Next, 10 mg of microspheres was placed in 1.5 ml microtubes in triplicate. To melt the microspheres, 250 μl of ACN was added to the microspheres and mixed with the vortex mixer at 3000 rpm for 30 seconds. The samples were then placed on the vortex mixer (Eppendorf ® MixMate®) at 2500 rpm for 5 minutes. An additional 250 μl ACN was added to the sample followed by the same mixing procedure to obtain a volume of 500 μl. The samples were then placed at -80°C for 5 minutes followed by centrifugation (Eppendorf 5424) at 1500rpm for five minutes. The samples were filtered using 0.2 μm PTFE syringe filters before adding the supernatant to amber vials. HPLC was performed using an Agilent 1200 equipped with quaternary pump and diode-array detector (DAD) to quantify the amount of puro present in samples. A standard of puro with eleven dilutions of a stock solution diluted in ACN was used to determine the concentrations of the samples. The column used was an Eclipse Plus C18 (Agilent) with 5 μm particle size, 4.6 mm internal diameter (ID), and was 150-mm long with similar phase and ID guard column. Samples were analyzed at 300 nm after mass spectroscopy was used to generate a spectrum for puro. Mass spectroscopy information was acquired using an Ultimate 3000 HPLC system connected to an Orbitrap (Thermo Fisher Scientific). The system was used in direct infusion mode with no separation. Solvents used for this analysis were HPLC-grade ACN mixed with

18.4 water dispensed from a Milli-Q integral purification system (Millipore, ON, Canada). Both contained 0.1% V/V trifluoroacetic acid (TFA) (Fisher Scientific, Hampton, NH). HPLC was performed using isocratic elution at a 70%: 30% ratio, respectively, with an injection volume of 20μl, flow rate of 1 ml/min, and temperature of 21°C. ChemStation software was used for data analysis with auto-integration adjusted manually when needed. Here, EE is defined as a comparison of the amount of puro encapsulated ($P_{encapsulted}$) to the amount of drug initially added ($P_{theoretical}$) and was determined using the following equation, previously described [42].

$$EE = P_{encapsulated}/P_{theoretical} * 100\% \qquad [1]$$

*hiPSC culture and formation of engineered tissues*

hiPSCs (iPS(Foreskin)-1, Lot 1-DL-01, WiCell) were cultured in 6-well plates coated with Vitronectin XF™ in the presence of -E8™ medium until reaching 80% confluency, as previously described [45]. Eleven confluent wells were used to form uniform EBs in an AggreWell™ 800 plate, as previously described[40, 42]. Four conditions were tested: unloaded microspheres, 2:1 Puro/RA microspheres, a negative control group, and a positive control group with 2:1 Puro/RA soluble drugs added from day 1. For the microsphere groups, a total of 0.5 mg of microspheres was suspended in STEMdiff™ NIM and incorporated into the aggregates to generate a total volume of 2 ml per AggreWell™ 800 well. Negative group cultures contained no microspheres nor soluble drug. Positive group cultures contained a final puro concentration of 1 μm and 0.5 μm RA. Media changes were performed by replacing 1.5 ml of STEMdiff NIM with 1% PenStrep every day until day 7. On day 7, EBs were transferred to each well of a PLO/laminin coated 24-well plate. The number of EBs in each well ranged from 2 to 5. EBs were cultured in 1 ml of STEMdiff™ NIM, and the media was changed every third day by replacing 500 μl with fresh media. By day 16, BrainPhys™ neuronal medium gradually replaced STEMdiff™ NIM, and, by day 25, each well contained 100% BrainPhys™ Neuronal Medium. To promote MN differentiation, BrainPhys™ was supplemented with N2(1%), B27(1%), BDNF(10ng/ml), GDNF(10ng/ml), IGF-1(10ng/ml), c-AMP(1μM), AA(200ng/ml), and PenStrep(1%) [46].

*Analysis of cell cultures using flow cytometry*

Cell marker expression was quantified with flow cytometry on days 15, 28, 35 and 60. Aggregates were enzymatically dissociated with 0.125% trypsin-EDTA for 2 minutes at 37°C followed by the addition of TNS. The resulting cells were washed three times by adding PBS and centrifuging at 600 g for 5 minutes. The cells were then fixed and stained per the manufacturer's instructions (R&D systems). Expression of SSEA-4 (pluripotency marker), βT-III (neuronal marker), and SOX-2 (pluripotent cells and undifferentiated cells in the neural epithelium), was evaluated on day 15. Expression of SSEA-4, βT-III and Olig2 was evaluated on day 28. Flow cytometry was performed to evaluate expression of Olig2, HB9 (mature MN) and ISL-1 (mature MN) on day 35. Expression of O4, ChAT and Sy38 was evaluated on day 60.

*Analysis of cell cultures using immunocytochemistry*

Cell cultures and engineered tissues were stained for various markers in accordance with the ICC protocol previously published [40, 44, 45]. Cells were stained for βT-III (1:500), SSEA-4 (1:250), PAX6 (1:500, neural precursor marker) and Nestin (1:500, NSC marker) on day 15. Cells were stained for the antibodies βT-III (1:500), NeuN (1:1000), HB9 (1:1000) and Olig2 (5ug/ml) on day 28. Immunocytochemistry (ICC) was performed using βT-III (1:500), Syn1 (1:200), Olig2 (5ug/ml), HB9 (1:100) and ChAT (1:500) on day 35. Primary antibodies were diluted in blocking solution (5% NGS) and incubated overnight. The cells were then washed three times with PBS and incubated with the secondary antibodies for 4 hours at room temperature. Finally, the cells were washed three more times and counterstained with DAPI (nuclear stain) with a 3-minute incubation at room temperature. Cell cultures were imaged with a Leica DMI3000 B microscope using a XCite Series 120Q fluorescent light source and QImaging RETIGA 2000R camera with 100X magnification. Captured images were done using QCapture Software 2.9.12. Composite images were created using the image-processing program ImageJ with the Magic Montage tool [42].

*Analysis of the morphological properties of engineered tissues*

Neurite growth and branching metrics were performed using the IncuCyte® ZOOM automatic live-cell imaging system as previously reported [42]. Briefly, the NeuroTrack™ software module was used to process the images, previously stained with βT-III, on days 15, 28 and 35. A processing definition was created for the analysis by creating a collection of images taken at the same magnification (10X), allowing the masking of the relevant lengths of neurites extending from the cell bodies and branch points. For day 15 cultures, a neurite aggregate analysis was performed using the Basic

Analyzer processing definition. Thirty-six images per well were collected, and the montage was created using Image J.

*Electrophysiological analysis of cultures*

Microelectrode array (MEA) recordings were generated using the Axion Muse MEA System with a single-well MEA containing 64 microelectrodes. After 41 days of *in vitro* differentiation, aggregates from the puro/RA-loaded microspheres group were removed from the PLO-laminin and re-plated in the MEA well coated with laminin. Aggregates were cultured in 400 μl of BrainPhys™ Neuronal Medium supplemented with the aforementioned nutrients, and media changes were performed every third day by replacing half of the total media. The extracellular voltage was recorded at each electrode with a sampling rate of 25kHz. A 20-minute recording was collected each day for 10 days. AxIS software (Axion Biosystems) was used to detect spikes (*i.e.* action potentials) from the raw electrical signal on each electrode. The signals were filtered using a Butterworth filter from 200 Hz to 3 kHz, and a threshold of ±6 standard deviation was independently set for each electrode; activity exceeding this threshold was considered a spike. Bursts were identified with a minimum of 5 spikes and maximum inter-spike interval of 0.1 seconds.

*Statistical analysis*

Puro EE and release study results are reported as the mean ± standard deviation (n=3). Flow cytometry and immunohistochemistry (ICC) (n=3), neurite extension and branching (n=6), electrophysiological analysis (n=1). Statistical analysis for cell studies was performed with a two-tailed Student's t-test using a confidence level of 95% ($\alpha$ <0.05).

<u>Results</u>

*Characterization of puro-encapsulated microspheres and their release kinetics*

Puro-encapsulated microspheres exhibited a smooth, rounded morphology **(FIG. 1A)** similar to that observed with unloaded microspheres **(FIG. 1B).** The average diameter was 3.4 ± 1.17 μm with the size distribution shown in **FIG. 1C.** The unloaded microsphere diameter was previously reported as 2.24 ± 2.04 μm [42]. The puro EE was 84% ± 2.12. An initial burst of drug release (~ 17%) was observed on Day 1 followed by slow, continuous release over the remaining 45 days. Ninety-one percent of the theoretically encapsulated drug was released by day 46. Characterization of 4μg/mg (w/w, RA/PCL) RA-loaded microspheres was previously reported, in which the microspheres exhibited an average diameter of 3.59± 2.48 μm and an EE of 60.9 ± 1.9% [40].

*Microsphere-directed differentiation of hiPSCs into functional neural tissue: Combining drug-releasing microspheres with hiPSCs to generate neural tissue*

Previous studies differentiated iPSCs into MN by adding 0.1 μM RA from day 10 and puro at a concentration of 1 μM from day 15 [47]. In preliminary studies, different ratios for the combination of both drug-loaded microspheres were examined on the differentiation of hiPSCs. A ratio of 2:1 puro/RA showed better results for neural differentiation of hiPSCs. A ratio of 2:1 was selected for the concentrations of puro and RA in the positive group as well as for the number of loaded microspheres containing the respective drugs. The concentration of puro for the positive group was initially maintained at 1 μm [47], followed by a decrease in concentration throughout differentiation to simulate the amount of a drug being released from the microsphere group. Likewise, the concentration of RA was selected from the aforementioned ratio followed by a decrease in concentration throughout differentiation. The appearance of microspheres was evident in the aggregates combined with unloaded as well as puro- and RA-loaded microspheres after one day of formation in the AggreWell™ 800 plate. The EBs containing microspheres appeared larger than the negative and positive groups by day 7 **(FIGS. 9A-9P).**

**FIGS. 2A-2D** show qualitative images of neurite growth and extension for all cell culture conditions after 35 days. The aggregates were stained for βT-III expression and then imaged using an IncuCyte automated imaging machine. Aggregates containing puro/RA microspheres showed extensive, organized neurites **(FIG. 2A).** The negative group and cell aggregates combined with unloaded microspheres show reduced, unorganized neurite growth **(FIGS. 2B-2C).** The positive group exhibited a larger cluster area compared with the puro/RA microsphere group. These cultures also exhibited long, organized neurites **(FIG. 2D).**

Quantitative analysis of neurite growth and branching was performed using an IncuCyte ZOOM® live-cell imaging system and the Neurotrack and Basic Analyzer configuration software on days 15, 28, and 35 **(FIGS. 3A-3C).** Cell aggregates loaded with unloaded microspheres exhibited the most neurite growth and branching per cluster area compared with the other groups on day 15 **(FIG. 3A).** The positive group and cells with puro/RA-loaded microspheres showed the lowest magnitudes per NA compared with the negative group at this time point as well. Significant statistical differences

were observed for neurite length between the positive and unloaded microsphere conditions and the positive-negative conditions. Cell aggregates with puro/RA-loaded microspheres exhibited an increased magnitude of neurite growth and branching per cluster area compared with the negative group on day 28 **(FIG. 3B).** The unloaded microsphere group showed a similar magnitude as the negative group at the same time point. By day 35, neurite growth and branching per cluster area of positive group and aggregates with puro/RA microspheres increased compared with the negative group and cell aggregates with unloaded microspheres. Statistical differences in neurite branching per cluster area were observed for positive-unloaded microspheres and positive-negative conditions **(FIG. 3C).**

*Analysis of protein expression in Day 15 cultures*

ICC was performed for all cultures at day 15 using βT-III (neuronal marker) and SSEA-4 (pluripotency marker) as well as a DAPI counterstain. All aggregates showed neural rosette formation (**FIGS. 4A, 4E, 4I,** and **4M**)**.** Neural rosettes are a developmental signature of neural progenitor cell, and the presence of these structures was observed in all groups. Interestingly, the negative group had the most neural rosettes compared with the other groups **(FIG. 4E).** βT-III expression was exhibited in all of the conditions (**FIGS. 4B, 4F, 4J,** and **4N**)**.** Neurite growth and extension was prominent in the positive group and the aggregates with puro/RA-loaded microspheres. SSEA-4 was only expressed in negative and positive groups; aggregates combined with microspheres did not show SSEA-4 expression (**FIGS. 4C, 4G, 4K,** and **4O**). On day 15, aggregates were also stained for PAX6 and Nestin. Surprisingly all the conditions were negative for these cell markers. Flow cytometry was performed at the same time point to quantify cell marker expression. On day 15, the expression of SOX-2, SSEA-4 and βT-III was quantified for all conditions (**FIGS. 4Q, 4R,** and **4S**). Each condition is represented by a letter: positive (P), negative-untreated (N), aggregates combined with unloaded microspheres (B), and aggregates combined with puro/RA-loaded microspheres (M). N and B showed increased expression of SOX-2. The expression of this marker was statistically significant between P and the rest of the conditions as well as in between B and M. SSEA-4 marker expression was high in all groups by day 15; no significant differences were observed between groups. Between M-P and P-B, significant differences were observed for the expression of βT-III on day 15 **(FIG. 4S).** M showed the highest expression of this marker with 94% followed by B and N with 86% and 68%, respectively. P showed only 6% of βT-III expression on day 15.

*Analysis of protein expression in Day 28 cultures*

Cell aggregates were stained for βT-III as well as NeuN (neuronal nuclei marker) and counterstained with DAPI on day 28 **(FIGS. 5A-5S).** All conditions showed significant levels of neurite growth as visualized by βT-III expression (**FIGS. 5B, 5F, 5J,** and **5N**). The positive group showed dense and organized neurites, and aggregates combined with puro/RA microspheres showed thinner, organized neurites compared with the negative group and aggregates combined with unloaded microspheres. NeuN expression was positive for all conditions (**FIGS. 5C, 5G, 5K,** and **5O**). In addition, cell aggregates were stained for Olig2 and HB9 (MN marker) on day 28. Olig2 expression was negative for all groups, and HB9 expression was only slightly positive for the aggregates combined with puro/RA microspheres **(FIGS. 10A-10P).** Quantitative marker expression was also examined for SSEA-4, βT-III and Olig2. The expression of SSEA-4 on day 28 decreased compared with levels on day 15. Few undifferentiated cells were present at this time point. SSEA-4 expression for the unloaded microspheres and negative groups were 0.5% and 0.46%, respectively. Significant differences were observed for the M-P and M-N groups. Similar decreases were observed for βT-III expression on day 28 compared with day 15. N expression for βT-III (41%) was the highest observed among the four groups, and statistical significances were observed between M-P, M-N, M-B and P-B. The quantitative expression of Olig2 for M was significantly higher (73%) compared with the other groups. P showed the lowest expression (19%) followed by N (21%). Statistical differences were observed for M-P and M-N for Olig2 expression.

*Analysis of protein expression in Day 35 cultures*

Expression of Olig2, βT-III, and Syn1 was analyzed using ICC on day 35 **(FIGS. 6A-6S).** The expression of Olig2 and Syn1 was negative for all conditions. βT-III expression showed neurite outgrowth for all conditions. Aggregates combined with unloaded microspheres exhibited the least neurite growth extension and no organization. The positive group and aggregates combined with puro/RA-loaded microspheres show extensive and organized neurites (**FIGS. 6B, 6F, 6J,** and **6N**). Quantitative expression of Olig2, HB9 and ISL-1 on day 35 was analyzed using flow cytometry. On day 35, reduced expression of OLIG2 was observed for all groups, except the positive group compared with day 28 (7.5%). M showed 2% of OLIG2 expression on day 35 compared with 24% on day 28. Statistical significances were observed between P-M and P-N **(FIG. 6Q).** The expression of HB9 on day 35 is shown in **FIG. 6R.** P and M showed similar levels of expression, whereas N expression was reduced; however, no significant differences were observed. N exhibited higher expression of ISL-1 by day 35 **(FIG. 6S),** and similar levels of expression for P and M were observed.

*Analysis of protein expression in Day 60 cultures*

The expression of mature neural markers, O4, Sy38, and ChaT, was quantified on day 60 with the majority of cultures staining positive for the mature neuronal marker ChaT **(FIGS. 7A-7C).** M showed increased expression of O4 compared with the other conditions **(FIG. 7A).** Sy38 expression was greatest on M and P with statistical differences between both. M also showed differences with B and N as well as P-N and P-B **(FIG. 7B).** ChAT expression for M was also high compared with the other groups and showed statistical differences with P, N, and B **(FIG. 7C).**

*Electrophysiological analysis*

Cell aggregates combined with puro/RA loaded microspheres were cultured and differentiated *in vitro* for 64 days. To assess the electrophysiological function of these cultures, extracellular field potentials were recorded using microelectrode array (MEA) technology **(FIG. 8A-8D).** Cells plated on the MEA plate on day 41 demonstrated spontaneous electrical activity by day 54. **FIG. 8A** shows the temporal raster plot for 5 minutes of recorded spontaneous activity on day 64. **FIG. 8B** shows the raw voltage data of spontaneous neuronal spikes and the representative successive wave forms of spikes in real time on electrode 23 of the MEA well. Recordings on 3 different electrodes were observed after electrical stimulation was applied **(FIG. 8C). FIG. 8D** shows the mean firing rate over 10 days of the differentiated neurons; by day 64, the mean firing rate was 0.018 Hz compared with 0.01 Hz from day 54.

Discussion

In this example, successful encapsulation of the small molecule puro in microspheres capable of generating its sustained release over 46 days is shown. The EE of puro into the microspheres (84%) was higher than previously reported for other encapsulated drugs, such as RA (60.0%) [40] and GS (42.4%) [42], and was similar to Clonazepam (72.6-95.1%) [48] and tamoxifen [49]. The microspheres were characterized and showed a smooth, spherical shape. The average microsphere diameter size was 3.4± 1.17 μm, similar in size to unloaded and RA-loaded microspheres with diameters of 2.24±1.04 and 3.59± 2.48, respectively [40, 42]. The amount and size of the microspheres are important parameters for sustained delivery of morphogens when combined with cell aggregates. Carpenedo *et al.* reported that smaller microspheres in the range of 2-5 μm allow for better incorporation within the cell aggregates [50]. Gomez *et al.* reported that excessive levels of microspheres disrupt cell-to-cell interactions that are required for cell survival, proliferation and differentiation [40]. The total amount of microspheres per cell aggregate used herein was the same as previously reported [40, 42] and results correlate by showing aggregate formation, survival and differentiation **(FIGS. 9A-9P).**

The three types of microspheres used (puro, RA, and unloaded) fall within the range for incorporation with cell aggregates. The release study was performed for a period of 46 days in order to analyze the effect of the drug and microspheres on the cells when being exposed for longer than 35 days, which is the time period reported by Zhang *et al.*, for differentiating immature MN [8]. The release kinetics of puro was analyzed over 46 days. By day 28, 68% of the encapsulated puro was released, which was higher than the levels of RA released at the same time point (28%) and GS (45%). By day 44, -87% of puro was released in comparison with 55% for GS for the same time points [40, 42]. A final cumulative release of 91% was observed on the release study **(FIG. 1D**) which corresponds to 2.2μg of puro released every day from 10mg of microspheres . For the cell aggregates combined with drug-loaded microspheres, only 0.34mg of puro-loaded microspheres were added. Therefore ~0.0748μg of puro was released on average per days. For the positive group, an average amount of puro of 0.38μg total was used per day of media change for the first 46 days of the study. Media changes were performed every day from day 0-7, followed by media changes every third day. The disclosed differentiation method using puro-loaded microspheres as a drug delivery system requires significantly smaller quantitates of puro than traditional differentiation protocols. Additionally, microsphere incorporation resulted in more homogenous differentiation. By allowing the cell aggregates to form in combination with the drug-loaded microspheres, the inside layers of these aggregates are directly exposed to the morphogens that are continuously released. On the other hand, only the outside layer of the positive group aggregates is in direct contact with the drug, resulting in concentration gradients and less homogeneous differentiation.

In this example, quantitative analysis of neurite length and branching was performed for days 15, 28 and 35. The number of branches and neurite length were larger for the positive group and drug loaded microspheres groups in day 28. Results for neurite growth on day 35 were extensive, organized neurites extended from the cell aggregate for both treatments. The length of neurites per cluster area was larger for the positive group than the drug loaded microspheres group, however neurite branching was similar for both groups. No statistical differences were observed in between these groups. These results indicate that the constant presence of both morphogens promotes neurite branching and extension. Cell aggregates for all conditions expressed βT-III after 15 days, which is a microtubule element present in all neural differentiation.

Minimal levels of SSEA-4 expression were observed by day 28, indicating that the exposure and sustained release of these morphogens from day 1 contribute to induced differentiation. Safer treatments can be achieved by avoiding differentiation of pluripotent cells into undesired cell lineages and malformations. The low expression of SSEA-4 is an indicator of the lack of pluripotency expression and it reflects the directed differentiation of the cell aggregates into neural tissues. SOX-2, one of the Yamanka factors, is expressed during embryonic development and by pluripotent cells as well as in the neural epithelium [51, 52]. The quantitative analysis of SOX-2 at this stage showed ~10% of total cells in the aggregates combined with puro and RA loaded microspheres, ~17% of cell aggregates with unloaded microspheres and less than 5% in the positive group. The high expression of the unloaded microsphere group could be attributed to a pluripotent state, whereas the lower expression in the positive and drug loaded microsphere group can be attributed to a more mature neural state on Day 15.

Unlike the results reported by Zhang *et al.*, expression of PAX6 and Nestin were not observed. In this example, hiPSCs are exposed to sustained release of RA and puro on day 1 while Zhang *et al.* added these molecules at day 10 and 15 respectively. It is possible that these markers were expressed earlier during this protocol of differentiation. Expression of PAX6 and Olig2 transcription factors triggers the notochord to release a concentration gradient of SHH [53]. The expression of Olig2, a transcription factor expressed in MN progenitors, was also analyzed by day 28 . Olig2 expressing cells differentiate into oligodendrocytes or MN when expression of HB9 and Isl1 increases and Olig2 expression levels decrease [18, 19, 47]. Interestingly the qualitative expression of Olig2 appeared to be negative for all groups, whereas the quantitative expression with flow cytometry showed ~24% of total cells expressing Olig2 in cell aggregates combined with puro and RA loaded microspheres. The absence of Olig2 expression for ICC analysis could be due to the concentration of antibody used. Similar expression was reported by Shin *et al.*, when adding SSH and RA to Olig2 GFP-positive cells [18]. By day 35 the quantitative expression of Olig2 was downregulated for the cell aggregates combined with puro and RA loaded microspheres, followed by the expression of HB9 and Isl1. The qualitative analysis of Olig2 and Syn1 (Synapsin I - protein expressed in the synaptic vesicles required for neurotransmitter release) was negative for all groups. Maturation of MN for 3-4 weeks is required for neurotransmitter release and signal transduction [8, 54]. After 8 weeks of culture *in vitro,* cell marker expression of the oligodendrocyte cell-surface marker (04) was observed mainly in cell aggregates combined with puro and RA loaded microspheres (2.5% of total cells). After several weeks of maturation, the expression of ChaT (enzyme that catalyzed the production of the neurotransmitter acetylcholine which is expressed by MN) was significantly expressed in the drug loaded microsphere group in comparison with other treatments. Additionally, Synaptophysin- the most abundant integral membrane protein of small synaptic vesicles [54], was expressed in this group. These results indicate the presence of mature MN derived from hiPSCs after 8 weeks of culture *in vitro.* These results correlate with the information obtained from the electrophysiological analysis where electrical activity was observed from spontaneous action potentials and response when electrical stimulation was applied.

These results demonstrate that an engineered tissue can be delivered as a cell therapy for treatment of SCI by combining patient derived-hiPSCs with drug loaded microspheres. In addition, drug loaded microspheres can be used to promote cell differentiation in both 2D culture and in 3D bioprinted tissues, avoiding the labor work of constantly adding the soluble morphogens in the media.

The disclosed l puro-loaded microspheres can be incorporated in bioinks used during 3D bioprinting of tissue as described herein. 3D bioprinting can automate tissue engineering and make it replicable while minimizing human intervention. These 3D bioprinted neural tissues can be used as drug screening tool and disease modeling with a tissue that can mimic human physiology.

References

[1] Takahashi et al., Cell, 131 (2007) 861-872.
[2] Khazaei et al., Front Cell Dev Biol, 4 (2016) 152.
[3] Bahmad et al., Front Mol Neurosci, 10 (2017) 50.
[4] Nagoshi, H. and Okano, J Neurochem, 141 (2017) 848-860.
[5] Lancaster et al., Nature, 501 (2013) 373-379.
[6] Conley et al., Int J Biochem Cell Biol, 36 (2004) 555-567.
[7] Kurosawa, J Biosci Bioeng, 103 (2007) 389-398.
[8] B.Y. Hu and S.C. Zhang, Nat Protoc, 4 (2009) 1295-1304.
[9] Wilem and Sakiyama-Elbert, Journal of Controlled Release, 213 (2015) 103-111.
[10] Silva et al., Prog Neurobiol, 114 (2014) 25-57.
[11] P.K. Stys, Journal of Cerebral Blood Flow & Metabolism, 18 (1998) 2-25.
[12] Yuan and He, Neurosci Bull, 29 (2013) 421-435.
[13] Assinck et al., Nature Neuroscience, 20 (2017) 637.

[14] Bensadoun et al. Journal of Controlled Release, 87 (2003) 107-115.
[15] Nori et al., Proc Natl Acad Sci USA, 108 (2011) 16825-16830.
[16] C.A. Priest et al., Regenerative Medicine, 10 (2015) 939-958.
[17] Asterias Biotherapeutics, Asterias Biotherapeutics Announces Dosing of First Patient in New SCiSTAR Clinical Trial Cohort Testing AST-OPC1 in an Expanded Cervical Spinal Cord Injury Patient Population, in, Asteriasbiotherapuetics.com, 2016.
[18] Shin et al., Stem Cells Dev., 16 (2007) 131-141.
[19] B.S. Jha et al., Stem Cell Rev, 11 (2015) 194-204.
[20] N. Bahrami et al., Mol Neurobiol, (2016).
[21] X. Wu et al., Chem Biol, 11 (2004) 1229-1238.
[22] Y. Yan et al., Acta Biomater, 42 (2016) 114-126.
[23] K.D. Magdalini Tsintou and Alexander Marcus Seifalian, Neural Regen Res, 10 (2015) 726-742.
[24] J. Zhang et al., Cell Tissue Res, 366 (2016) 129-142.
[25] A. Montgomery et al., Biomater Sci, 3 (2015) 401-413.
[26] C. Lopez-Serrano et al., Cell Transplant, (2016).
[27] C.S. Ahuja et al., Neuropathology, 29 (2009) 248-257.
[29] T.Z. Hiroshi Nomura et al., Tissue engineering part A, 14 (2008) 649-665.
[30] J. Edgar et al., Acta Biomater. 2017, 51:237-245.
[31] H. Li et al., Adv Healthc Mater, 5 (2016) 802-812.
[32] M. Hiraoka et al., Bioconjugate chemistry, 20 (2009) 976-983.
[33] N.K. Mohtaram et al., Journal of Biomedical Materials Research Part A, 103 (2015) 2591-2601.
[34] C.D. Johnson et al., Cells Tissues Organs, 202 (2016) 116-135.
[35] A.M. Bratt-Leal et al., Biotechnol Prog, 25 (2009) 43-51.
[36] A.P. Van Winkle et al., Cells Tissues Organs, 196 (2012) 34-47.
[37] A.M. Bratt-Leal et al., Biomaterials, 34 (2013) 7227-7235.
[38] O. Qutachi, K.M. and Shakesheff, L.D., J Control Release, 168 (2013) 18-27.
[39] Makadia and Siegel, Polymers (Basel). 2011 Sep 1;3(3):1377-1397.
[40] J. Gomez et al., Cellular and Molecular Bioengineering, 8 (2015) 307-319.
[41] V.R. Sinha et al., Int J Pharm, 278 (2004) 1-23.
[42] Agbay A et al., Biomed Mater, 2018, 13(3):034104
[43] J.C. Gomez et al., Cellular and Molecular Bioengineering, 8 (2015) 307-319.
[44] A. Agbay et al., Drug Delivery and Translational Research, 4 (2014) 159-170.
[45] M. Robinson et al., Biomarker Insights, 2015:10(S1).
[46] B.-Y. Hu et al., Proceedings of the National Academy of Sciences of the United States of America, 107 (2010) 4335-4340.
[47] B.-Y. Hu and S.-C. Zhang, Nature protocols, 4 (2009) 1295-1304.
[48] S.Y.C. C. Changyong and N. Jae-Won, Polymer, 47 (2006) 4571.
[49] Shenoy and Amiji, Int J Pharm. 2005 Apr 11;293(1-2):261-70.
[50] R.L. Carpenedo et al., J Biomed Mater Res A, 94 (2010) 466-475.
[51] S.R. Hutton and L.H. Pevny, Developmental Biology, 352 (2011) 40-47.
[52] X. Liu et al., Cell Res, 18 (2008) 1177-1189.
[53] J. Briscoe and J. Ericson, Current Opinion in Neurobiology, 11 (2001) 43-49.
[54] G. Thiel, Brain Pathology, 3 (1993) 87-95.

**Example 2: Guggulsterone-releasing microspheres direct the differentiation of human induced pluripotent stem cells into neural phenotypes**

In this example, guggulsterone (GS), a drug for terminal differentiation of DNs from PSCs, was encapsulated within PCL microspheres using a single emulsion technique. As demonstrated herein, GS can be released from these biodegradable microspheres over 44 days. In addition, these drug-releasing microspheres were successfully incorporated within PSC-derived cell aggregates to demonstrate that they promote high levels of neuronal differentiation.

Methods and Materials

Poly (ε-caprolactone) (PCL) (Mn ~ 45,000), polyvinyl alcohol (PVA) (Mw ~ 13,000-23,000, 87-89% hydrolyzed), (E)-guggulsterone (≥95% HPLC, powder), laminin from Engelbreth-Holm-Swarm murine sarcoma basement membrane, poly-L-omithine (PLO) 0.01% solution, Normal goat serum (NGS), and Triton X-100 were from Sigma-Aldrich (St. Louis,

MO, USA). Dichloromethane (DCM) was from Fisher Scientific (Ottawa, ON, Canada). Goat anti-mouse IgG (H+L) highly cross-adsorbed secondary antibody, Alexa Fluor® 488 and 4,6-diamidino- 2-phenylindole, dihydrochloride (DAPI) nucleic acid stain were from Thermo Fisher Scientific (Waltham, MA, USA). Flow cytometry fixation buffer, flow cytometry permeabilization buffer/wash buffer, neuron-specific beta-III tubulin perCP-conjugated antibody (IC1195C), mouse IgG2A perCP-conjugated isotype control (IC003C), human Olig1, 2, 3 PE-conjugated antibody (IC2230P), and normal mouse IgM PE-conjugated control (IC015P) were from R&D Systems (Minneapolis, MN, USA). Anti-GFAP antibody [GF5] (ab10062) and mouse IgG2b [PLPV219] isotype control (ab91366) were from Abcam (Cambridge, MA, USA). Anhydrous ethyl alcohol was from Commercial Alcohols (Brampton, ON, Canada). Acetonitrile (ACN) HPLC 190 was from Caledon Laboratory Chemicals (Georgetown, ON, Canada). Phosphate buffer solution (PBS) was from Invitrogen (Burlington, ON, Canada). TeSR™-E8™ Kits for hESC/hiPS Maintenance, Vitronectin XF™ Kits, STEMdiff™ Neural Induction Medium, AggreWell™800 plates, anti-beta-tubulin III mouse monoclonal [clone AA10] IgG2a antibody, and ReLeSR™ were from STEMCELL Technologies (Vancouver, BC, Canada). Undifferentiated hiPSs (iPS(Foreskin)-1, Lot 1-DL-01) produced by viral mediated transfection (lentiviral expression of Oct4, Sox2, Nanog, Lin28) were from WiCell (Madison, WI, USA).

*Preparation of single emulsion microspheres*

Microspheres were fabricated using an oil-in-water (o/w) emulsion followed by the evaporation of the organic solvent as previously described [40]. For the water phase, a 2% PVA solution was generated by dissolving PVA in de-ionized water for 1 hour at 85°C while mixing at 850 rpm on a Corning PC-420D magnetic mixer. One hundred milliliters of 0.3% (w/v) PVA solution was generated by diluting 2% PVA with de-ionized water and held at 35°C. Five hundred milligrams of PCL was dissolved in 3 mL of DCM on a magnetic mixer for 15 minutes at 900 rpm to produce the oil phase; 0.3 mg of the drug (dissolved in 100% ethanol) was added to the oil phase to produce microspheres at a concentration of 0.6 μg/mg (w/w, GS /PCL) microspheres. Unloaded microspheres were fabricated by adding an equal volume of ethanol without the drug. After removal from the magnetic mixer, 3 mL of 2% PVA were slowly added to the oil solution to prevent disruption of the boundary layer. An emulsion of the solution (w/o) was then produced by vortex mixing (Fisher Scientific) at 3000 rpm for 15 seconds. This (w/o) emulsion was immediately added to the 0.5% PVA water phase and held at 35°C at a mixing speed of 500 rpm for 4 hours (or more) to evaporate the organic solvent. After mixing, the microspheres were isolated by centrifugation at 4000 rpm (Eppendorf 5810R) and washed with de-ionized water. In some examples, the solution is filtered before centrifugation, such as sterile filtering (e.g., Steriflip® filter units, Millipore®, or reversible strainers, STEMCELL™ TECHNOLOGIES). For long-term storage, the microspheres were lyophilized for 24 hours and stored at -20°C to - 80°C.

*Characterization of surface morphology and particle size analysis*

Morphological characterization of dried particles was performed using a Hitachi S-4800 FE scanning electron microscopy (SEM) machine to image the microspheres after fabrication, as described previously [40, 42]. Lyophilized microspheres were transferred to loading stubs by ethanol suspension and evaporation and then coated with gold-palladium using an Anatech Hummer VI sputter coater to enhance surface conductivity. Images were collected using an accelerated voltage of 1.0 at working distances of 8.0 mm and 8.2 mm. The average diameter of the microspheres was determined by using the ImageJ image processing program to conduct manual diameter measurements on SEM microsphere images. Characterization of particles in solution (swollen particles) was performed using a particle sizer. Lyophilized microspheres were suspended in PBS at varying concentrations and loaded onto a ZetaPALS zeta potential/particle size analyzer (Brookhaven Instrument Corp.).

*Determination of guggulsterone encapsulation efficiency*

The amount of GS encapsulated per unit weight of microspheres was determined by extracting the drug from the fabricated microspheres. A measured amount of lyophilized microspheres was placed in a 1.5 mL propylene microtube, and 200 μL of ACN was added to each sample. The samples were vortexed at 3000 rpm for 30 seconds, vortexed for 5 minutes at 350 rpm (Eppendorf® MixMate®), and mixed with a micropipette for 10 seconds. These mixing steps were repeated twice to dissolve the PCL. Next, 1000 μL of additional ACN was added to the samples and mixed by micropipette for 10 seconds and then vortexed at 3000 rpm for 30 seconds. The solution was then centrifuged at 22°C and 13000 rpm for 5 min using an Eppendorf 5424 Microcentrifuge. Thereafter, 1000 μL of the supernatant was transferred to a microtube and diluted as necessary for high performance liquid chromatography-mass spectrometry (HPLC-MS). The concentration of the drug in the sample was determined using HPLC-MS (Ultimate 3000 MSQ, Thermo Scientific with ChromeleonTM software) and run against a GS standard composed of five dilutions of a stock solution composed

of GS dissolved in ACN. Analysis was performed using a C18 column (Phenomenex Luna 5u C18) with a constant eluent mobile phase composition of 80% ACN and 20% water with 0.1% trifluoroacetic acid while detecting at a characteristic absorption wavelength of 255 nm in the ultraviolet-visible spectrum based on the parameters described by Ahkade *et al.* (Akhade, Agrawal, & Laddha, 2013). The sample injection volume was 50 μL, and the flow rate was 1.5 mL/min. To calculate encapsulation efficiency, the actual encapsulated GS ($G_{encapsulated}$) was compared with the amount of GS originally added ($G_{theoretical}$) according to Eq. (1).

$$\text{Encapsulation efficiency} = (G_{encapsulated}/G_{theoretical}) \times 100\% \qquad (1)$$

*In vitro guggulsterone release study*

*In vitro* release studies were performed in triplicate. Ten milligrams of microspheres was suspended in 1 mL of PBS in a microtube. The tubes were then loaded onto a Sarstedt Sarmix mr-1 tube rotator and incubated at 37°C. The PBS supernatant for each tube was replaced every 2 days while tubes were removed and collected from the rotator at the following predetermined time points: day 2, 4, 8, 12, 16, 20, 24, 28, 36, and 44. For the collected tubes, the PBS supernatant was removed, and the microspheres were washed with deionized water, lyophilized, and weighed. The remaining GS concentration inside the microspheres was determined as previously described by dissolving microspheres with DCM, extracting the drug/precipitating PCL with 100% ethanol, and reading the absorbance at 255 nm using a Tecan Infinite® M200Pro plate reader (Gomez, *et al.*, 2015). The amount of GS released was calculated by subtracting the GS remaining in the microspheres from the theoretical GS present in each amount of microspheres at day 0. Percentages of the drug that were assumed to be released for each sample day were calculated by comparing drug amounts leftover and initial amounts of the drug inside the microspheres to create a cumulative release graph.

*Pluripotent stem cell culture*

hiPSs were maintained in an undifferentiated state on 6-well plates coated with Vitronectin XF™ using TeSR™-E8™ media as previously described [23]. This system did not require the use of feeder cells, and the components were xeno-free. The media was changed daily. For passaging, ReLeSR™ was used to select and remove undifferentiated hiPSs from the wells. Cells were passaged at a ratio of 1:6 and plated on fresh Vitronectin XF™ coated plates.

*Cellular aggregate formation and incorporation of microspheres*

ReLeSR™ was used to select and remove undifferentiated hiPSs from the wells. Uniform hiPS aggregates were formed by adding a single cell suspension in STEMdiff™ Neural Induction Medium of 1x106 hiPSs to wells in AggreWell™ 800 plates and centrifuged for 5 min at 100×g to deposit the cells at the bottom of each microwell. Before incorporation with the cells, the microspheres were sterilized by low power air-plasma treatment (Harrick PDC-32G) for 30 seconds as described previously [40]. When forming microsphere-incorporated hiPS aggregates, 0.5 mg of microspheres suspended in STEMdiff™ Neural Induction Medium was added to each AggreWell™ 800 well for a total of 2 mL of media in each well and centrifuged for 5 min at 100×g (**FIGS. 11A-11B**). The aggregates in the AggreWell™ 800 plates were maintained in 2 mL of STEMdiff™ Neural Induction Medium with daily media changes for 5 days. On day 5, the aggregates were harvested for immunocytochemistry or transferred to PLO/laminin-coated 24-well plates at a single aggregate per well for the remaining duration of the cell study (until day 12 or day 20). Positive control treatments contained aggregates without microspheres and GS in the media at a concentration of 2.5 μM. Negative control treatments contained aggregates without microspheres and media without soluble drug added. Unloaded microspheres without the drug encapsulated were also incorporated within aggregates to investigate the physical effects of the polymer microspheres themselves.

*Immunocytochemistry of cultures*

The cells were processed for immunostaining according to prior protocols [23, 40]. Briefly, after growth for 12 or 20 days, the cell aggregates were permeabilized with 0.1% Triton X-100 and then blocked with 5% NGS. Primary antibody anti-beta-tubulin III added and incubated overnight. Secondary antibody Alexa Fluor® 488 was then added, and the cells were counterstained with DAPI following incubation. The cells were then visualized with a Leica DMI3000 B microscope using an XCite Series 120Q fluorescent light source and QImaging RETIGA 2000R camera at 100X magnification. Images were captured using QCapture Software 2.9.12.

*Flow cytometry analysis of cultures*

Cell marker expression was quantified with flow cytometry on day 20 for β-tubulin III, Olig-2, and GFAP. The aggregates were enzymatically dissociated with 0.125% trypsin-EDTA for 2 min at 37°C followed by adding trypsin neutralization solution (TNS). The resulting cells were washed three times by adding PBS and centrifuging at 600 × g for 5 minutes. The cells were then fixed and stained per manufacturer's instructions (R&D systems). Briefly, the cells were incubated in the fixation buffer for 30 minutes at room temperature, washed, and resuspended in permeabilization buffer. Marker antibodies or isotype controls were added to the buffer and incubated for 1 hour at 4°C in the dark. The cells were then washed twice and resuspended in PBS. Marker expression data was collected using a Guava EasyCyte HT (Millipore) flow cytometer.

*Neurite morphology analysis of cultures*

Immunostained cell aggregates were imaged with an IncuCyte® ZOOM automatic live-cell imaging system (Essen BioScience, Ann Arbor, MI) at 10X magnification. Quantification of the morphological metrics of neurite length and branch points was performed using IncuCyte® ZOOM Software (2016A). Using the NeuroTrackTM software module, day 12 and day 20 green channel fluorescence (anti-beta-tubulin III positive) images were analyzed by masking the total neurite coverage and calculating the average length and branch points with the same processing definition. The cell aggregate area was analyzed for day 12 images using a NeuroTrack™ software processing definition, whereas a Basic Analyzer processing definition (used for confluence masking) was used to analyze the day 20 images. Metrics were calculated per single aggregate with two aggregates per treatment group for all negative and positive control time points and one aggregate per treatment group for microsphere-incorporated time points. Composite cell aggregate images were created using the Magic Montage plugin for the ImageJ image-processing program.

*Statistical analysis*

Results are reported as the mean values ± standard deviation of the mean. Statistical analysis was performed with the Minitab® 17.3.1 statistics software applying one-way analysis of variance with Tukey's post-hoc analysis on neurite morphology metrics and flow cytometry data with a 95% confidence level where significance is considered for $p < 0.05$.

<u>Results</u>

*Microsphere characterization*

Guggulsterone-containing microspheres were fabricated using a single emulsion technique and then imaged with an SEM to determine their morphology and size distribution (**FIGS. 12A-12E**). The microspheres displayed a spherical shape with smooth surfaces, while their diameters were not uniform. In contrast, single emulsion unloaded microspheres also displayed a similar morphology but displayed a much more uniform size distribution and average diameter. The average diameter of the dried GS microsphere was 6.14 ± 9.09 μm with a median of 3.28 μm. Measuring microsphere diameters in suspension with a particle-sizer yielded an average microsphere diameter of 14.8 ± 5.9 μm, which is within the standard deviation of the dried microsphere measured average. Dried unloaded microspheres had an average diameter of 2.24 ± 1.04 μm with a median of 2.04 μm.

The drug encapsulation efficiency of GS inside the microspheres was 42.4 ± 3.5 % of the total GS added during the fabrication process. Release kinetics of the drug from the microspheres was observed inversely by determining drug remaining inside the microspheres over a range of sample days ending on day 44 (**FIG. 13**). The slope of the graph exhibits a calculated average release of 37 ng drug released per day from 10 mg of microspheres. Considering the theoretical total amount of drug initially in each sample (~2.5 μg in 10 mg of microspheres) on day 0, a cumulative release profile of the drug from the microspheres was calculated (**FIGS. 12A-12E**). On the final day of release studies, the microspheres released ~60% of the drug theoretically encapsulated.

*Microsphere incorporation*

The microspheres were incorporated with hiPSs at a ratio of 0.5 mg of microspheres and 1 × 106 hiPSs to form cell aggregates (**FIGS. 14A-14P**). After 5 days, aggregates had formed in all test conditions. Incorporation of GS-encapsulated microspheres was evident due to the dark spheroids inside of the aggregates visualized throughout the aggregate formation process and after attaching onto PLO/laminin plates. Unloaded microspheres were also visible during the *in vitro* study. Although successful incorporation of guggulsterone-encapsulated microspheres was observed,

large microspheres (up to ~60 μm in diameter) beyond the expected average had been incorporated as well. After attaching onto PLO/laminin plates and growing until day 15, all conditions exhibited growth and spreading. After 20 days, more extensive cell growth was observed in all conditions.

*Immunocytochemistry*

After 12 days *in vitro,* 7 days after attachment on PLO/laminin plates, the cell aggregates exhibited neurite outgrowth from the center of the aggregate in all test conditions and stained positive for TUJ1 (**FIGS. 15A-15D**). Cell aggregates in all conditions exhibited more extensive neurite outgrowth by day 20 compared to day 12 (**FIGS. 16A-16D**). High resolution images are shown in **FIGS. 17-24**. Both microsphere-incorporated treatment aggregates displayed a prominent aggregate center that was difficult to resolve for finer details on day 12 and day 20. On day 12, statistical comparisons yielded a significant difference for the neurite metrics between groups for neurite length, but not branching. The GS microsphere treatments exhibited greater neurite length compared to the negative control and unloaded microsphere treatments on day 12 (FIGS. 25A-25B). Similarly, the day 12 positive control exhibited greater neurite length compared to the negative control. At the end of the *in vitro* study, the neurite metrics on day 20 yielded no significant difference between all conditions with respect to neurite length and branching.

*Flow cytometry*

Neuronal marker expression was examined after 20 days *in vitro* to determine if GS release from microspheres affected aggregate differentiation. Quantification of cells in each condition expressing βIII-tubulin (TUJ1), Olig2, and GFAP yielded a percentage of cells identified as differentiating into post-mitotic neurons, oligodendrocytes, and astrocytes, respectively. After 20 days, TUJ1 expression for the GS microsphere condition was the highest at ~24% (**FIGS. 26A-26C**). This expression level was significantly higher compared to all other conditions tested. The unloaded microsphere condition exhibited a greater expression (-15%) compared to the negative control (-10%). The conditions that yielded the lowest TUJ1 expression were the positive controls; soluble GS at 0.25 μM at ~4% and 2.5 μM at ~7%. For Olig-2 expression, GS microsphere treatment produced the highest expression (-17%), while the expression in all other conditions did not differ significantly (under 5%). Expression of GFAP was low in all conditions (~1%) with both the positive controls and microsphere conditions slightly higher than the negative control.

## Discussion

*Microsphere characterization*

GS delivery for long-term release in PSC differentiation was examined. First, GS - encapsulated microspheres less than 10 μm were successfully produced; the size distribution of the microspheres was not uniform. The histogram and diameter averages show a varied size distribution. The majority of microspheres were under 10 μm; however, a small population of microspheres exhibited much larger diameters, which was reflected in a large standard deviation (9.09 μm). In contrast, the unloaded microspheres exhibited a smaller diameter and small size distribution. This difference in microsphere size may be due to parameter differences during the fabrication process, which can slightly vary between batches. It has been shown that temperature, stirring rate, and amount of emulsifier can all influence the microsphere resulting size [43]. In fact, the molecule encapsulated can also affect microsphere size [44].

Diameter measurements with a particle sizer for GS -encapsulated microspheres suspended in PBS yielded an average just above 10 μm. By suspending the particles in a liquid, the size of the particles should increase by swelling. A non-homogeneous size distribution may have contributed to the possibility of larger microspheres in the particle-sizer samples, which could have inflated the average. Additionally, the difference between hand-measured and particle-sizer averages may be due to the particle sizer having difficulties with measuring a non-uniform sample of molecules which tend to settle out in solution.

The GS encapsulation efficiency inside the microspheres was determined as almost half of the drug added at 42.4 ± 3.5 %. This percentage is similar, though slightly lower, than retinoic acid-encapsulated microspheres (-48-58%) [40, 47]. Because the parameters for producing the guggulsterone microspheres were similar to these studies and resulted in a lower encapsulation efficiency, it is possible that the structure of the drugs decreased loading [40]. GS contains a signature cholesterol four-hydrocarbon ring, whereas previous studies encapsulated the drug retinoic acid, which is composed of a single cyclic ring structure with a hydrocarbon chain. Other studies of steroid molecule encapsulation in single-emulsion microspheres report encapsulation efficiencies as low as ~9% for hydrocortisone, ~22% for levonorgestrel, and ~55%, the highest efficiency, for hydrocortisone 21-acetate [44, 48]. In these studies, organic solvent solubility may affect encapsulation efficiency. Indeed, the increasing solubility of levonorgestrel, GS, and retinoic acid in ethanol is consistent with their reported relative encapsulation efficiencies (~22%, ~42%, and ~60%, respectively).

The release kinetics of GS from the microspheres were observed over 44 days to parallel and surpass the terminal 38 day differentiation treatment of GS in the previous hiPS study [23]. For cumulative release, the rate of drug release is similar to the previous PCL single emulsion microsphere system fabricated by Gomez *et al.* [40]. At day 28, ~45% of the total GS encapsulated was released, which is similar to retinoic acid release from PCL microspheres at ~ 28% and ~53% for two different concentrations (the latter concentration being 7.5 times greater than the former) [40]. These results show that microspheres with a GS have a release time course greater than 38 days using PCL. Furthermore, while ~62% of the drug was released from the microspheres at the end of the 44 days, additional release of the remaining drug is possible. If the release rate was consistent beyond 44 days, the microspheres would deliver GS for an additional 27 days, for a total of 71 days of drug release.

The average release of GS per day from ~10 mg of microspheres was calculated as ~37 ng. For incorporation with hiPSs, 0.5 mg of microspheres were added to each aggregate-forming well to keep the aggregates intact [40]. This amount corresponds to a concentration of ~0.93 ng/mL of GS released. In comparison, Robinson *et al.* added GS at a concentration of 2.5 μM, which corresponds to a concentration of 781 ng/mL in each aggregate-forming well [23]. Accordingly, the determined release from the microspheres is not sufficient to match the drug concentrations outlined in previous DN-producing GS studies [22, 23]. However, the concentrations used in prior studies were not confirmed to be the effective concentrations to elicit differentiation of PSCs into DNs, and it is likely that such an effective concentration is lower than what is outlined. To date, no study has determined the effective concentration of GS for such applications. In addition, the microspheres allow drug release inside of cellular aggregates, which may yield more homogenous local delivery of the drug. Due to concerns of mass transfer limitations inside of cell aggregates and evidence of aggregate structural changes that restrict diffusive transport, the drug concentrations required for release from microspheres may be lower than media soluble drug concentrations for the same differentiation induction effect [28] [29]. In fact, Ferreira *et al.* reported that lower concentrations of the vascular differentiation factors bFGF and PIGF released from PLGA microspheres resulted in comparable or even superior vascular differentiation through incorporation into EBs vs. soluble drug in media (bFGF at 175 pg/mL vs. 50 ng/mL and PIGF at 887 pg/mL vs. 50 ng/mL) [49]. In this example, vascular differentiation from microsphere release was observed at concentrations much lower than the concentrations of the soluble drug in media.

Degradation of GS can affect the amount of drug release and, subsequently, the efficacy of differentiation. GS is susceptible to degradation, including photodegradation [50]. However, encapsulation of the drug within microspheres to protect against harmful conditions may prevent such degradation.

*Microsphere incorporation, immunocytochemistry, and flow cytometry*

Microspheres were successfully incorporated within hiPS aggregates. The cell aggregates did not disintegrate for both microsphere treatments after 5 days in the wells and maintained their spherical structure when attaching onto the PLO/laminin plates. Incorporation of large GS-encapsulated microspheres (up to ~ 60 μm) inside the aggregates was visually confirmed. Unloaded microspheres were also visible in the aggregates. The large size distribution of GS-encapsulated microspheres may have caused inhomogeneous incorporation of particles into the cellular aggregates, hence the presence of larger microspheres.

The incorporation of microspheres influenced the observed morphology of the aggregates. All cell aggregates containing microspheres exhibited a prominent aggregate center that seemed to restrict spreading. Qualitatively, the positive and negative control aggregates spread out more after attaching onto the PLO/laminin plates. The microspheres may have prevented spreading, as cells may have attached to their surfaces, tethering other cells nearby. The polymer PCL does not readily support cell attachment due to its lack of functional groups and inherent hydrophobicity. However, surface modifications can alter this property [51-55]. Among these modifications, plasma treatment increases cellular adhesion on the polymer surface [51, 53, 55]. For the cellular *in vitro* portion of this example, use of plasma sterilization on the microspheres before incorporation may have imparted a similar increased adhesion property to the polymer, and the large surface area of the microspheres may have affected the aggregate's ability to spread out.

β-tubulin III staining was present in the neurites of all conditions due to the production of neural-ectodermal fated cells using the cell culture media. From day 12 to day 20, the number of neurites increased as well as their length, branch points, and complexity of arrangement. Quantitative analysis of neurite length for the negative control and unloaded microspheres on day 12 were less than the guggulsterone-encapsulated microspheres. The positive control did not differ from the GS-encapsulated microspheres but was greater than the negative control for neurite length. This indicates that treatment with guggulsterone increases neurite length, even though this difference did not persist to day 20 as all treatments produced similar neurite metrics at the final time point. It is possible that this treatment promotes more rapid differentiation of hiPSs.

At day 20, TUJ1 expression was highest in GS microsphere-incorporated conditions along with unloaded microspheres compared to positive and negative controls. Both positive control conditions had the lowest expression of TUJ1. Although the expectation of GS as a differentiator for dopaminergic neurons would demonstrate a higher number

of cells expressing TUJ1, GS was previously confirmed to influence late stage differentiation as opposed to early differentiation [22]. Perhaps GS does not have a potent effect or has limited effect at this earlier time point. Conversely, the release of GS from incorporated microspheres produced the highest expression. Soluble GS may not influence differentiation beyond the surface of the aggregate while incorporation of particles allowed a high local concentration of the drug for cells within the aggregate. In addition, the unloaded microsphere condition exhibited a high expression as well. These results indicate that the presence of the microspheres influences hiPS differentiation. Other PCL scaffold formulations promote differentiation and growth of cells into neurons. Mohtaram *et al.* showed that the surface morphology of PCL scaffolds can influence neuron differentiation, Abbasi *et al.* observed that PCL scaffold orientation can influence TUJ1 expression, and Jakobsson *et al.* found that the presence of a PCL scaffold can alter neuron growth morphology [56-58]. Finally, the availability for cell attachment on the microspheres may also influence differentiation. PCL functionalized with carboxyl groups by plasma sterilization increases cell attachment and growth of neurons [57, 59].

At day 20, Olig2 expression was the highest in GS microsphere-incorporated aggregates. The other conditions did not differ significantly among one another and exhibited relatively low expression. Again, soluble GS may have not been at high enough concentrations to penetrate into the aggregates. Although GS may promote oligodendrocyte differentiation at an earlier time point, Olig2 may inhibit astrogenesis [60, 61]. Indeed, low GFAP (astrocyte marker) expression at day 20 was observed across all conditions. Signal transducers and activator of transcription 3 (STAT3) is a transcription factor required for astrocyte differentiation and promotes GFAP gene expression [60-62]. In this signaling pathway, Olig2 inhibits STAT3 [63, 64]. GS may allow for a high expression of Olig2 that prevents differentiation into astrocytes, enhancing the efficiency of dopaminergic neuron differentiation later. Similarly, the increase in efficiency of dopaminergic differentiation reported by Gonzalez *et al.* and the increase in early neurite growth shown herein may be due to the inhibition of the STAT3 pathway by GS causing a decrease in astrocyte production and an increase in neuron yield [22].

GS may not promote a specific pathway that directs differentiation to a dopaminergic fate but may inhibit a pathway that directs the differentiation to another cell type. STAT3 is also implicated in the growth of tumor cells and is constitutively active in in many human cancer cells [67]. GS has been studied as a drug for the treating cancer, and evidence suggests that it does so by inhibiting STAT3 signaling [68]. Here, high Olig2 expression in the GS -releasing microsphere condition supports a mechanism of GS inhibiting STAT3 and thus astrocyte differentiation through Olig2.

References

1. Dauer, W. and S. Przedborski, Neuron, 2003. 39(6): p. 889-909.
2. Hegarty et al., Dev Biol, 2013. 379(2): p. 123-38.
3. Fahn, S., Mov Disord, 2015. 30(1): p. 4-18.
4. Jankovic, J. and L.G. Aguilar, Neuropsychiatr Dis Treat, 2008. 4(4): p. 743-57.
5. Miyasaki, J.M., Continuum (Minneap Minn), 2016. 22(4 Movement Disorders): p. 1104-16.
6. Stoker, T.B. and R.A. Barker, Regen Med, 2016. 11(8): p. 777-786.
7. Weinberger, L. et al., Nat Rev Mol Cell Biol, 2016. 17(3): p. 155-69.
8. Takahashi, K. and S. Yamanaka, Cell, 2006. 126(4): p. 663-76.
9. Takahashi, K. et al., Cell, 2007. 131(5): p. 861-72.
10. Thomson, J.A. et al.,. Science, 1998. 282(5391): p. 1145-7.
11. Carpenedo, R.L. et al., Biomaterials, 2009. 30(13): p. 2507-15.
12. Lake, J. et al., J Cell Sci, 2000. 113 (Pt 3): p. 555-66.
13. Martinez, S. et al., Development, 1999. 126(6): p. 1189-200.
14. Chi, C.L. et al., Development, 2003. 130(12): p. 2633-44.
15. Fasano, C.A. et al., Cell Stem Cell, 2010. 6(4): p. 336-47.
16. Basson, M.A. et al., Development, 2008. 135(5): p. 889-98.
17. Crossley et al., Nature, 1996. 380(6569): p. 66-8.
18. Briscoe, J., Nat Chem Biol, 2006. 2(1): p. 10-1.
19. Arenas et al., Development, 2015. 142(11): p. 1918-36.
20. Kriks, S. et al., Nature, 2011. 480(7378): p. 547-51.
21. Kirkeby, A. et al., Cell Rep, 2012. 1(6): p. 703-14.
22. Gonzalez, R. et al., Sci Rep, 2013. 3: p. 1463.
23. Robinson, M. et al., Biomarker Insights, 2015(Supplementary Video 20064): p. 61-70.
24. Bratt-Leal et al., Biotechnol Prog, 2009. 25(1): p. 43-51.
25. Niederreither, K. et al., Development, 2000. 127(1): p. 75-85.
26. Corson, L.B. et al., Development, 2003. 130(19): p. 4527-37.

27. Brennan, J. et al., Nature, 2001. 411(6840): p. 965-9.
28. Van Winkle et al., Cells Tissues Organs, 2012. 196(1): p. 34-47.
29. Sachlos, E. and D.T. Auguste, Biomaterials, 2008. 29(34): p. 4471-80.
30. Bratt-Leal, A.M. et al., Biomaterials, 2011. 32(1): p. 48-56.
31. Bratt-Leal, A.M. et al., Biomaterials, 2013. 34(30): p. 7227-35.
32. Carpenedo et al., J Biomed Mater Res A, 2010. 94(2): p. 466-75.
33. Lim, J.J. et al., Acta Biomater, 2011. 7(3): p. 986-95.
34. Wang, Y. et al., Acta Biomater, 2016. 29: p. 42-51.
35. Varde, N.K. and D.W. Pack, Expert Opin Biol Ther, 2004. 4(1): p. 35-51.
36. Yang et al., Biomaterials, 2001. 22(3): p. 231-241.
37. Coccoli et al., J Mater Sci Mater Med, 2008. 19(4): p. 1703-11.
38. Sinha, V.R. et al., Int J Pharm, 2004. 278(1): p. 1-23.
39. Woodruff and Hutmacher, Progress in Polymer Science, 2010. 35(10): p. 1217-1256.
40. Gomez, J.C. et al., Cellular and Molecular Bioengineering, 2015. 8(3): p. 307-319.
41. Qutachi et al., J Control Release, 2013. 168(1): p. 18-27.
42. Agbay et al., Drug Deliv Transl Res, 2014. 4(2): p. 159-70.
43. Freiberg, S. and X. Zhu, International Journal of Pharmaceutics, 2004. 282(1-2): p. 1-18.
44. Giunchedi et al., J Microencapsul, 1998. 15(2): p. 185-95.
45. Zhao, Adv Drug Deliv Rev, 2013. 65(11-12): p. 1420-46.
46. Dutta, D. et al., J Biomed Mater Res A, 2015.
47. Jeong, Y.I. et al., Int J Pharm, 2003. 259(1-2): p. 79-91.
48. Beck, L.R. et al., Adv Contracept, 1985. 1(2): p. 119-29.
49. Ferreira, L. et al., Advanced Materials, 2008. 20(12): p. 2285-2291.
50. Agrawal, H. et al., J Pharm Biomed Anal, 2004. 36(1): p. 23-31.
51. Atyabi, S.M. et al., Cell Biochem Biophys, 2016. 74(2): p. 181-90.
52. Chung, T.W. et al., J Mater Sci Mater Med, 2009. 20(1): p. 397-404.
53. Yildirim, E.D. et al., Biofabrication, 2010. 2(1): p. 014109.
54. Bramfeldt, H. and P. Vermette, J Biomed Mater Res A, 2009. 88(2): p. 520-30.
55. Recek, N. et al., International Journal of Polymer Science, 2016. 2016: p. 9.
56. Mohtaram, N.K. et al., Biomed Mater Res A, 2014.
57. Abbasi, N. et al., J Biomed Mater Res A, 2015.
58. Jakobsson, A. et al., Nanomedicine, 2017. 13(4): p. 1563-1573.
59. Li, B. et al., Biomaterials, 2005. 26(24): p. 4956-63.
60. Wen et al., Cell Adh Migr, 2009. 3(1): p. 107-17.
61. Hong, S. and M.R. Song, PLoS One, 2014. 9(1): p. e86851.
62. Cheng, P.Y. et al., PLoS One, 2011. 6(7): p. e22018.
63. Fukuda et al., Cell Death Differ, 2004. 11(2): p. 196-202.
64. Kageyama et al., Crit Rev Neurobiol, 1995. 9(2-3): p. 177-88.
65. Castelo-Branco et al., J Cell Sci, 2004. 117(Pt 24): p. 5731-7.
66. Castelo-Branco et al., Proc Natl Acad Sci USA, 2003. 100(22): p. 12747-52.
67. Buettner et al., Clin Cancer Res, 2002. 8(4): p. 945-54.
68. Ahn et al., Cancer Res, 2008. 68(11): p. 4406-15.

**Example 3: Engineering neural tissue from human pluripotent stem cells using small molecule-releasing microspheres and 3D bioprinting**

As demonstrated in Examples 1 and 2, drug-loaded microspheres can promote functional neural differentiation and maturation from human induced pluripotent stem cells (hiPSCs). Shown in this example, 3D constructs (non-3D bioprinted) with neural progenitor cells exposed to neural induction media on a 3D set-up differentiated slower than 2D cultures [2]. **FIG. 27A** shows expression of nestin (neural stem cell marker) and beta tubulin-III (βT-III, neuronal marker) for cells cultured in Fibrin scaffolds, whereas cells cultured in 2D only express βT-III **(FIG. 27B)**. These results indicate that cells in 3D culture remain in a progenitor state. Scaffold geometry and thickness can be considered when 3D bioprinting neural progenitor cells, as cells tend to differentiate variously on a thick vs thinner scaffold (FIGS. 28A-128D vs **FIG. 29A**). This could be due to the inability of compounds to penetrate the scaffold and reach the cells within the inner-most part of the 3D bioprinted construct. This was also demonstrated when these 3D constructs were exposed to toxic compounds for 7 days starting at day 14 and showed very similar behavior to the control sample with no toxic compound by day 21 **(FIGS. 29A-29C)**.

These results explain the need for a technology that can allow the exposure of cells to morphogens and factors

in order to promote survival and differentiation. Additionally, neural differentiation varies from weeks to months in order to achieve the desired maturation and functionality. To facilitate this state, the cells are exposed to morphogens for extended periods of time.

The disclosed encapsulated drug-loaded microspheres solve this problem. By combining the cells with the microspheres as herein described and with bioink, cell differentiation can be achieved in a high throughput manner. The source of morphogens for differentiation can be provided from the inner-most part of the 3D construct, instead ineffective penetration of the morphogens into the scaffold. In some examples, the source of morphogens can also include the outer layers of the 3D construct for even distribution.

The aforementioned bioink was combined with drug-loaded microspheres. hiPSCs were cultured for 56 days and at four different time points performed immunocytochemistry to compare differentiation profiles with the previously obtained in 2D. Six groups were tested: soluble Purmorphamine (Puro)/Retinoic acid (RA), Puro/RA microspheres, soluble Puro/Guggulsterone (GS), and PURO/GS microspheres, untreated cells, and cell aggregates combined with blank microspheres.

At day 15, ICC was performed, and all groups were labeled with SSEA-4 (green, pluripotency marker), βT-III (red), and the nuclear stain DAPI (blue) (**FIGS. 31A** and **31E**). Samples with soluble Puro/RA showed higher expression of SSEA-4 in outside layers compared to Puro/RA-loaded microspheres. Thus, more pluripotent cells remained at this time point, whereas the microsphere group exhibited a more even combination of SSEA-4 and βT-III. On day 28, all groups were stained for OLIG-2 (blue, oligodendrocyte marker), HB9 (red, motor neuron marker), and NeuN (green, neuronal nuclei marker) (**FIGS. 31B** and **31F**). Cells with soluble Puro/RA showed higher expression of NeuN, which is a neural nuclei marker, along with OLIG2. However, the puro/RA microsphere group showed higher OLIG2 expression and some HB9 expression. These results correlate with the motor neuron differentiation protocol performed by Hu et al. [3].

At day 35, differentiated cells were stained with HB9 (red), βT-III (green), and OLIG2 (blue) (**FIGS. 31C** and **31G**). The group with soluble puro/RA showed co-expression of βT-III and OLIG2 along with some HB9 expression. The puro/RA microsphere group also showed co-expression of βT-III and OLIG2 and no HB9. By day 56, the fibrin gels remained, encapsulating the cell aggregates. Immunostaining was performed with βT-III (green), ChAT (blue, acetylcholine transferase), and synapsin I (red, protein expressed in the synaptic vesicles required for neurotransmitter release) (**FIGS. 31D** and **31H**). The group with the soluble drug showed increased expression of both βT-III and CHaT; co-expression is shown as yellow. Microsphere-treated groups showed increased expression of ChaT, indicating cholinergic neuron differentiation by day 56 (FIG. 30H).

The combinations of soluble puro and GS as well as puro/GS-loaded microspheres were analyzed. By day 15, the cultures were stained for SSEA-4 (green), βT-III (red), and DAPI (blue) **(FIGS. 32A** and **32C)**. Samples with the soluble drug in the media showed higher SSEA-4 expression comparison to the puro/GS microspheres. The microsphere group also showed βT-III expression, indicating more mature differentiation at this time point. By day 28, samples were stained for OLIG-2 (blue), HB9 (red), and NeuN (green) **(FIGS. 32B** and **32D)**. The drug-loaded microsphere group showed increased expression of OLIG2, whereas the soluble drug group expressed both HB9 and OLIG2.

Fibrin scaffolds containing the drug GS degraded much faster than samples containing RA. Smaller sizes of cell aggregates are generated either by using less number of cells when using Aggrewell™800 or by using Aggrewell™400 in order to generate aggregates combined with drug releasing microspheres of a 400-600 μm diameter and allow a proper flow and extrusion from the microfluidic print head fabricated by Aspect Biosystems. Alternatively, a single cell suspension of either hiPSCs or neural progenitor cells and drug releasing microspheres are delivered from one of the print head channels. The single cell plus microspheres suspension or cell aggregates combined with drug loaded microspheres are then combined with the bioink on the microfluidic device in order to generate 3D constructs. These cultures are then maintained for several weeks to allow maturation and the formation of functional neural tissues.

References

1. Hospodiuk et al., Biotechnology Advances, 2017. 35(2): 217-239.
2. Abelseth, L., Cytotoxic response of neural progenitor cells in 3D fibrin scaffolds, U. Victoria, Editor. 2017.
3. Hu, B.-Y. and S.-C. Zhang, Nature protocols, 2009. 4(9): 1295-1304.


**Example 4: Cell aggregates with microspheres formed using human induced pluripotent stem cells (hiPSCs) and then incorporated into bioink on day 7**

Overview

Cell aggregates with or without microspheres are formed using human induced pluripotent stem cells (hiPSCs)

and then incorporated into bioink on day 7.

Formation and culture of cell aggregates

Cell aggregates containing microspheres are formed using hiPSCs. Cell aggregates are formed on day 0 using AggreWell 800 plate (STEMCELL Technologies) and cultured in neural induction medium (NIM) and 1% Penstrep (PS) for 7 days. In some groups, the cell aggregates are supplemented with or without the small molecules CHIR99021 (CHIR), DMH-1, and SB431542 (SB). Some groups contain purmorphamine (Puro) and retinoic acid (RA) from day 7 with or without the small molecules (CHIR, DMH-1, and SB). Some groups include cell aggregates formed with unloaded microspheres from day 0 with or without the small molecules (CHIR, DMH-1, and SB). Some groups contain puro and RA loaded microspheres from day 0 with or without the small molecules (CHIR, DMH-1, and SB).

Formation of Bioink

On day 7, cell aggregates are resuspended in a bioink solution composed of 20 mg/mL of fibrinogen (e.g., 341578, Sigma, St. Louis, MO, USA), 0.5% w/ v of alginate (120,000-190,000 g/mol, M/G ratio 1.56) (e.g., 180947, Sigma, St. Louis, MO, USA), and 0.3 mg/mL of genipin dissolved in de-ionized water (e.g., G4796, Sigma, St. Louis, MO, USA) in tris-buffered saline). All the procedures are performed under sterile conditions. The bioink can next be loaded into a bioprinter (following manufacturer's directions) for bioprinting functional tissues.

In view of the many possible embodiments to which the principles of the disclosure may be applied, it should be recognized that the illustrated embodiments are only examples of the invention and should not be taken as limiting the scope of the invention. Rather, the scope of the invention is defined by the following claims.

## Claims

**1.** A bioink, comprising:

one or more cells;
a carrier material; and
discrete microspheres, the discrete microspheres comprising:

one or more biodegradable polymers; and
one or more morphogenic compounds,
wherein the one or more compounds release with degradation of the one or more biodegradable polymers.

**2.** The bioink of claim 1, wherein one or more cells comprise cell aggregates.

**3.** The bioink of claim 1 or claim 2, wherein one or more cells comprise stem cells or progenitor cells, and wherein the stem cells are optionally selected from induced pluripotent stem cells (iPSCs) or neural stem cells, optionally wherein the iPSCs are human iPSCs (hiPSCs).

**4.** The bioink of claim 3, wherein the progenitor cells comprise neural progenitor cells.

**5.** The bioink of any one of claims 1 to 4, wherein the one or more morphogenic compounds comprise one or more small-molecule morphogen mimetics, morphogens, or any combination thereof.

**6.** The bioink of claim 5, wherein:

(a) the one or more small-molecule morphogen mimetics comprise purmorphamine (puro), guggulsterone (GS), or both; and/or
(b) the one or more morphogens comprise one of neurotrophic factors (NTFs), retinoic acid (RA), insulin-like growth factor 1 (IGF-1), wherein the NTFs optionally comprise glial cell line-derived neurotrophic factor (GDNF), brain-derived neurotrophic factor (BDNF), or both.

**7.** The bioink according to claim 1 wherein the one or more cells comprise cell aggregates containing microspheres formed using hiPSCs and wherein the ink further comprises small molecules selected from the group consisting of CHIR99021 (CHIR), DMH-1, and SB431542.

**8.** The bioink of any one of claims 1-7, wherein the one or more biodegradable polymers comprise polylactic acid (PLA), polyglycolic acid (PLGA), polycaprolactone (PCL), or any combination thereof.

**9.** The bioink of any one of claims 1-8, wherein the carrier material comprises: one or more polymers, crosslinkers, hydrogels, or any combination thereof, wherein the carrier material is optionally a hydrogel, and wherein the hydrogel optionally comprises methacrylamide chitosan (MAC), fibrin, alginate, or any combination thereof.

**10.** An ex vivo method of three-dimensional bioprinting, comprising bioprinting at least one layer of the bioink of any of claims 1 to 9 onto a surface.

**11.** An ex-vivo method of producing functional tissue, comprising:

bioprinting at least one layer of the bioink of any of claims 1 to 9 onto a surface;
measuring one or more markers of differentiation or maturation; and
detecting a level of expression of one or more markers of differentiation or maturation expected in a functional tissue.

**12.** The method of claim 10 or claim 11, wherein the bioprinting comprises bioprinting using an extrusion-based, droplet-based and laser based bioprinter.

**13.** The method of any one of claims 10-12, wherein the bioprinting comprising bioprinting using a inkjet bioprinter.

**14.** The method of any one of claims 11-13, wherein:

(a) the functional tissue is neural tissue;
(b) the one or more markers comprise nestin, beta-tubulin III, stage-specific embryonic antigen-4 (SSEA-4), NeuN, oligodendrocyte transcription factor (OLIG2), homeobox HB9 (HB9), paired box protein (PAX6), synapsin I, SRY-box 2 (SOX-2), insulin gene enhancer protein (ISL-1), anti-oligodendrocyte 04 (04), choline acetyltransferase (ChAT), synaptophysin, Oct3/4, c-Myc, kruppel-like factor 4 (KLF4), tyrosine hydroxylase, or neurites; and/or
(c) detecting the level of expression of one or more markers of differentiation or maturation expected in a functional tissue comprises detecting:

increased expression of ChAT, 04, NeuN, beta-tubulin III, HB9, synaptophysin, tyrosine hydroxylase, or neurites compared with expression expected in a pluripotent stem cell; or
decreased expression of SSEA-4, Sox-2, Olig2, Isl-1, synapsin I, Oct3/4, c-myc, nestin, PAX6, or KLF4 compared with expression expected in a pluripotent stem cell.

**15.** An ex-vivo method of bioprinting comprising:

contacting at least one cell type with a microsphere solution and a bioink solution comprising one or more carrier materials, producing a bioink-cell-microsphere composition;
contacting the bioink-cell composition with a crosslinker, producing a crosslinked bioink-cell composition; and
bioprinting at least one layer of the crosslinked bioink-cell composition using a bioprinter wherein the microsphere solution comprises discrete microspheres, the discrete microspheres comprising: one or more biodegradable polymers, and one or more morphogenic compounds that release with degradation of the one or more biodegradable polymers.

**16.** The method of claim 15, wherein:

(a) the bioink solution comprises fibrinogen, alginate, and genipin;
(b) the crosslinker comprises $CaCl_2$, chitosan, and thrombin;
(c) the at least one cell type forms cell aggregates; and/or
(d) the at least one cell type comprises neuronal cells, stem cells, stem cell-derived cells, human induced pluripotent stem cells (hiPSCs), and/or cancer cells.

**17.** A method of fabricating bioink wherein said method comprises incorporating microspheres comprising one or more biodegradable polymers;

one or more morphogenic compounds,
wherein the one or more compounds release with degradation of the one or more biodegradable polymers and a carrier material into a bioink.

**18.** The bioink of any of claims 1 to 9, wherein the one or more cells are combined with a carrier material and the discrete microspheres in the print head of a bioprinter; and wherein the one or more cells are cell aggregates or a single cell suspension of hiPSCs or neural progenitor cells and the microspheres are drug releasing microspheres.

**Patentansprüche**

**1.** Biotinte, umfassend:

eine oder mehrere Zellen;
ein Trägermaterial; und
separate Mikrokugeln, wobei die separaten Mikrokugeln Folgendes umfassen:

ein oder mehrere biologisch abbaubare Polymere; und
eine oder mehrere morphogene Verbindungen,
wobei die eine oder mehreren Verbindungen beim Abbau des einen oder der mehreren biologisch abbaubaren Polymere freigegeben werden.

**2.** Biotinte nach Anspruch 1, wobei die eine oder mehreren Zellen Zellaggregate umfassen.

**3.** Biotinte nach Anspruch 1 oder Anspruch 2, wobei die eine oder mehreren Zellen Stammzellen oder Vorläuferzellen umfassen und wobei die Stammzellen optional aus induzierten pluripotenten Stammzellen (iPSCs) oder neuralen Stammzellen ausgewählt sind, wobei die iPSCs optional humane iPSCs (hiPSCs) sind.

**4.** Biotinte nach Anspruch 3, wobei die Vorläuferzellen neurale Vorläuferzellen umfassen.

**5.** Biotinte nach einem der Ansprüche 1 bis 4, wobei die eine oder mehrere morphogenen Verbindungen ein oder mehrere niedermolekulare Morphogenmimetika, Morphogene oder eine beliebige Kombination davon umfassen.

**6.** Biotinte nach Anspruch 5, wobei:

(a) das eine oder die mehreren niedermolekularen Morphogenmimetika Purmorphamin (Puro), Guggulsteron (GS) oder beides umfassen; und/oder
(b) das eine oder die mehreren Morphogene eines von neurotrophenen Faktoren (NTFs), Retinsäure (RA), einem insulinähnlichen Wachstumsfaktor 1 (IGF-1) umfassen, wobei die NTFs optional einen aus einer Gliazelllinie gewonnenen neurotrophenen Faktor (GDNF), einen aus dem Gehirn gewonnenen neurotrophenen Faktor (BDNF) oder beide umfassen.

**7.** Biotinte nach Anspruch 1, wobei die eine oder mehreren Zellen Zellaggregate umfassen, die unter Verwendung von hiPSCs gebildete Mikrokugeln enthalten, und wobei die Tinte ferner kleine Moleküle umfasst, die aus der Gruppe ausgewählt sind, die aus CHIR99021 (CHIR), DMH-1 und SB431542 besteht.

**8.** Biotinte nach einem der Ansprüche 1-7, wobei das eine oder die mehreren biologisch abbaubaren Polymere Polymilchsäure (PLA), Polyglykolsäure (PLGA), Polycaprolacton (PCL) oder eine beliebige Kombination davon umfassen.

**9.** Biotinte nach einem der Ansprüche 1-8, wobei das Trägermaterial Folgendes umfasst: ein oder mehrere Polymere, Vernetzer, Hydrogele oder eine beliebige Kombination davon, wobei das Trägermaterial optional ein Hydrogel ist und wobei das Hydrogel optional Methacrylamidchitosan (MAC), Fibrin, Alginat oder eine beliebige Kombination davon umfasst.

**10.** Ex-vivo-Verfahren zum dreidimensionalen Biodrucken, umfassend Biodrucken von mindestens einer Schicht der Biotinte nach einem der Ansprüche 1 bis 9 auf eine Oberfläche.

**11.** Ex-vivo-Verfahren zum Herstellen von Funktionsgewebe, umfassend:

Biodrucken von mindestens einer Schicht der Biotinte nach einem der Ansprüche 1 bis 9 auf eine Oberfläche;
Messen eines oder mehrerer Differenzierungs- oder Reifungsmarker; und
Detektieren eines Expressionsniveaus eines oder mehrerer Differenzierungs- oder Reifungsmarker, die in einem Funktionsgewebe erwartet werden.

**12.** Verfahren nach Anspruch 10 oder Anspruch 11, wobei das Biodrucken Biodrucken unter Verwendung eines extrusionsbasierten, tröpfchenbasierten und laserbasierten Biodruckers umfasst.

**13.** Verfahren nach einem der Ansprüche 10-12, wobei das Biodrucken Biodrucken unter Verwendung eines Tintenstrahl-Biodruckers umfasst.

**14.** Verfahren nach einem der Ansprüche 11-13, wobei:

(a) das Funktionsgewebe ein Nervengewebe ist;
(b) der eine oder die mehreren Marker Folgendes umfassen, Nestin, Beta-Tubulin III, das stufenspezifische embryonale Antigen-4 (SSEA-4), NeuN, einen Oligodendrozyten-Transkriptionsfaktor (OLIG2), eine Homeobox HB9 (HB9), ein Paired-Box-Protein (PAX6), Synapsin I, eine SRY-Box 2 (SOX-2), ein Insulin-Gen-Enhancer-Protein (ISL-1), den Anti-Oligodendrozyten 04 (04), eine Cholin-Acetyltransferase (ChAT), Synaptophysin, Oct3/4, c-Myc, einen kruppelähnlichen Faktor 4 (KLF4), Tyrosinhydroxylase oder Neuriten; und/oder
(c) das Detektieren des Expressionsniveaus eines oder mehrerer Differenzierungs- oder Reifungsmarker, die in einem Funktionsgewebe erwartet werden, Detektieren von Folgendem umfasst:

einer erhöhte Expression von ChAT, 04, NeuN, Beta-Tubulin III, HB9, Synaptophysin, Tyrosinhydroxylase oder Neuriten im Vergleich zu einer erwarteten Expression in einer pluripotenten Stammzelle; oder
einer verringerten Expression von SSEA-4, Sox-2, Olig2, Isl-1, Synapsin I, Oct3/4, c-myc, Nestin, PAX6 oder KLF4 im Vergleich zu einer erwarteten Expression in einer pluripotenten Stammzelle.

**15.** Ex-vivo-Verfahren zum Biodrucken, umfassend:

Kontaktieren von mindestens einem Zelltyp mit einer Mikrokugellösung und einer Biotintenlösung, die ein oder mehrere Trägermaterialien umfasst, wodurch eine Biotinten-Zell-Mikrokugel-Zusammensetzung hergestellt wird;
Kontaktieren der Biotinten-Zell-Zusammensetzung mit einem Vernetzer, wodurch eine vernetzte Biotinten-Zell-Zusammensetzung hergestellt wird; und
Biodrucken von mindestens einer Schicht der vernetzten Biotinten-Zell-Zusammensetzung unter Verwendung eines Biodruckers, wobei die Mikrokugellösung separate Mikrokugeln umfasst und die separaten Mikrokugeln Folgendes umfassen: ein oder mehrere biologisch abbaubare Polymere und eine oder mehrere morphogene Verbindungen, die beim Abbau des einen oder der mehreren biologisch abbaubaren Polymere freigegeben werden.

**16.** Verfahren nach Anspruch 15, wobei:

(a) die Biotintenlösung Fibrinogen, Alginat und Genipin umfasst;
(b) der Vernetzer CaC12, Chitosan und Thrombin umfasst;
(c) der mindestens eine Zelltyp Zellaggregate bildet; und/oder
(d) der mindestens eine Zelltyp neuronale Zellen, Stammzellen, aus Stammzellen gewonnenen Zellen, humane induzierte pluripotente Stammzellen (hiPSCs) und/oder Krebszellen umfasst.

**17.** Verfahren zum Fertigen von Biotinte, wobei das Verfahren Einarbeiten von Mikrokugeln umfasst, die ein oder mehrere biologisch abbaubare Polymere umfassen;
eine oder mehrere morphogene Verbindungen, wobei die eine oder mehreren Verbindungen beim Abbau der einen oder mehreren biologisch abbaubaren Polymere und eines Trägermaterials in eine Biotinte freigegeben werden.

**18.** Biotinte nach einem der Ansprüche 1 bis 9, wobei die eine oder mehreren Zellen mit einem Trägermaterial und den separaten Mikrokugeln in dem Druckkopf eines Biodruckers kombiniert sind; und wobei die eine oder mehreren Zellen Zellaggregate oder eine Einzelzellsuspension von hiPSCs oder neuralen Vorläuferzellen sind und die Mikrokugeln Arzneimittel freigebende Mikrokugeln sind.

**Revendications**

1. Bioencre, comprenant :

   une ou plusieurs cellules ;
   un matériau support ; et
   des microsphères discrètes, les microsphères discrètes comprenant :

      un ou plusieurs polymères biodégradables ; et
      un ou plusieurs composés morphogéniques,
      dans lequel l'un ou plusieurs composés se libèrent avec la dégradation de l'un ou plusieurs polymères biodégradables.

2. Bioencre selon la revendication 1, dans laquelle une ou plusieurs cellules comprennent des agrégats cellulaires.

3. Bioencre selon la revendication 1 ou la revendication 2, dans laquelle une ou plusieurs cellules comprennent des cellules souches ou des cellules progénitrices, et dans laquelle les cellules souches sont facultativement sélectionnées parmi les cellules souches pluripotentes induites (iPSC) ou les cellules souches neuronales, facultativement dans laquelle les iPSC sont des iPSC humaines (hiPSC).

4. Bioencre selon la revendication 3, dans laquelle les cellules progénitrices comprennent des cellules progénitrices neuronales.

5. Bioencre selon l'une quelconque des revendications 1 à 4, dans laquelle l'un ou plusieurs composés morphogènes comprennent un ou plusieurs mimétiques morphogènes à petites molécules, des morphogènes, ou toute combinaison de ceux-ci.

6. Bioencre selon la revendication 5, dans laquelle :

   (a) l'un ou plusieurs mimétiques morphogènes à petites molécules comprennent de la purmorphamine (puro), de la guggulstérone (GS), ou les deux ; et/ou
   (b) l'un ou plusieurs morphogènes comprennent l'un des facteurs neurotrophiques (NTF), de l'acide rétinoïque (RA) et du facteur de croissance analogue à l'insuline 1 (IGF-1), dans lequel les NTF comprennent facultativement un facteur neurotrophique dérivé de lignées de cellules gliales (GDNF), un facteur neurotrophique dérivé du cerveau (BDNF), ou les deux.

7. Bioencre selon la revendication 1, dans laquelle l'une ou plusieurs cellules comprennent des agrégats cellulaires contenant des microsphères formées à l'aide d'hiPSC et dans laquelle l'encre comprend en outre de petites molécules sélectionnées dans le groupe constitué de CHIR99021 (CHIR), DMH-1 et SB431542.

8. Bioencre selon l'une quelconque des revendications 1 à 7, dans laquelle l'un ou plusieurs polymères biodégradables comprennent de l'acide polylactique (PLA), de l'acide polyglycolique (PLGA), de la polycaprolactone (PCL) ou toute combinaison de ceux-ci.

9. Bioencre selon l'une quelconque des revendications 1 à 8, dans laquelle le matériau support comprend : un ou plusieurs polymères, agents de réticulation, hydrogels, ou toute combinaison de ceux-ci, dans lequel le matériau support est facultativement un hydrogel, et dans lequel l'hydrogel comprend facultativement du méthacrylamide chitosane (MAC), de la fibrine, de l'alginate ou toute combinaison de ceux-ci.

10. Procédé ex vivo de bioimpression tridimensionnelle, comprenant la bioimpression d'au moins une couche de la bioencre selon l'une quelconque des revendications 1 à 9 sur une surface.

11. Procédé ex vivo de production de tissu fonctionnel, comprenant :

   la bioimpression d'au moins une couche de la bioencre selon l'une quelconque des revendications 1 à 9 sur

une surface ;
la mesure d'un ou plusieurs marqueurs de différenciation ou de maturation ; et
la détection d'un niveau d'expression d'un ou plusieurs marqueurs de différenciation ou de maturation attendus dans un tissu fonctionnel.

**12.** Procédé selon la revendication 10 ou la revendication 11, dans lequel la bioimpression comprend la bioimpression à l'aide d'une bioimprimante basée sur l'extrusion, basée sur les gouttelettes et basée sur un laser.

**13.** Procédé selon l'une quelconque des revendications 10 à 12, dans lequel la bioimpression comprend la bioimpression utilisant une bioimprimante à jet d'encre.

**14.** Procédé selon l'une quelconque des revendications 11 à 13, dans lequel :

(a) le tissu fonctionnel est un tissu neuronal ;
(b) l'un ou plusieurs marqueurs comprennent la nestine, la bêta-tubuline III, l'antigène embryonnaire spécifique de stade 4 (SSEA-4), NeuN, le facteur de transcription des oligodendrocytes (OLIG2), l'homéobox HB9 (HB9), la protéine à boîte appariée (PAX6), la synapsine I, SRY-box 2 (SOX-2), la protéine activatrice du gène de l'insuline (ISL-1), l'anti-oligodendrocytes 04 (04), la choline acétyltransférase (ChAT), la synaptophysine, l'Oct/3/4, c-Myc, le facteur 4 de type kruppel (KLF4), la tyrosine hydroxylase ou les neurites ; et/ou
(c) la détection du niveau d'expression d'un ou plusieurs marqueurs de différenciation ou de maturation attendus dans un tissu fonctionnel comprend la détection de :

l'expression accrue de ChAT, 04, NeuN, bêta-tubuline III, HB9, synaptophysine, tyrosine hydroxylase ou neurites par rapport à l'expression attendue dans une cellule souche pluripotente ; ou
la diminution de l'expression de SSEA-4, Sox-2, Olig2, Isl-1, synapsine I, Oct3/4, c-myc, nestin, PAX6 ou KLF4 par rapport à l'expression attendue dans une cellule souche pluripotente.

**15.** Procédé ex vivo de bioimpression comprenant :

la mise en contact d'au moins un type de cellule avec une solution de microsphères et une solution de bioencre comprenant un ou plusieurs matériaux support, la production d'une composition de microsphère de cellules de bioencre ;
la mise en contact de la composition de cellules de bioencre avec un agent de réticulation, produisant une composition de cellules de bioencre réticulées ; et
la bioimpression d'au moins une couche de la composition de cellules de bioencre réticulées à l'aide d'une bioimprimante, dans lequel la solution de microsphères comprend des microsphères discrètes, les microsphères discrètes comprenant : un ou plusieurs polymères biodégradables, et un ou plusieurs composés morphogéniques qui se libèrent avec la dégradation de l'un ou plusieurs polymères biodégradables.

**16.** Procédé selon la revendication 15, dans lequel :

(a) la solution de bioencre comprend du fibrinogène, de l'alginate et de la génipine ;
(b) l'agent de réticulation comprend du CaC12, du chitosane et de la thrombine ;
(c) l'au moins un type de cellules forme des agrégats cellulaires ; et/ou
(d) l'au moins un type de cellules comprend des cellules neuronales, des cellules souches, des cellules dérivées de cellules souches, des cellules souches pluripotentes induites par l'homme (hiPSC) et/ou des cellules cancéreuses.

**17.** Procédé de fabrication d'une bioencre, dans lequel ledit procédé comprend l'incorporation de microsphères comprenant un ou plusieurs polymères biodégradables ;

un ou plusieurs composés morphogéniques,
dans lequel l'un ou plusieurs composés se libèrent avec la dégradation de l'un ou plusieurs polymères biodégradables et d'un matériau support en une bioencre.

**18.** Bioencre selon l'une quelconque des revendications 1 à 9, dans laquelle l'une ou plusieurs cellules sont combinées avec un matériau support et les microsphères discrètes dans la tête d'impression d'une bioimprimante ; et dans lequel l'une ou plusieurs cellules sont des agrégats cellulaires ou une suspension cellulaire unique de hiPSC ou de

cellules progénitrices neuronales et les microsphères sont des microsphères libérant un médicament.

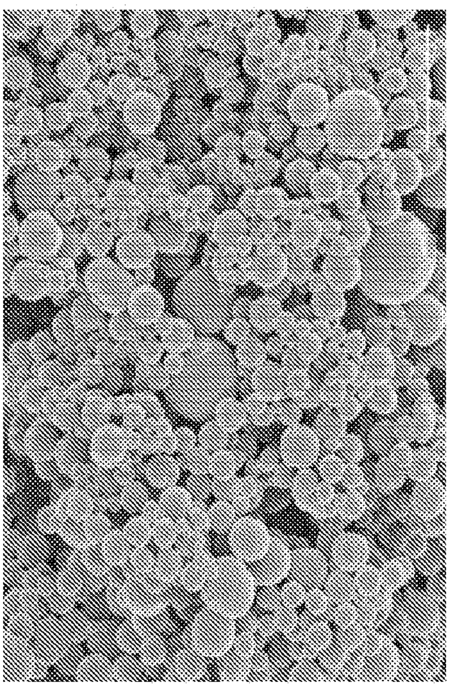

FIG. 1A

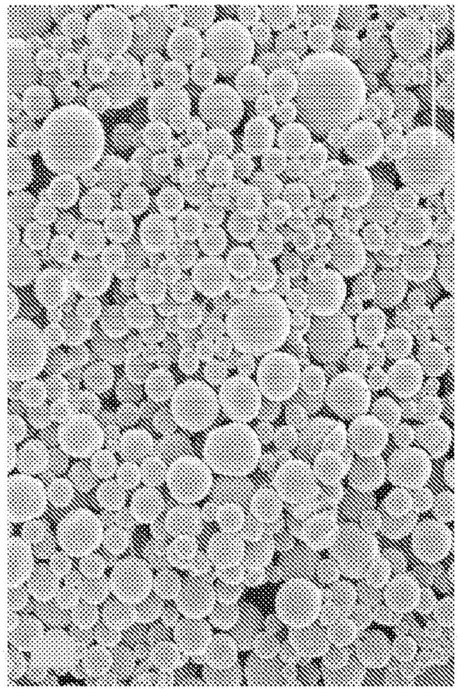

FIG. 1B

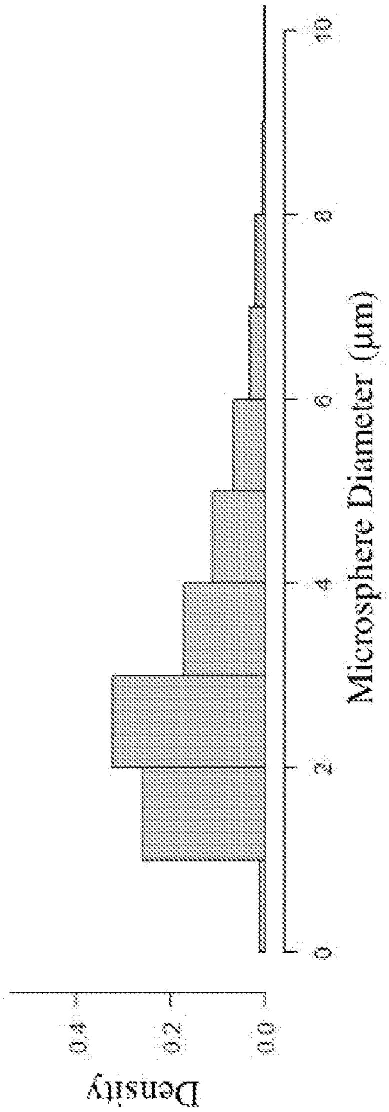


FIG. 1C

FIG. 1D

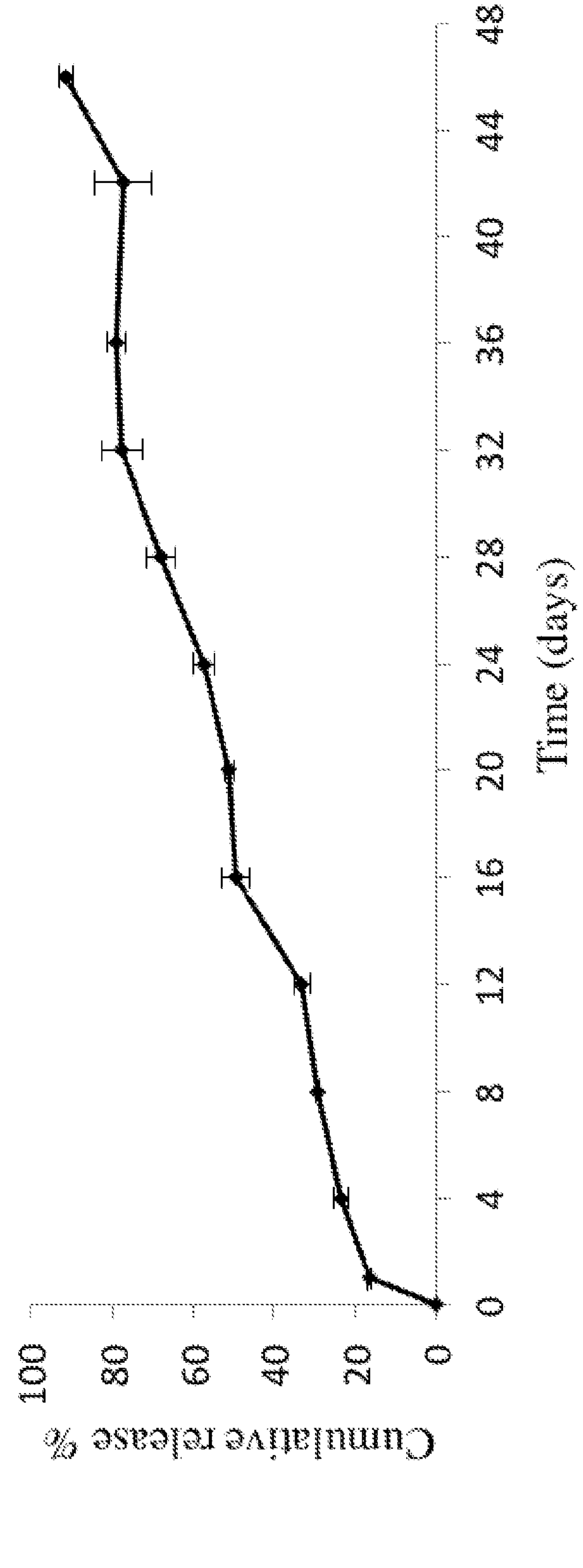
Cumulative release %
0
20
40
60
80
100
Time (days)
0
4
8
12
16
20
24
28
32
36
40
44
48

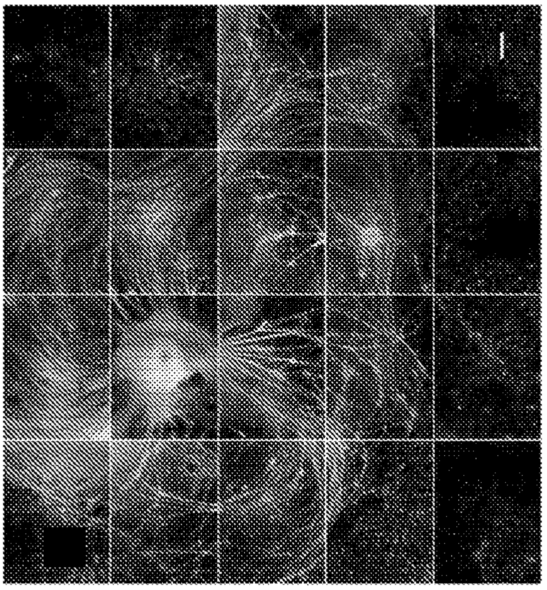
FIG. 2A

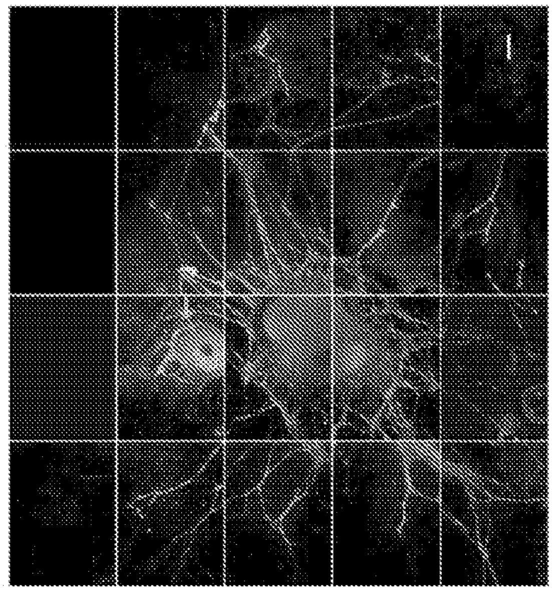
FIG. 2B

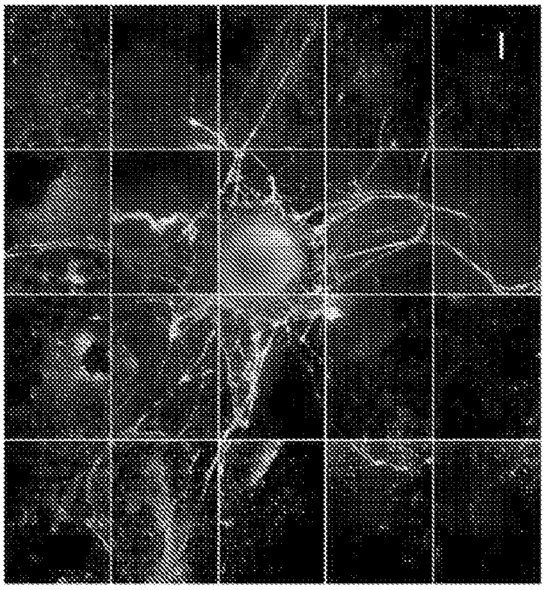
FIG. 2C

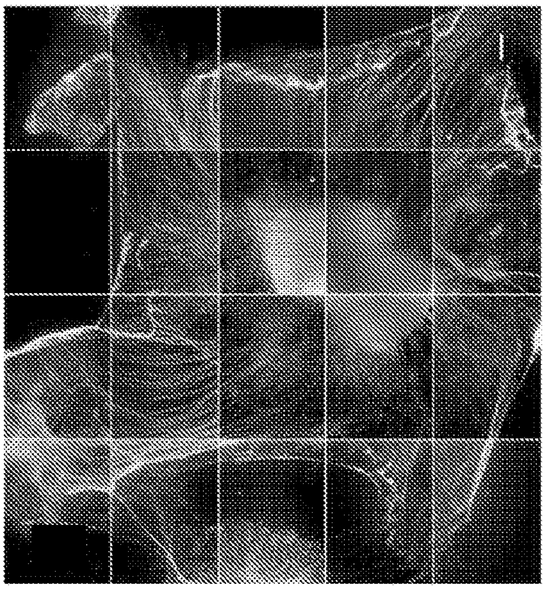
FIG. 2D

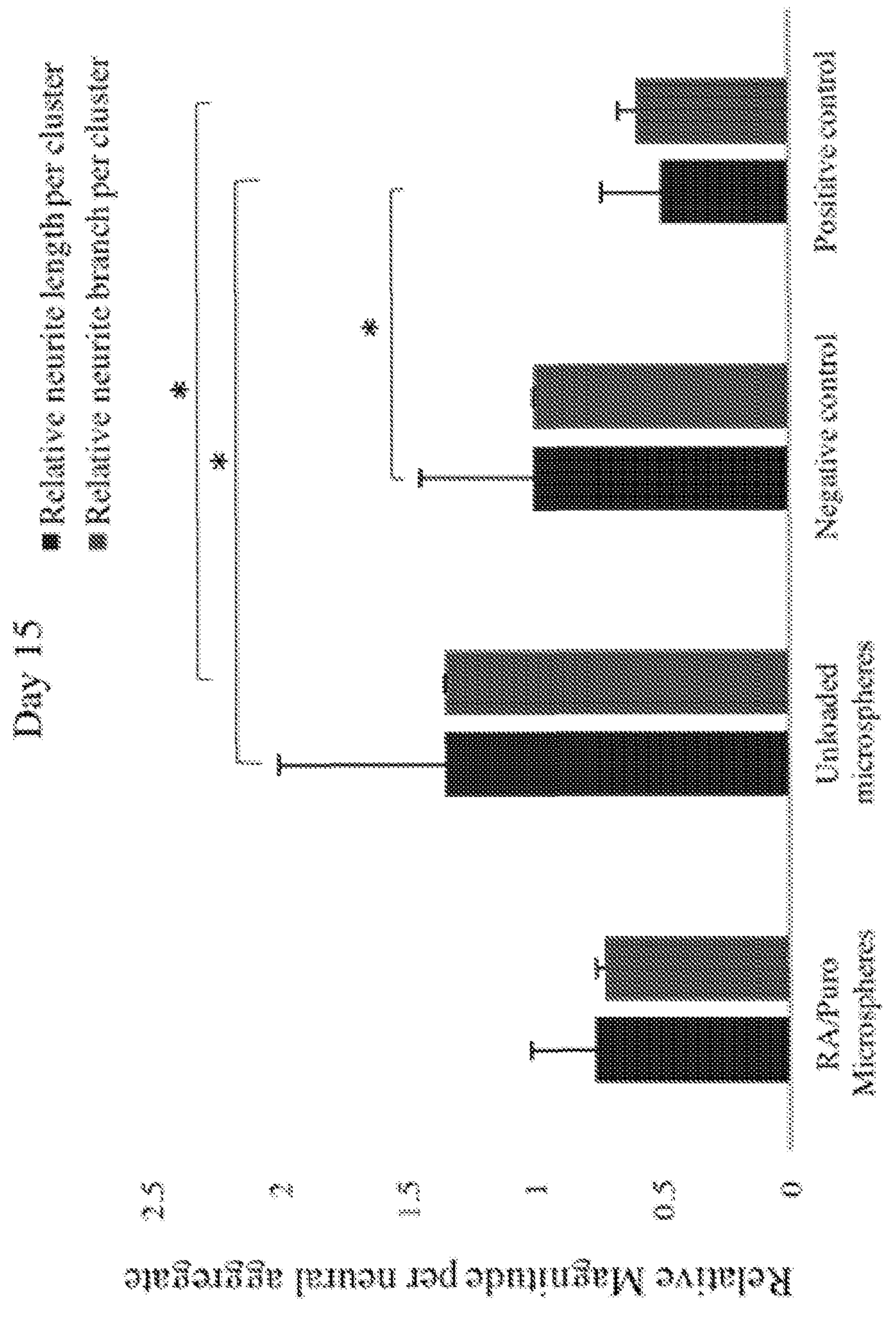
Day 15
Relative neurite length per cluster
Relative neurite branch per cluster
Relative Magnitude per neural aggregate
2.5
2
1.5
1
0.5
0
RA/Puro Microspheres
Unloaded microspheres
Negative control
Positive control
*
*
*

FIG. 3A

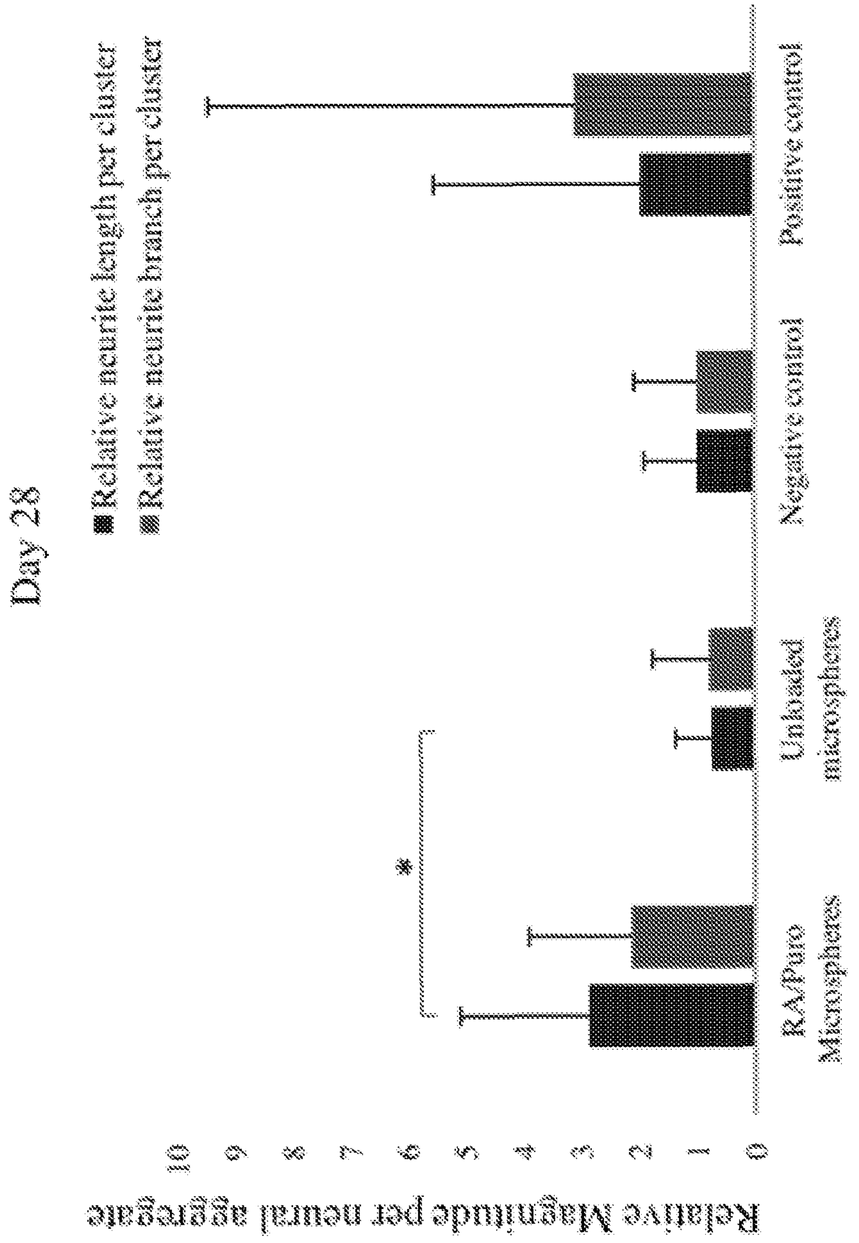
Day 28
Relative neurite length per cluster
Relative neurite branch per cluster
Relative Magnitude per neural aggregate
0
1
2
3
4
5
6
7
8
9
10
*
RA/Puro Microspheres
Unloaded microspheres
Negative control
Positive control

FIG. 3B

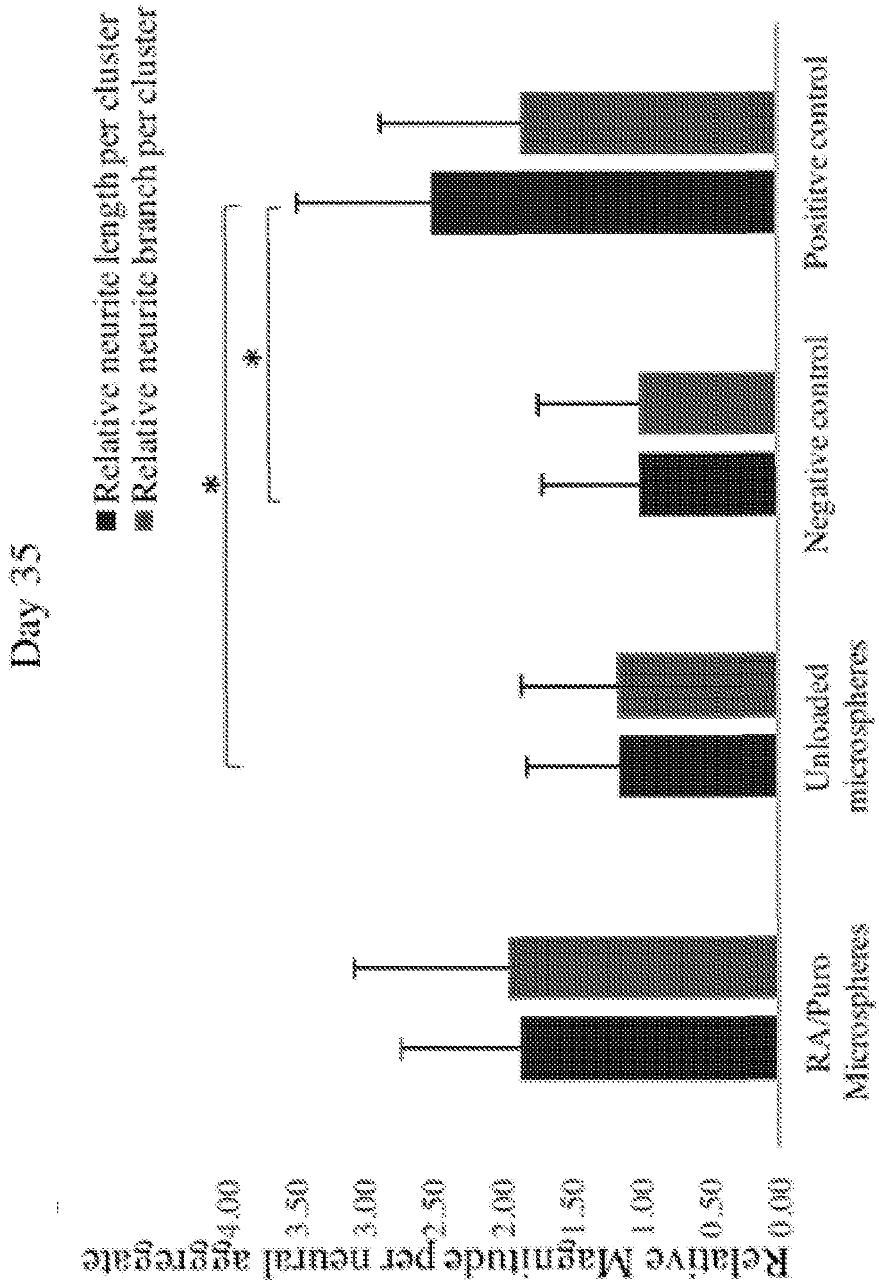
Day 35
Relative neurite length per cluster
Relative neurite branch per cluster
*
*
Relative Magnitude per neural aggregate
4.00
3.50
3.00
2.50
2.00
1.50
1.00
0.50
0.00
RA/Puro Microspheres
Unloaded microspheres
Negative control
Positive control

FIG. 3C

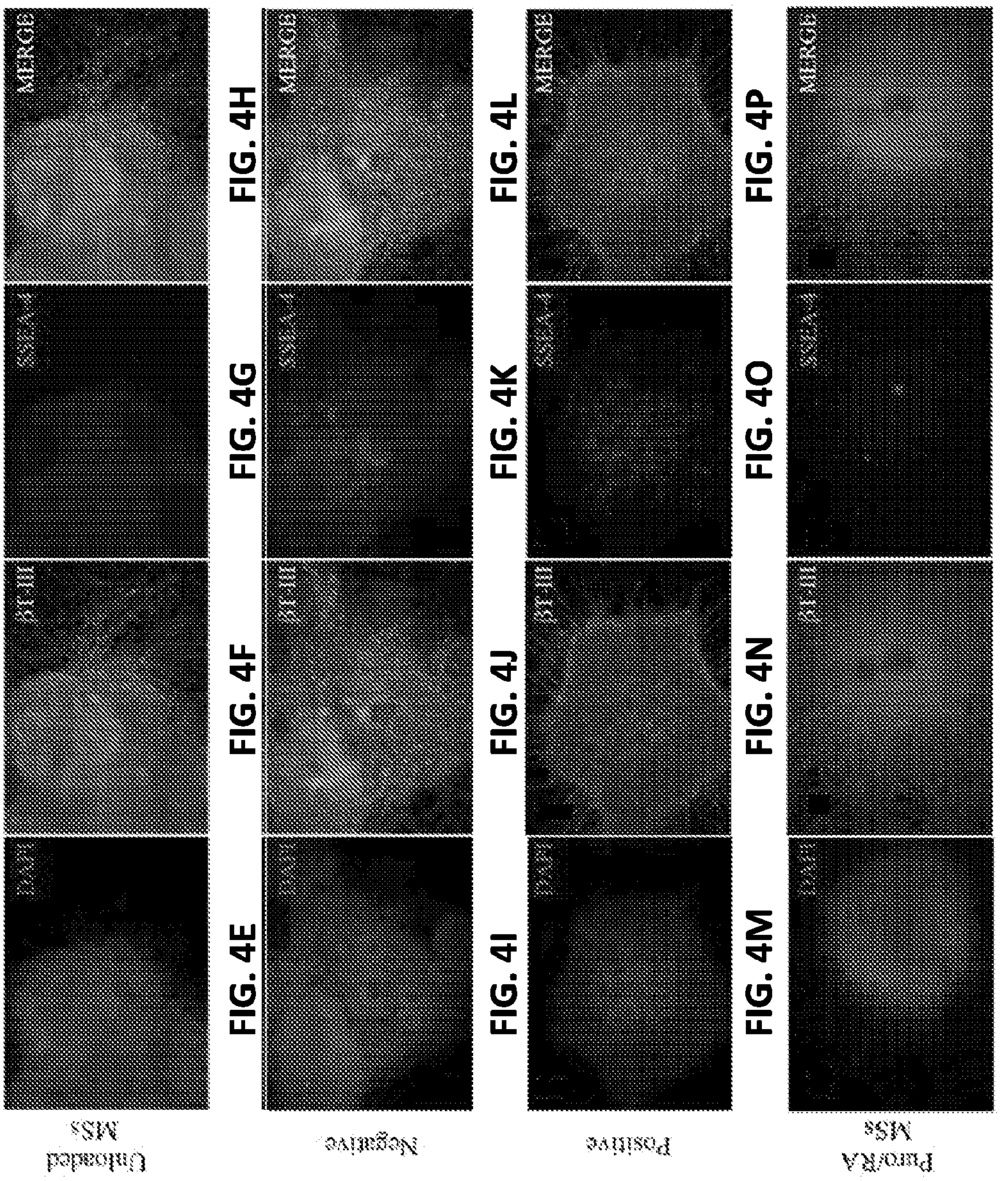


FIG. 4A FIG. 4B FIG. 4C FIG. 4D
FIG. 4E FIG. 4F FIG. 4G FIG. 4H
FIG. 4I FIG. 4J FIG. 4K FIG. 4L
FIG. 4M FIG. 4N FIG. 4O FIG. 4P

FIG. 4Q

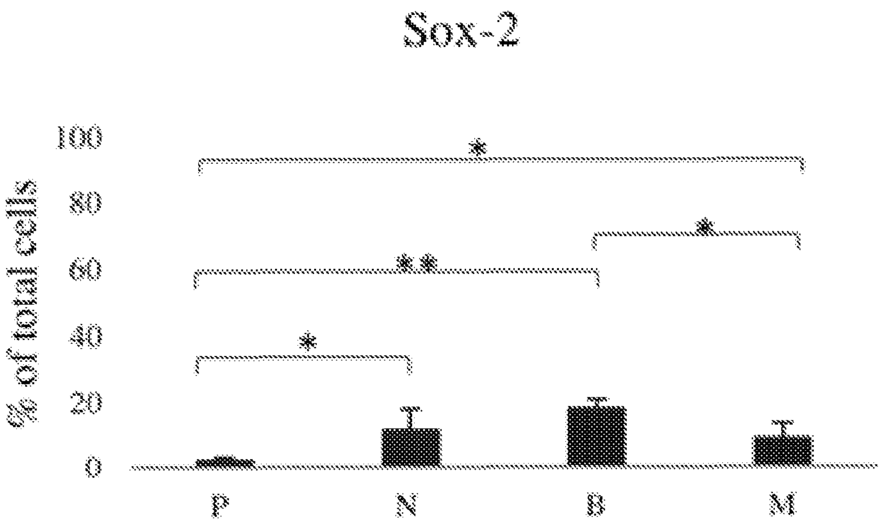


FIG. 4R

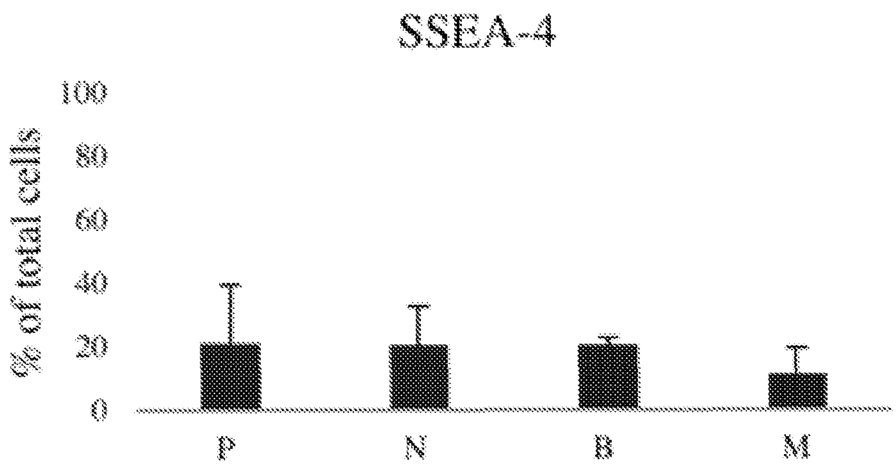


FIG. 4S

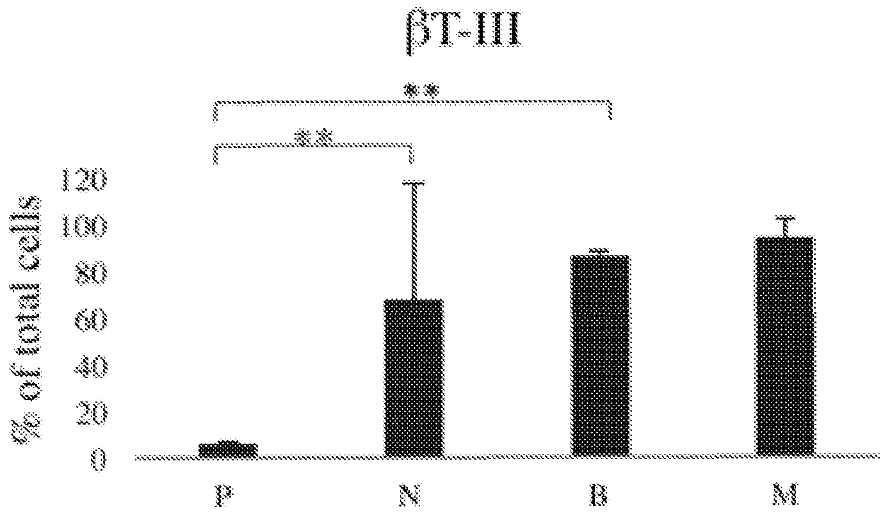

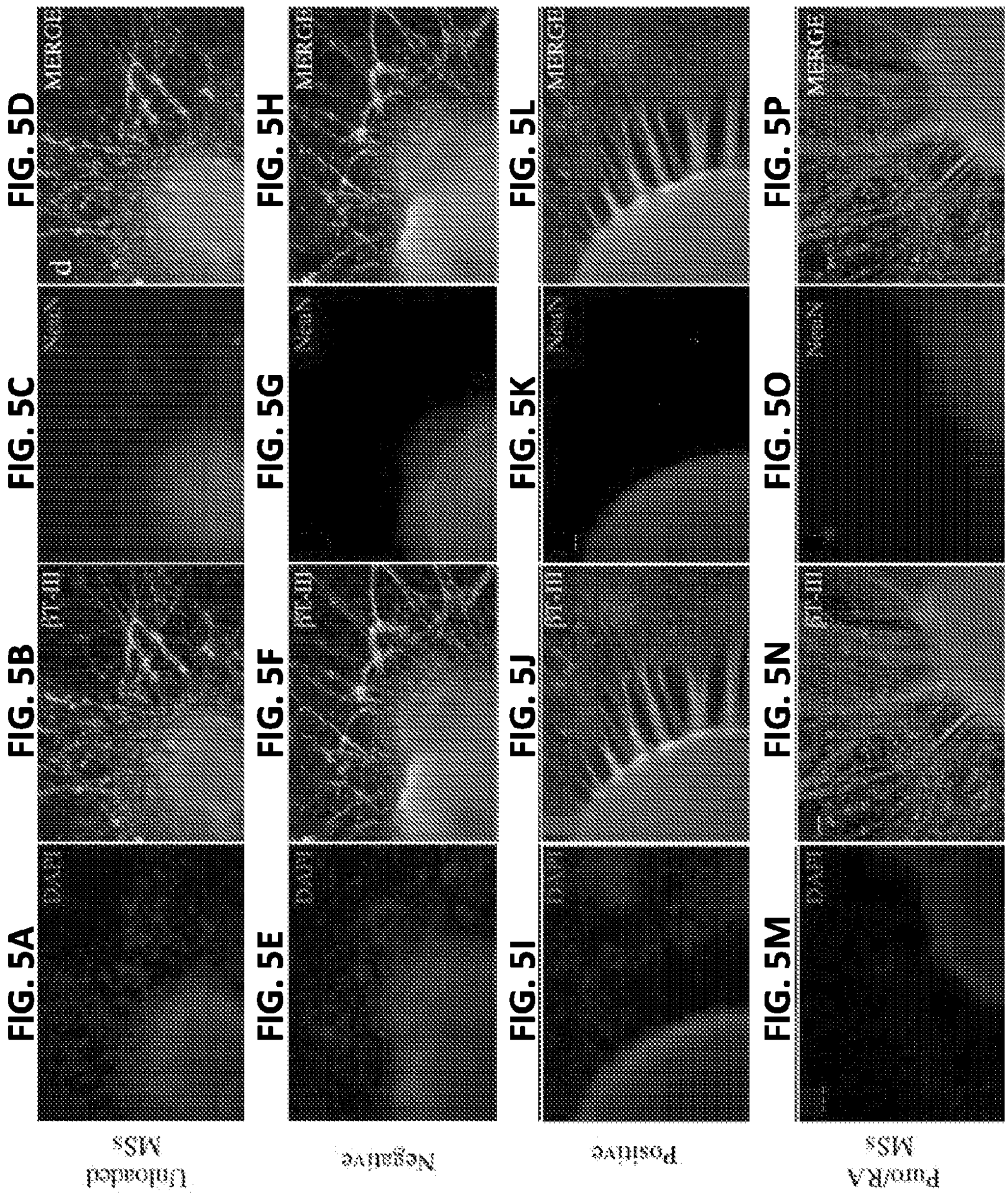


FIG. 5A FIG. 5B FIG. 5C FIG. 5D
FIG. 5E FIG. 5F FIG. 5G FIG. 5H
FIG. 5I FIG. 5J FIG. 5K FIG. 5L
FIG. 5M FIG. 5N FIG. 5O FIG. 5P

FIG. 5Q

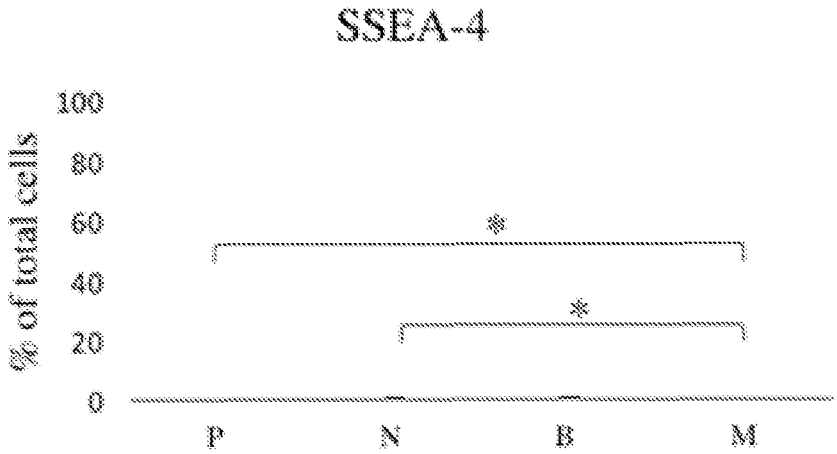


FIG. 5R

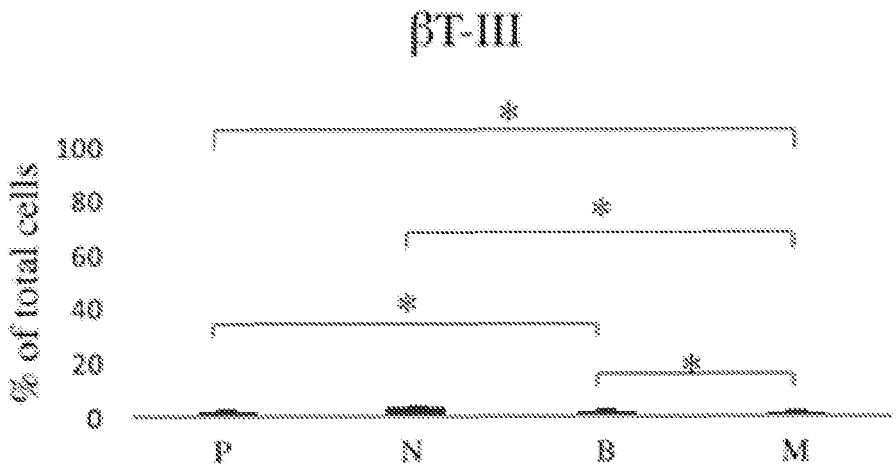


FIG. 5S

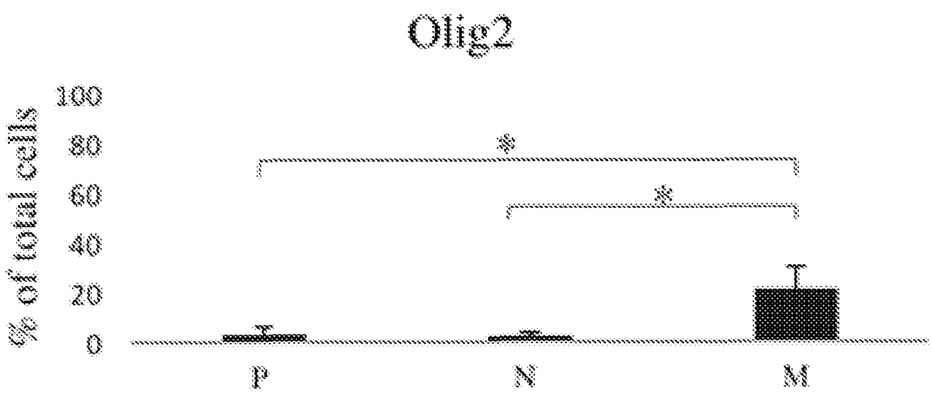

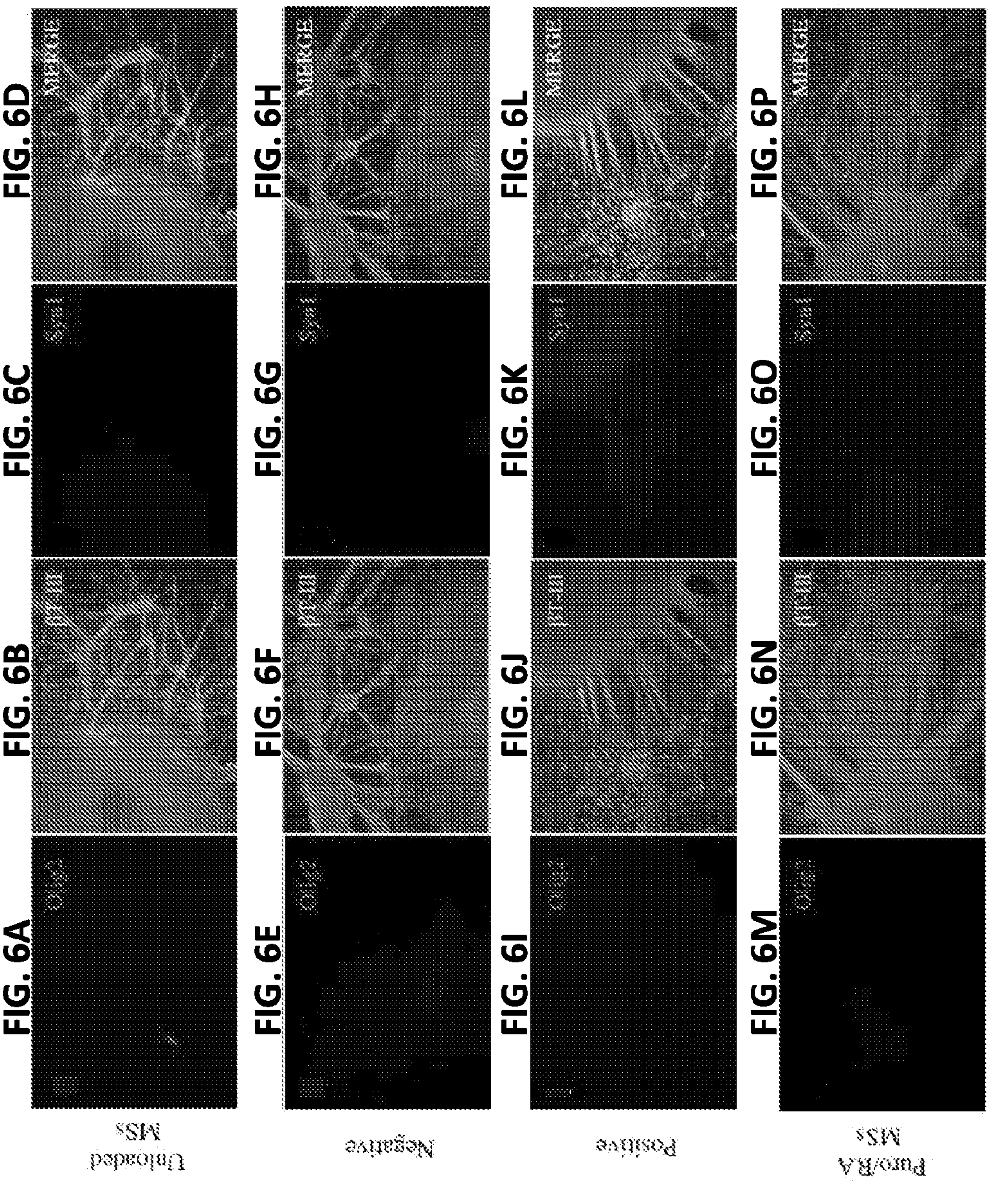


FIG. 6A FIG. 6B FIG. 6C FIG. 6D
FIG. 6E FIG. 6F FIG. 6G FIG. 6H
FIG. 6I FIG. 6J FIG. 6K FIG. 6L
FIG. 6M FIG. 6N FIG. 6O FIG. 6P

FIG. 6Q

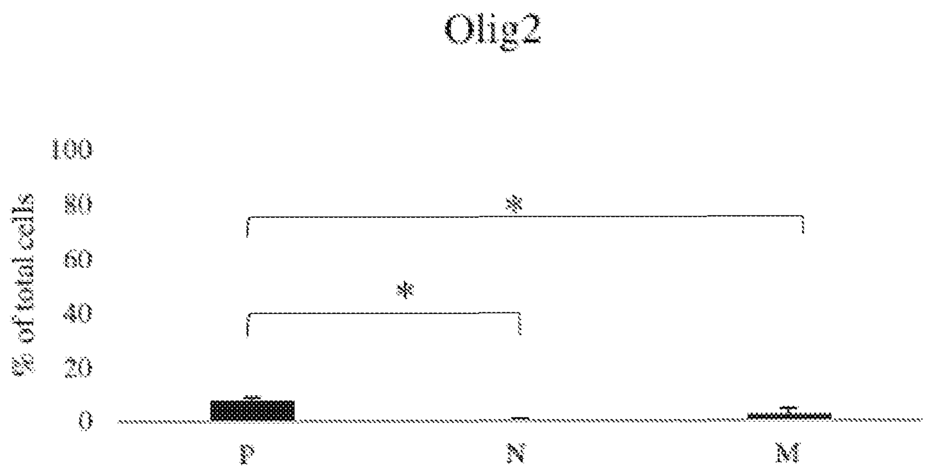


FIG. 6R

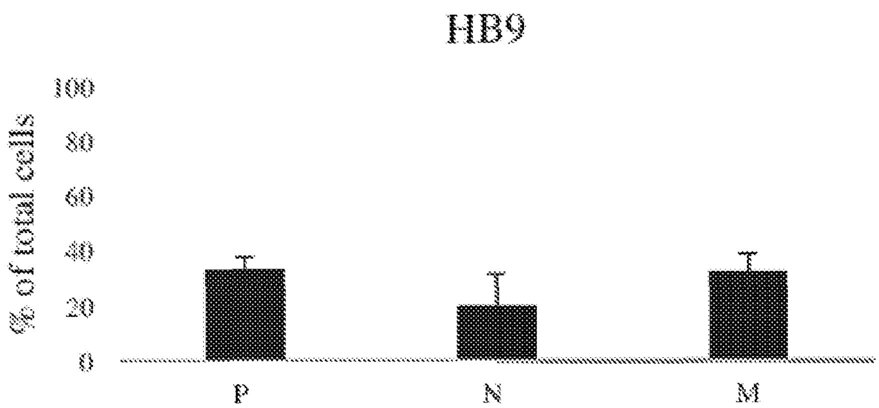


FIG. 6S

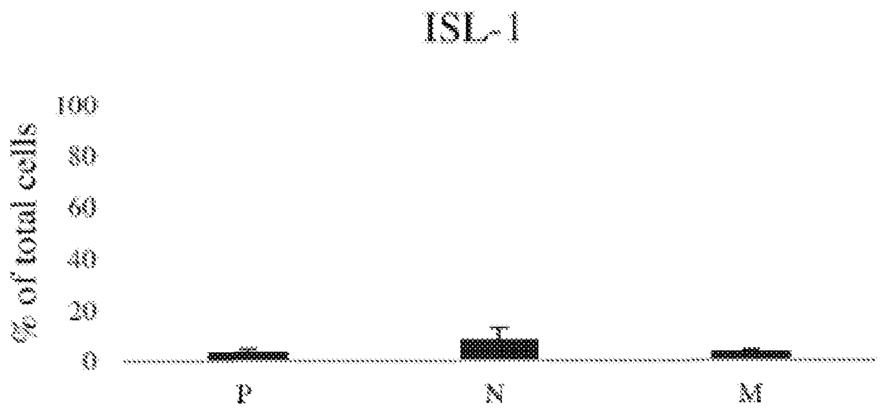

FIG. 7A

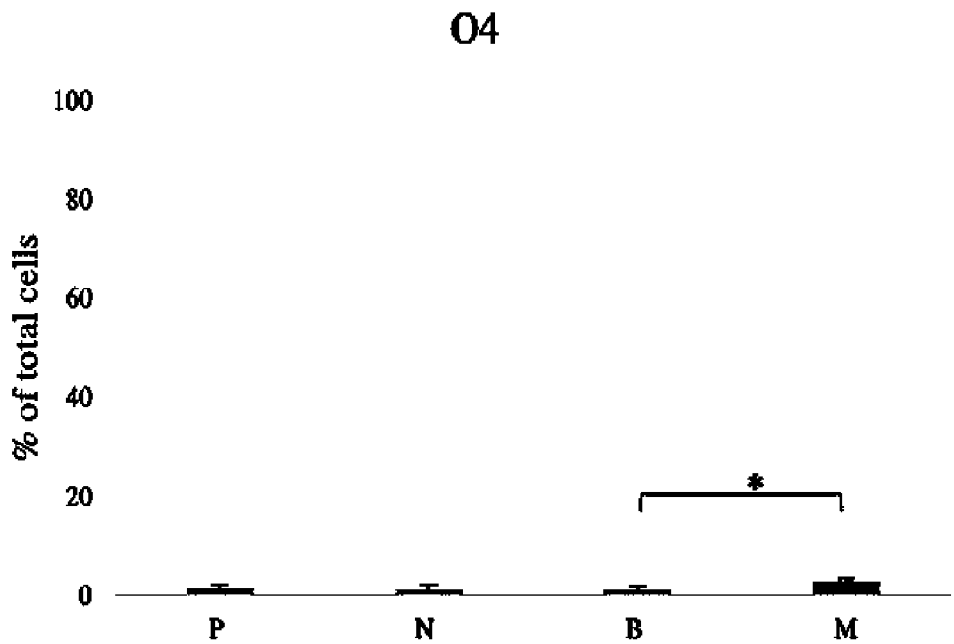
O4
100
80
60
40
20
0
% of total cells
*
P
N
B
M

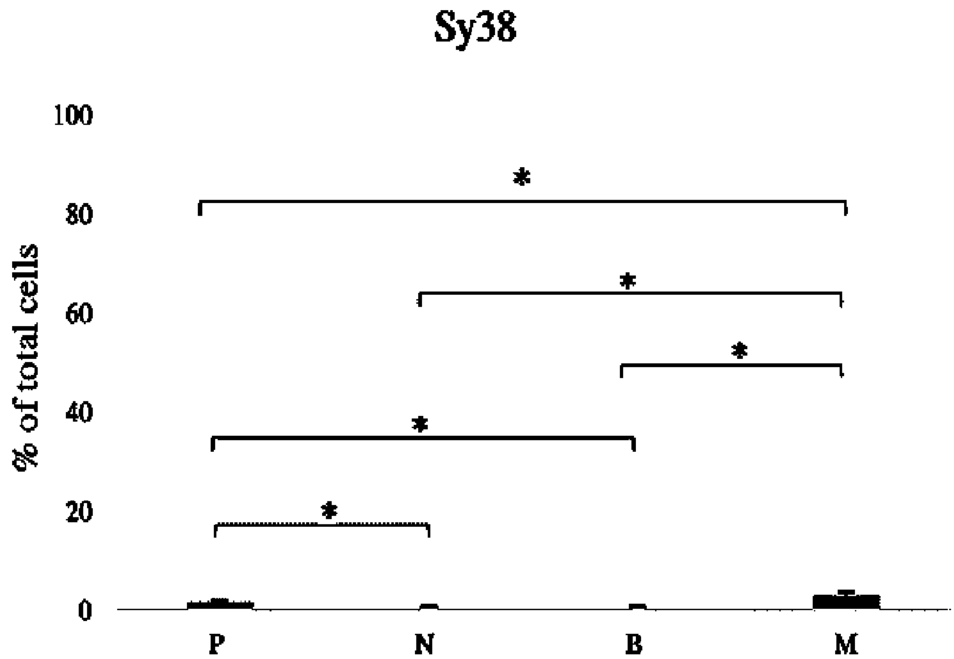
Sy38
100
80
60
40
20
0
% of total cells
*
*
*
*
*
P
N
B
M

FIG. 7B

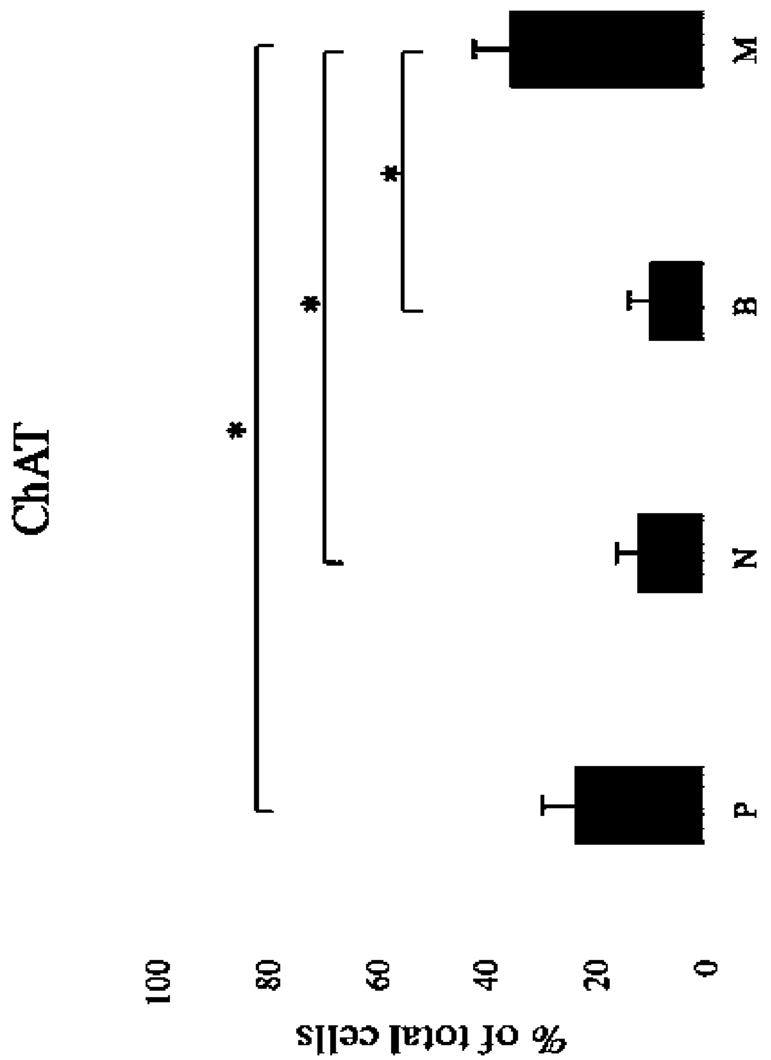


FIG. 7C

FIG. 8A

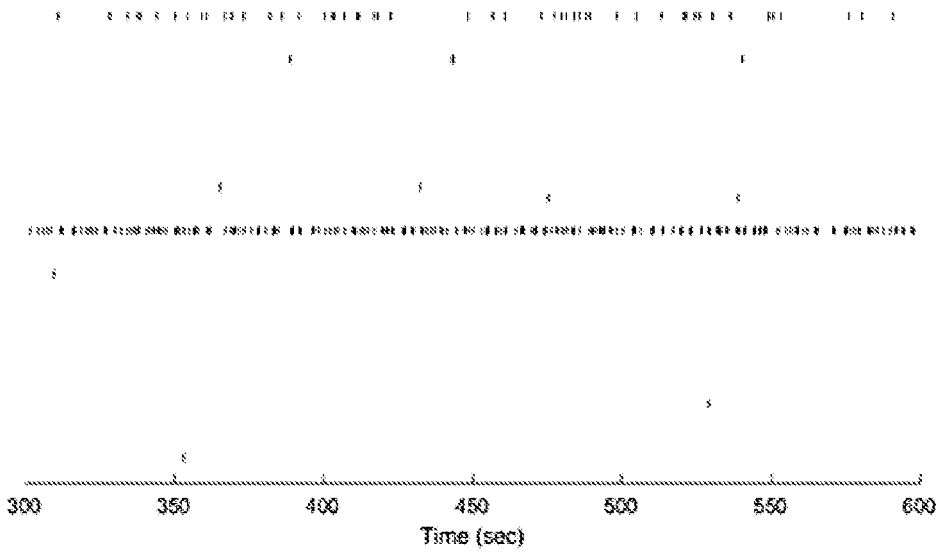


FIG. 8B

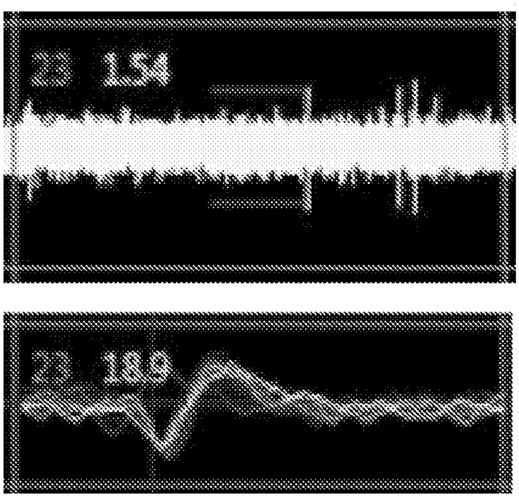

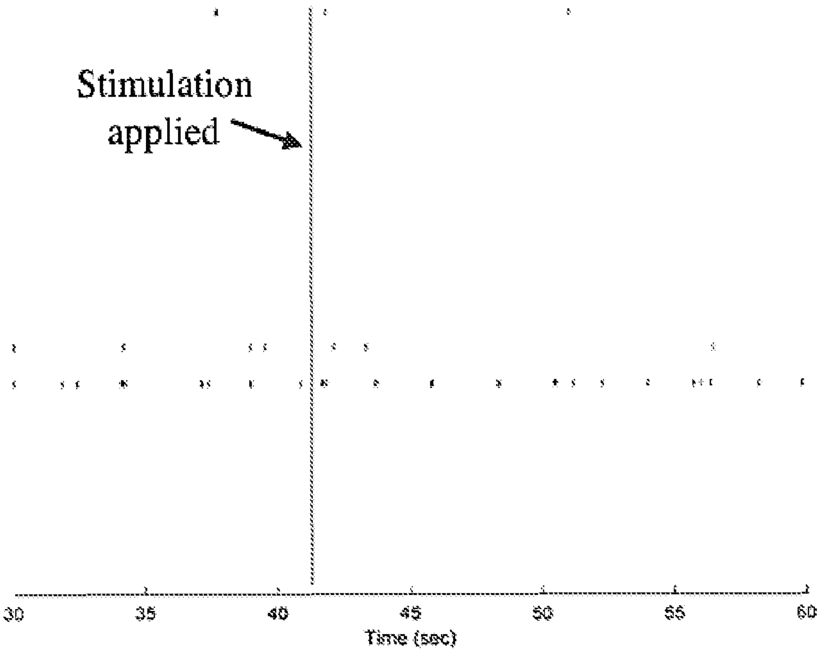
Stimulation
applied
30
35
40
45
50
55
60
Time (sec)

FIG. 8C

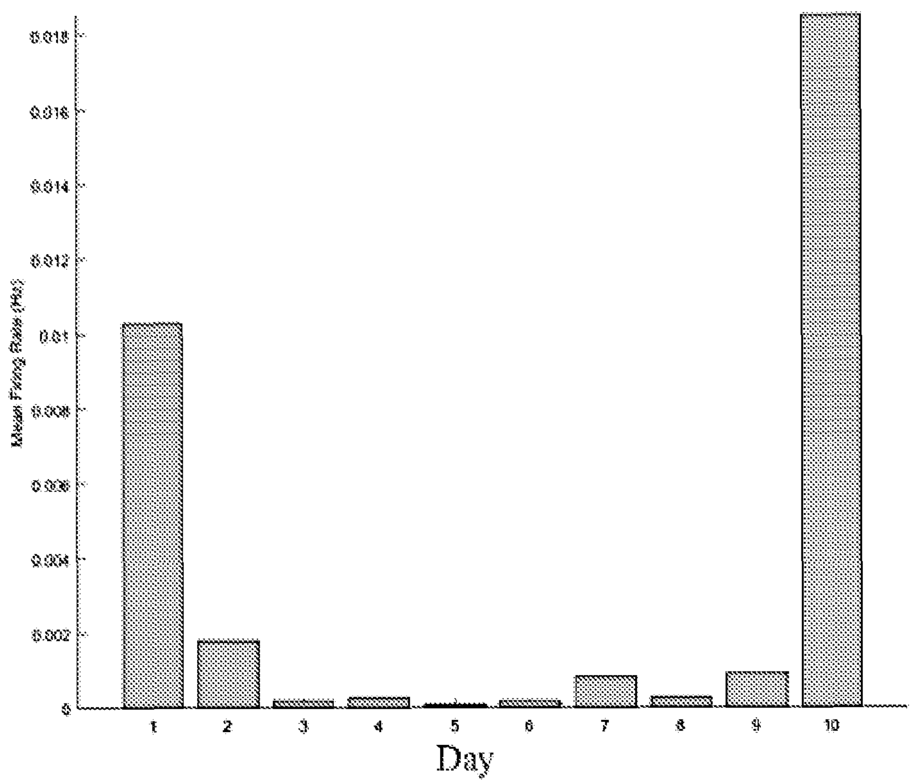
0.018
0.016
0.014
0.012
0.01
0.008
0.006
0.004
0.002
0
1
2
3
4
5
6
7
8
9
10
Day

FIG. 8D

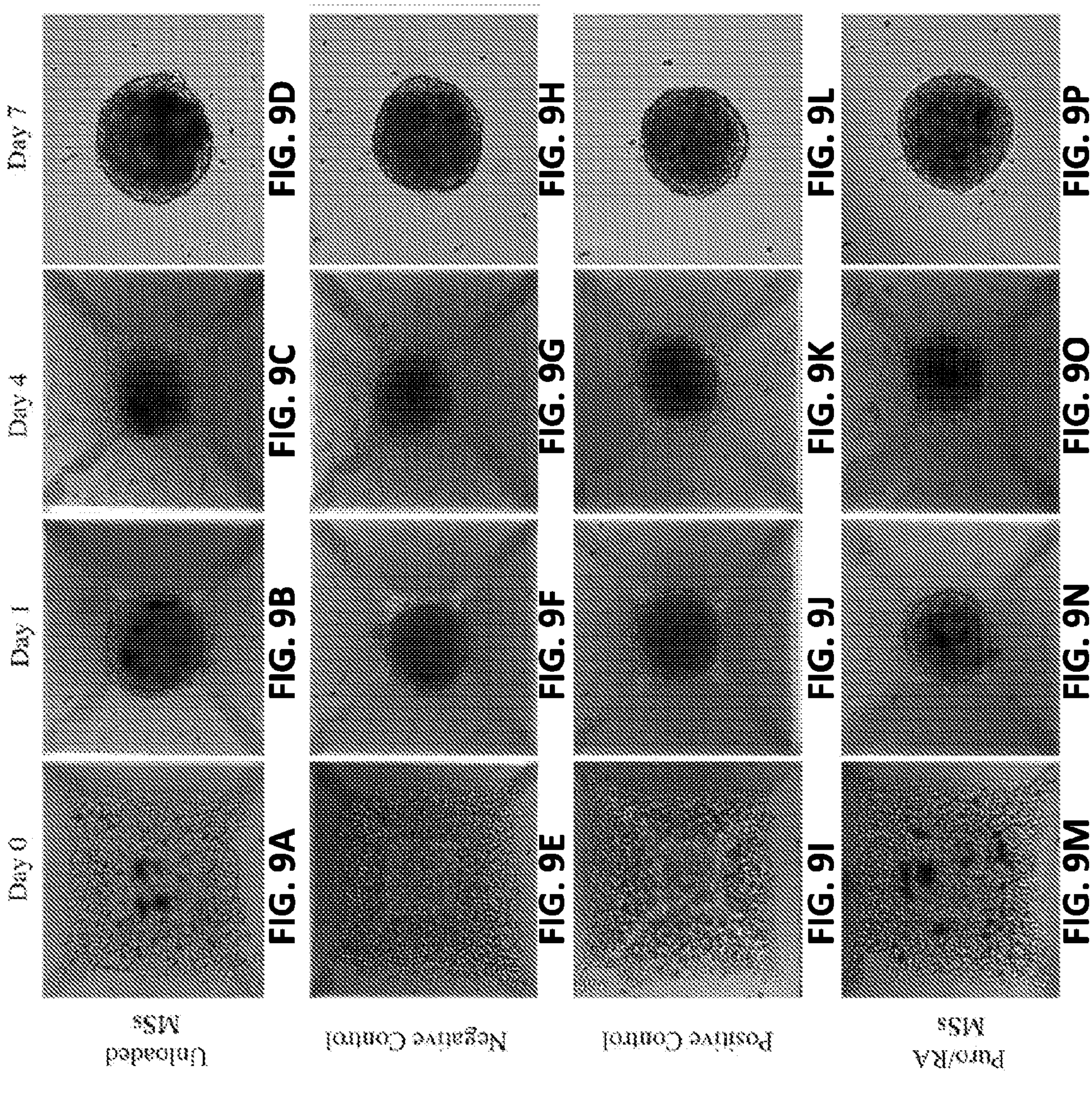


FIG. 9A FIG. 9B FIG. 9C FIG. 9D
FIG. 9E FIG. 9F FIG. 9G FIG. 9H
FIG. 9I FIG. 9J FIG. 9K FIG. 9L
FIG. 9M FIG. 9N FIG. 9O FIG. 9P

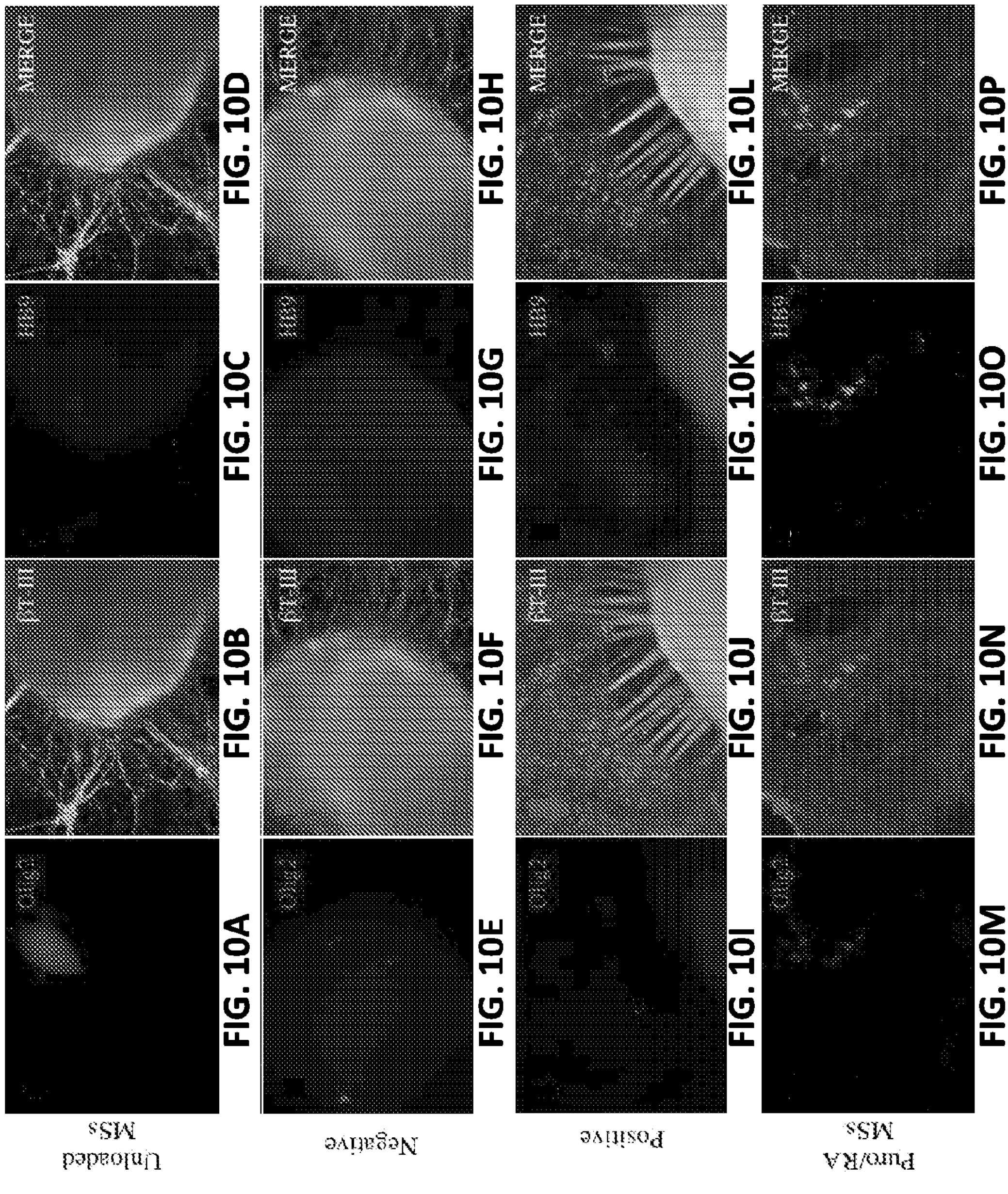


FIG. 10A FIG. 10B FIG. 10C FIG. 10D

FIG. 10E FIG. 10F FIG. 10G FIG. 10H

FIG. 10I FIG. 10J FIG. 10K FIG. 10L

FIG. 10M FIG. 10N FIG. 10O FIG. 10P

FIG. 11A

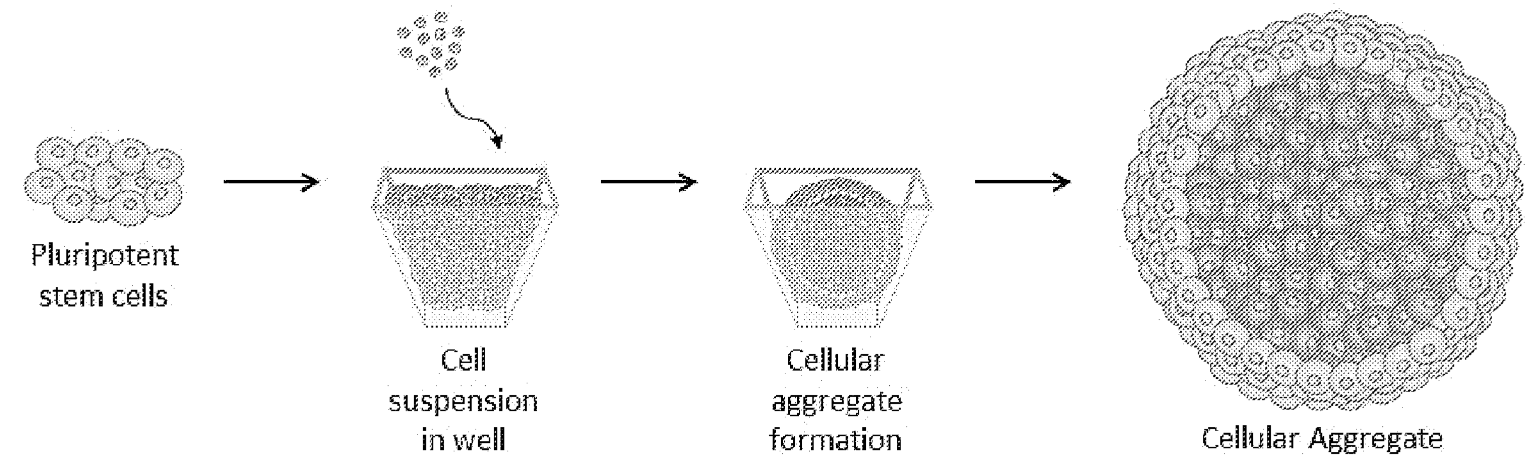


FIG. 11B

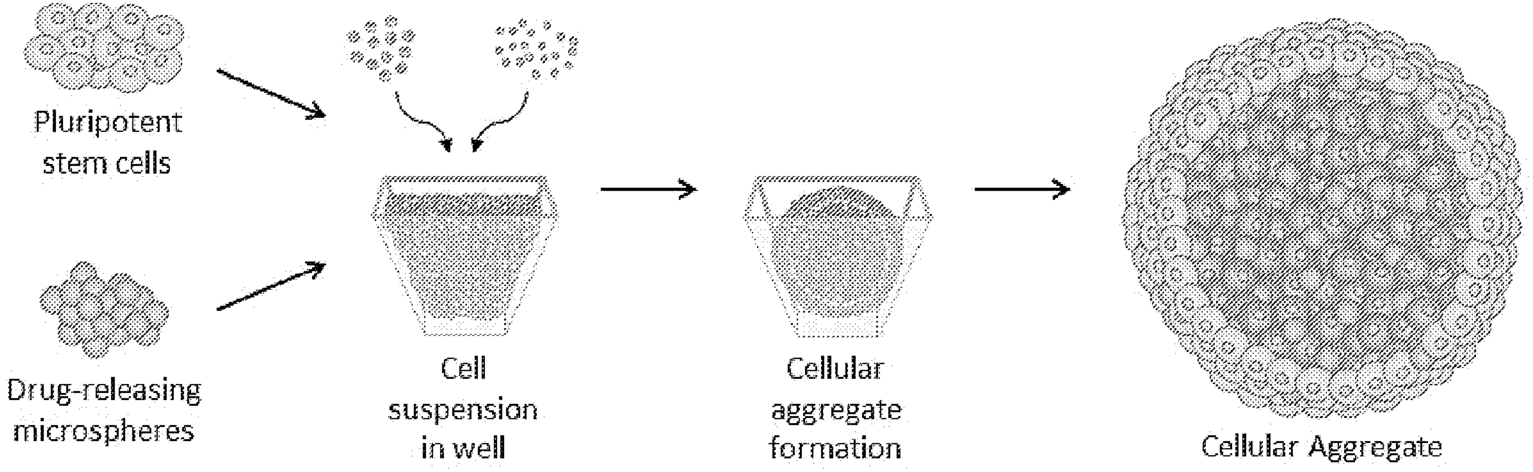

FIG. 12A

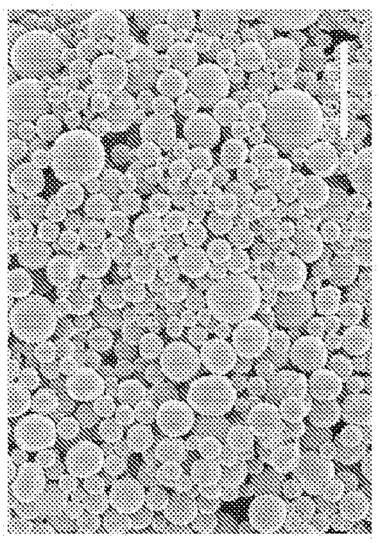

FIG. 12B

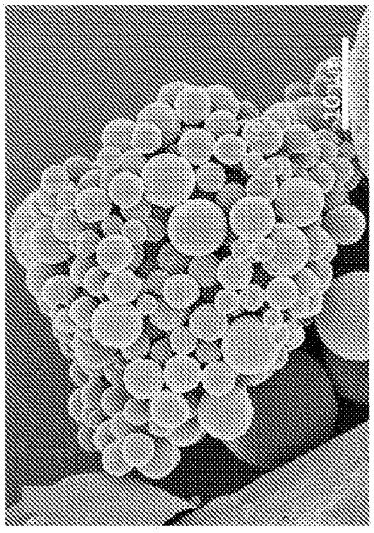

FIG. 12C

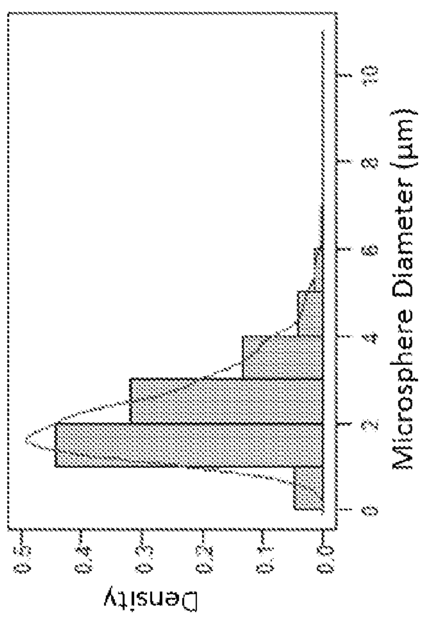


FIG. 12D

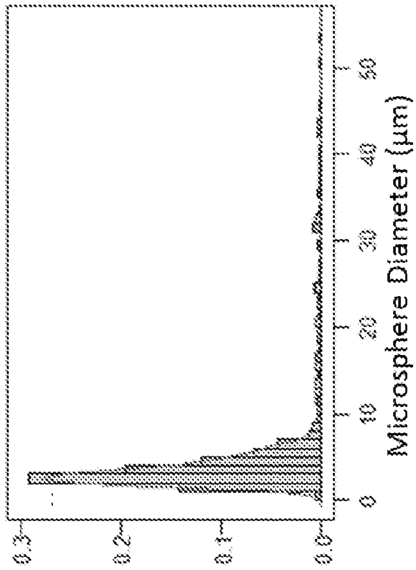

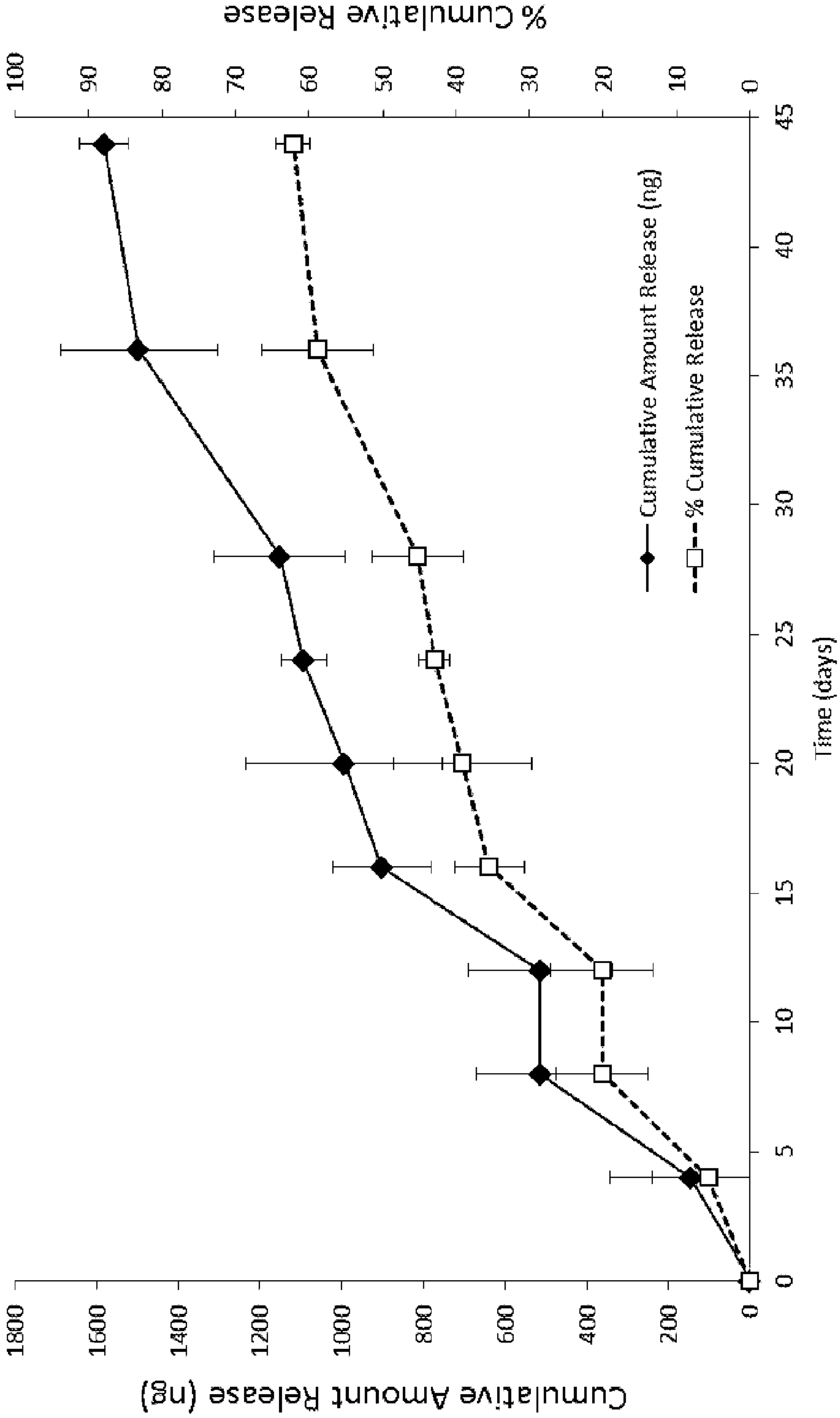
% Cumulative Release
Cumulative Amount Release (ng)
Time (days)
Cumulative Amount Release (ng)
% Cumulative Release
0
5
10
15
20
25
30
35
40
45
0
200
400
600
800
1000
1200
1400
1600
1800
0
10
20
30
40
50
60
70
80
90
100

FIG. 12E

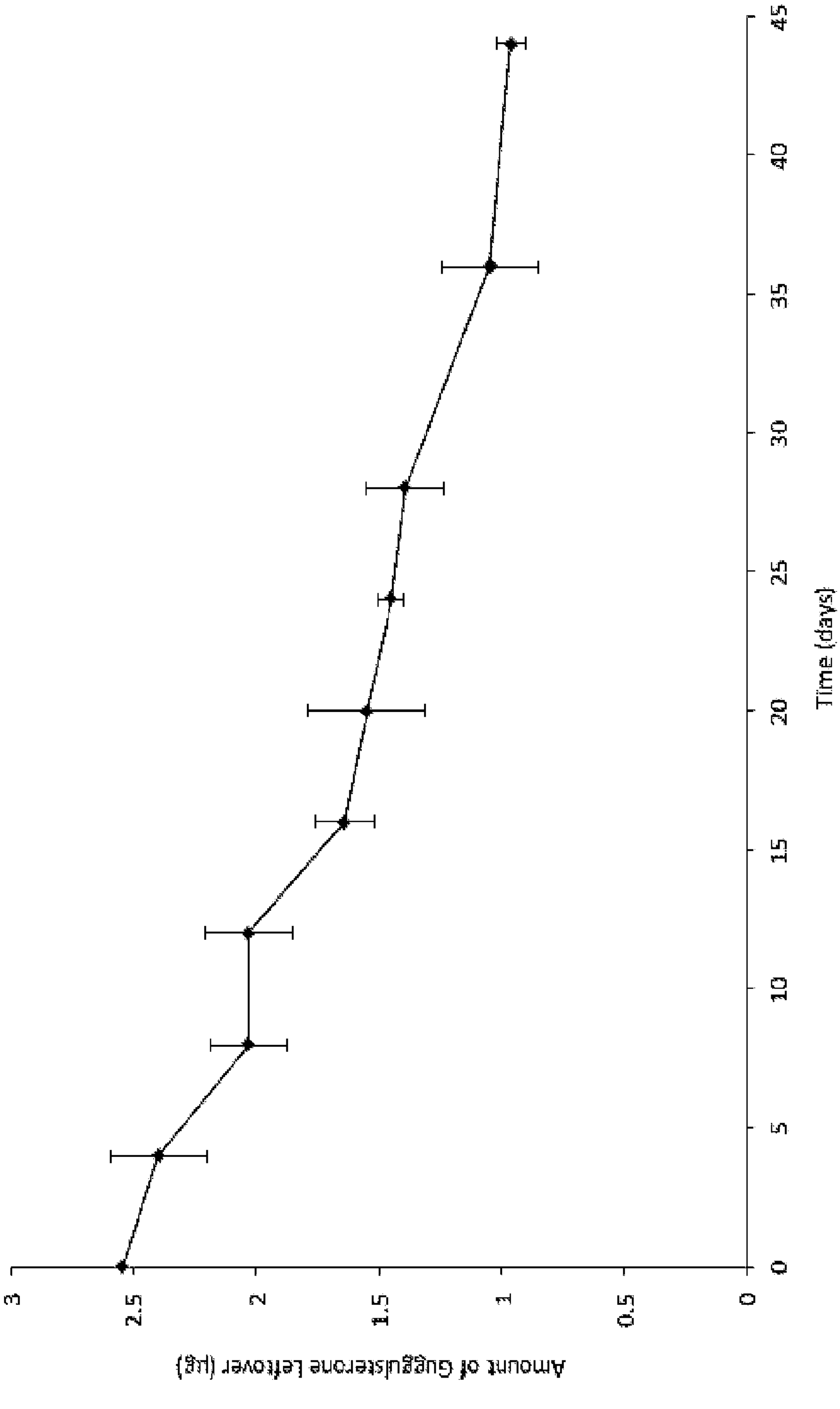
Amount of Guggulsterone leftover (µg)
Time (days)
0
0.5
1
1.5
2
2.5
3
0
5
10
15
20
25
30
35
40
45

FIG. 13

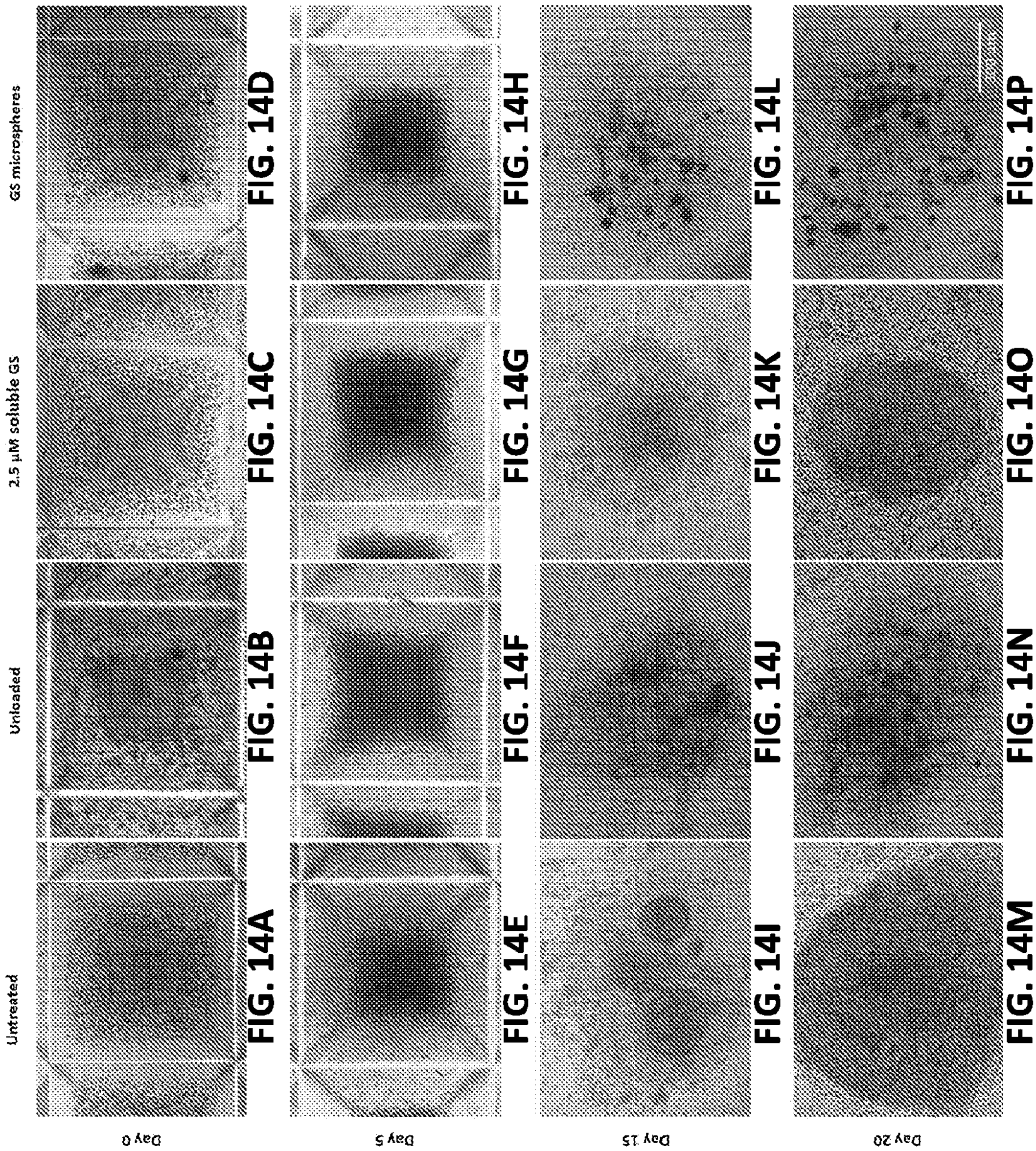


FIG. 14A FIG. 14B FIG. 14C FIG. 14D

FIG. 14E FIG. 14F FIG. 14G FIG. 14H

FIG. 14I FIG. 14J FIG. 14K FIG. 14L

FIG. 14M FIG. 14N FIG. 14O FIG. 14P

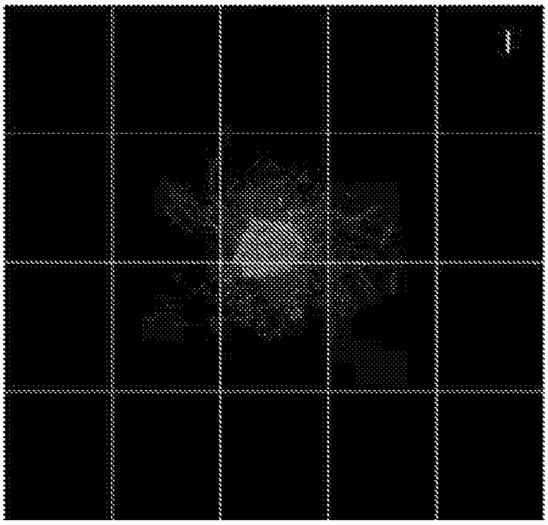

FIG. 15A

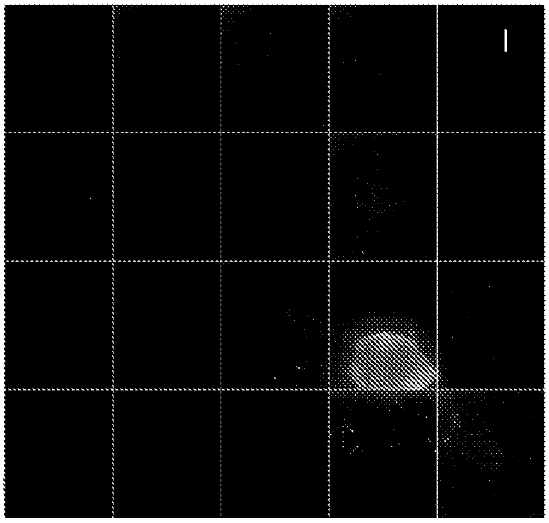

FIG. 15B

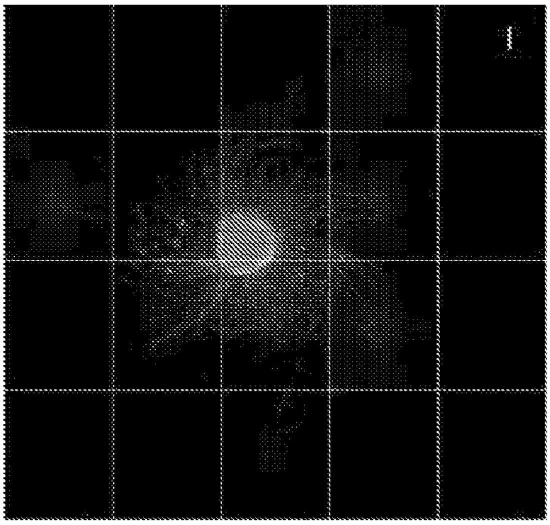

FIG. 15C

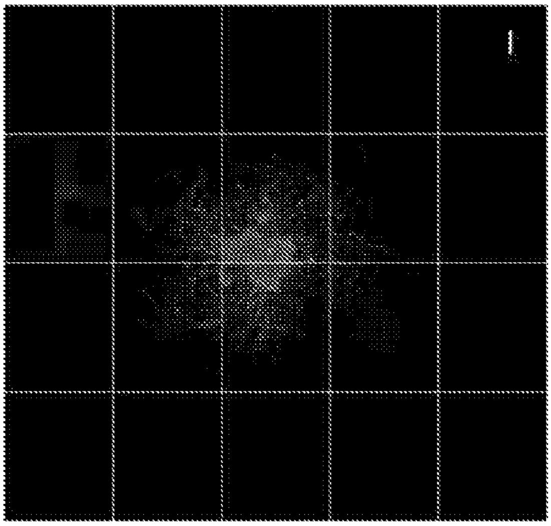

FIG. 15D

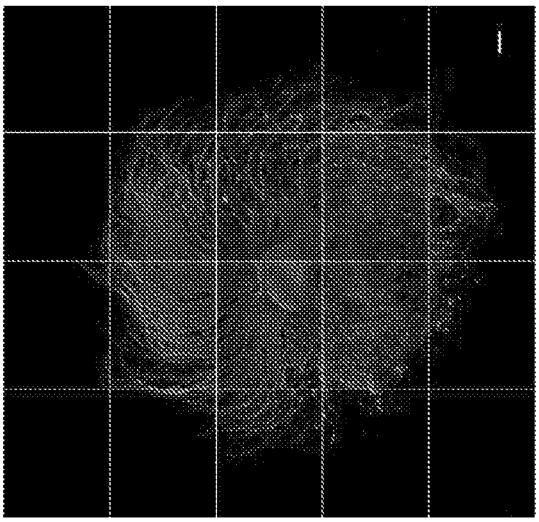

FIG. 16A

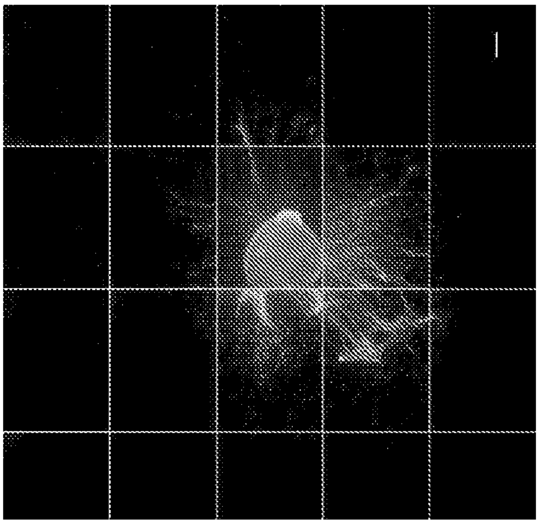

FIG. 16B

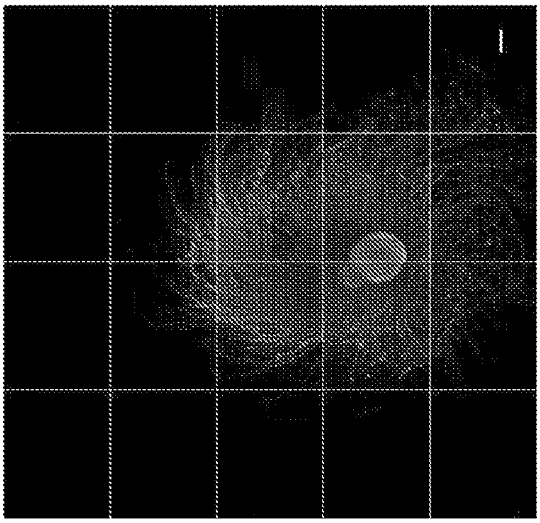

FIG. 16C

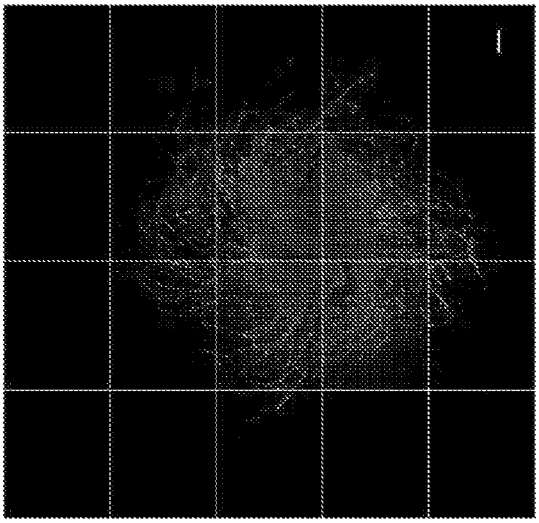

FIG. 16D

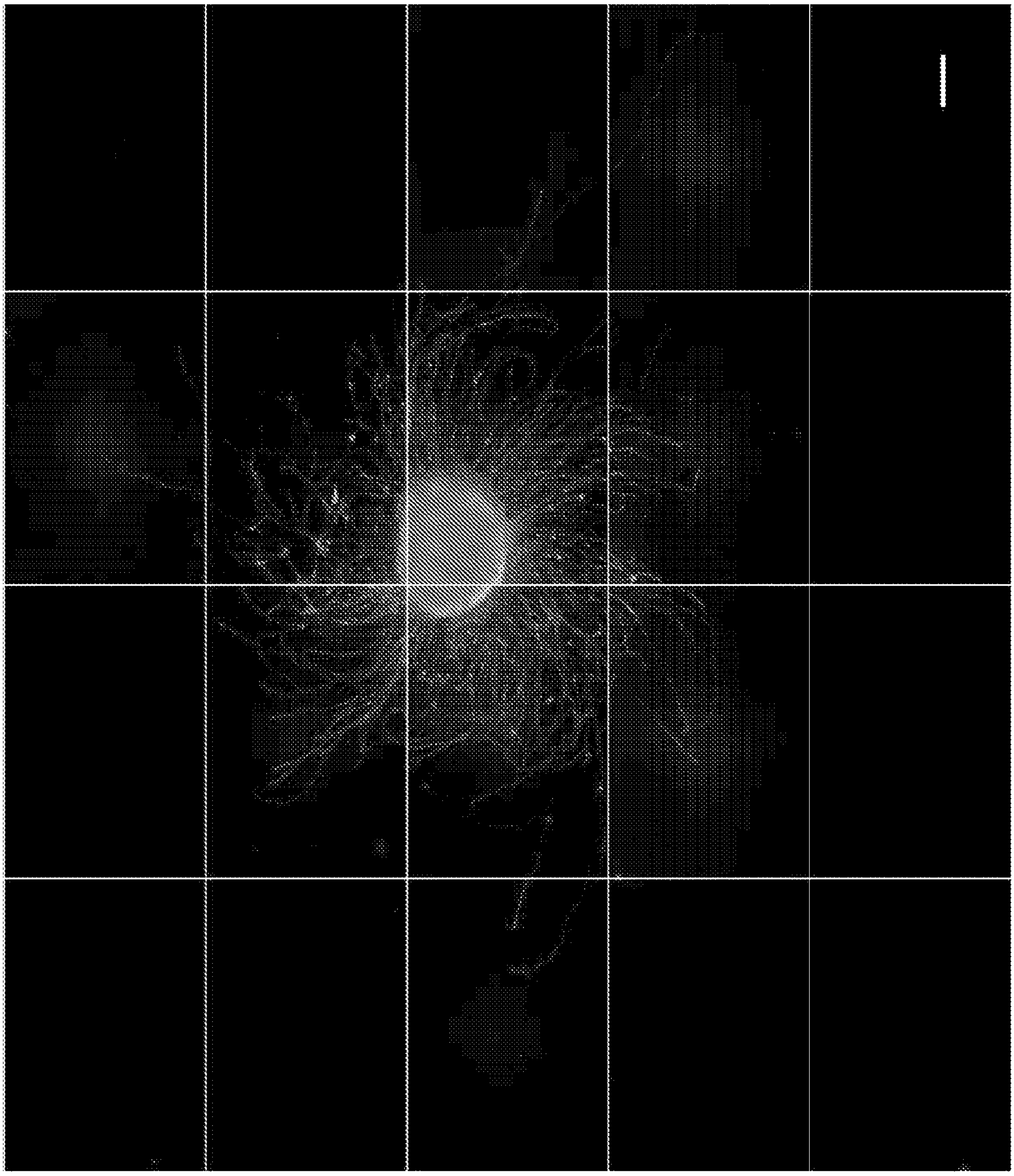

FIG. 17

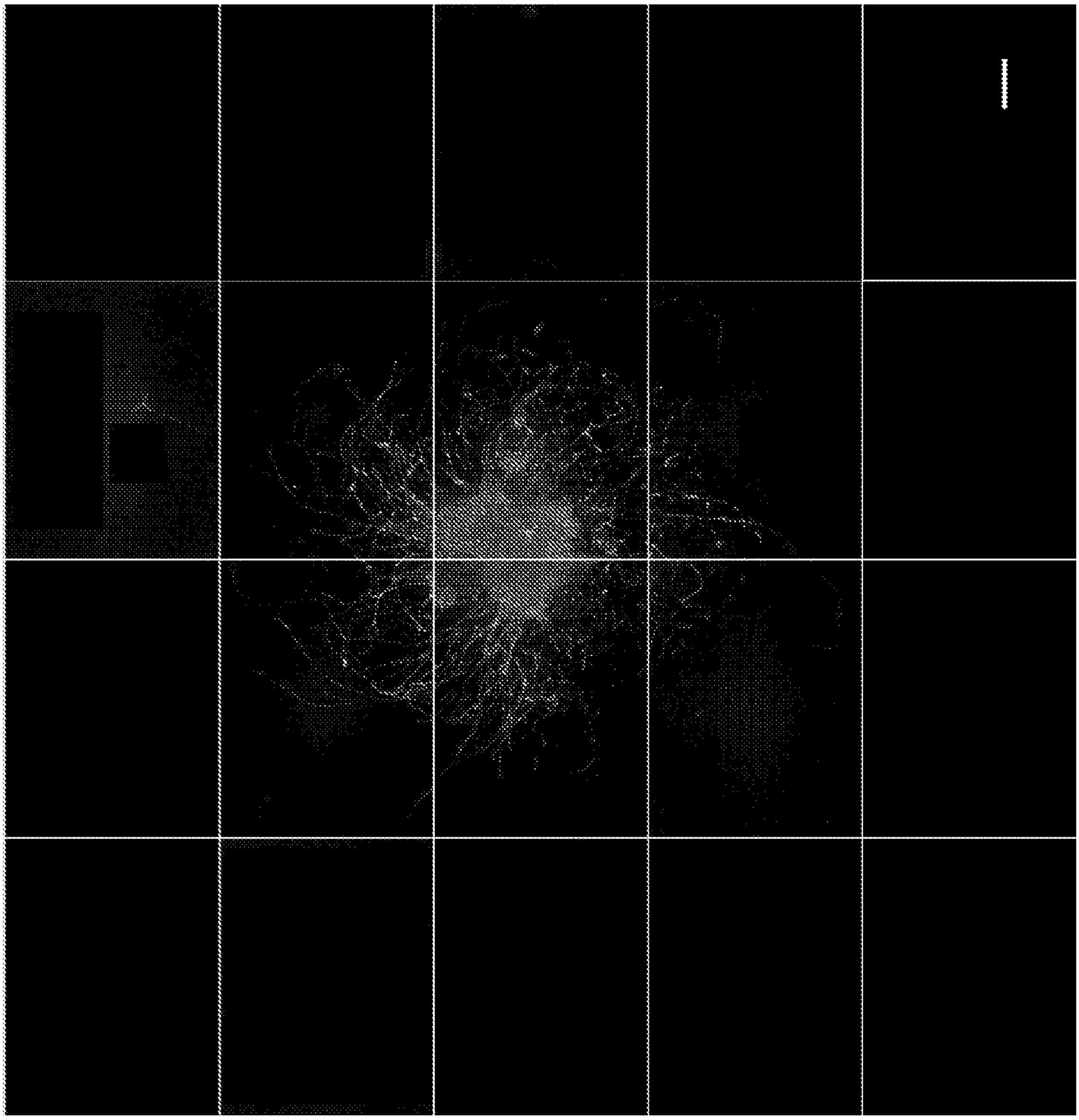

FIG. 18

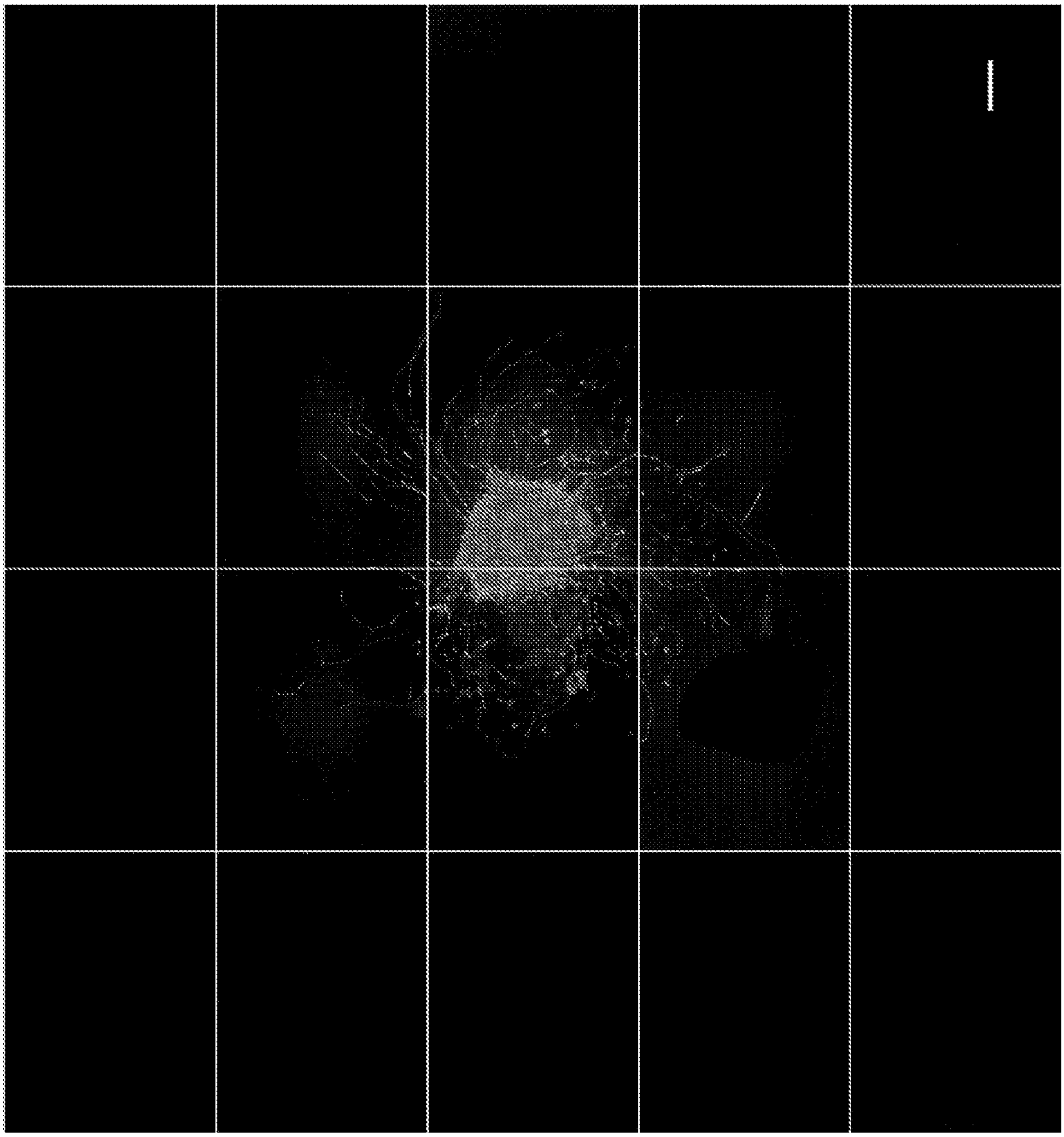

FIG. 19

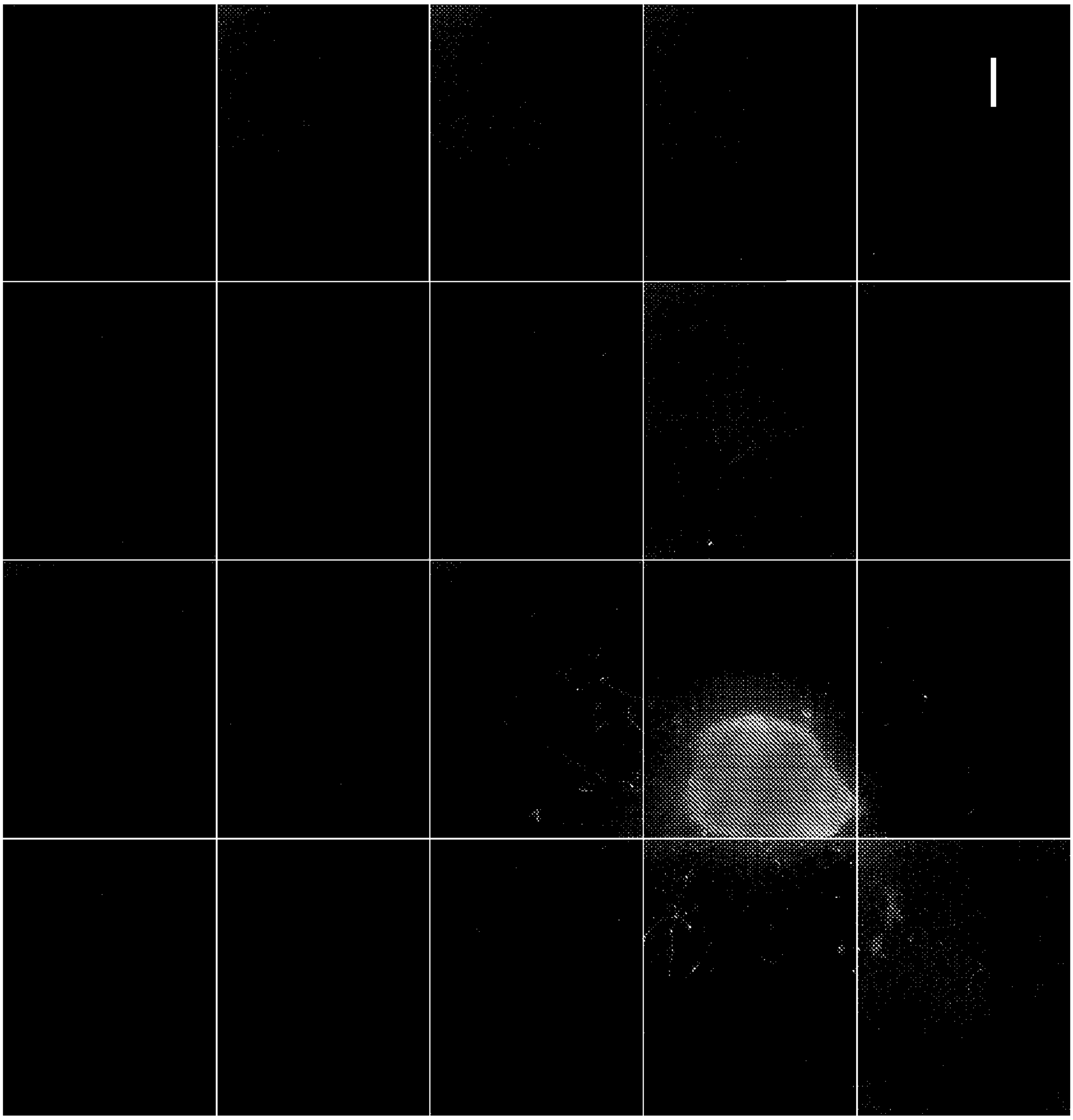

FIG. 20

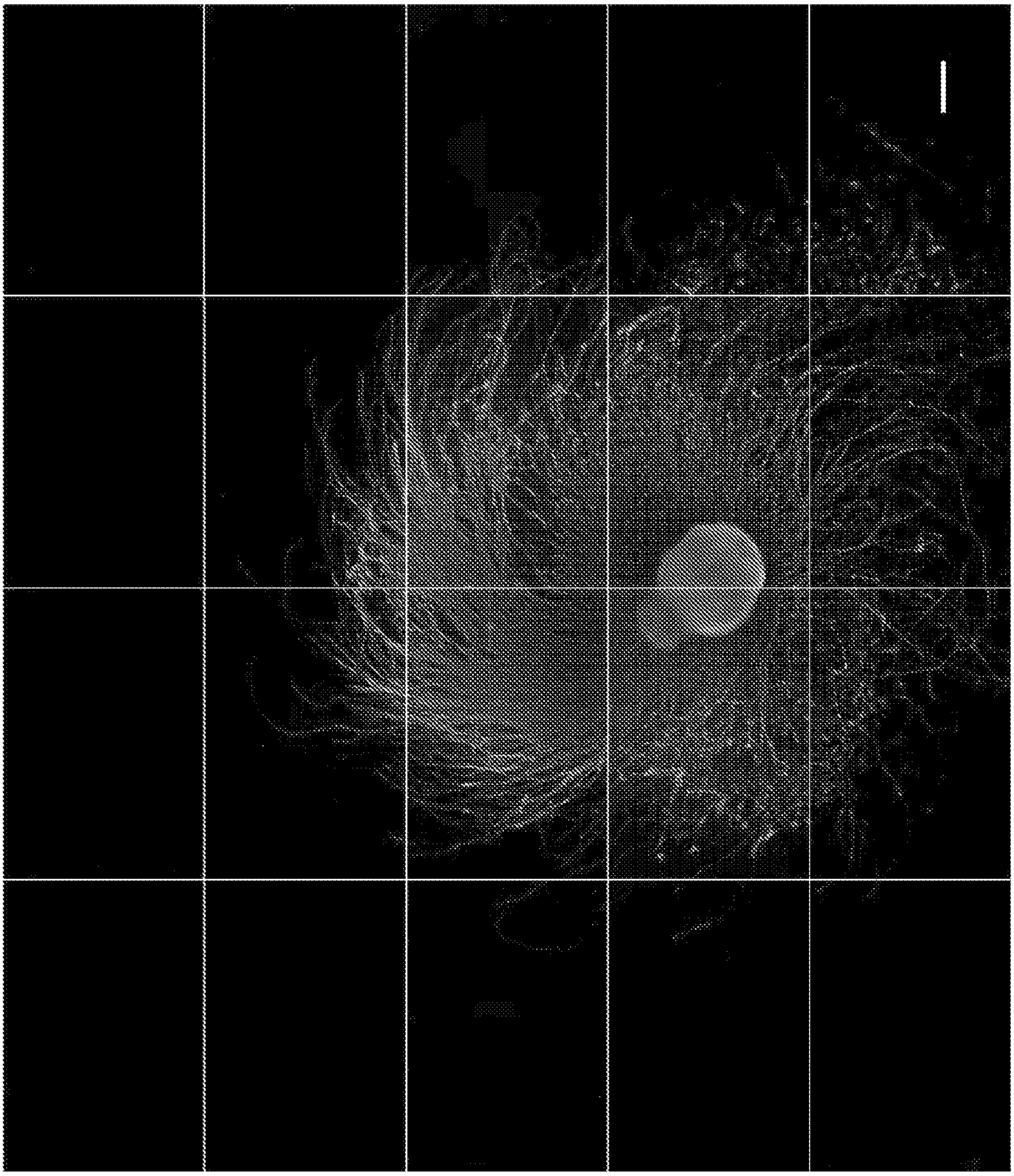

FIG. 21

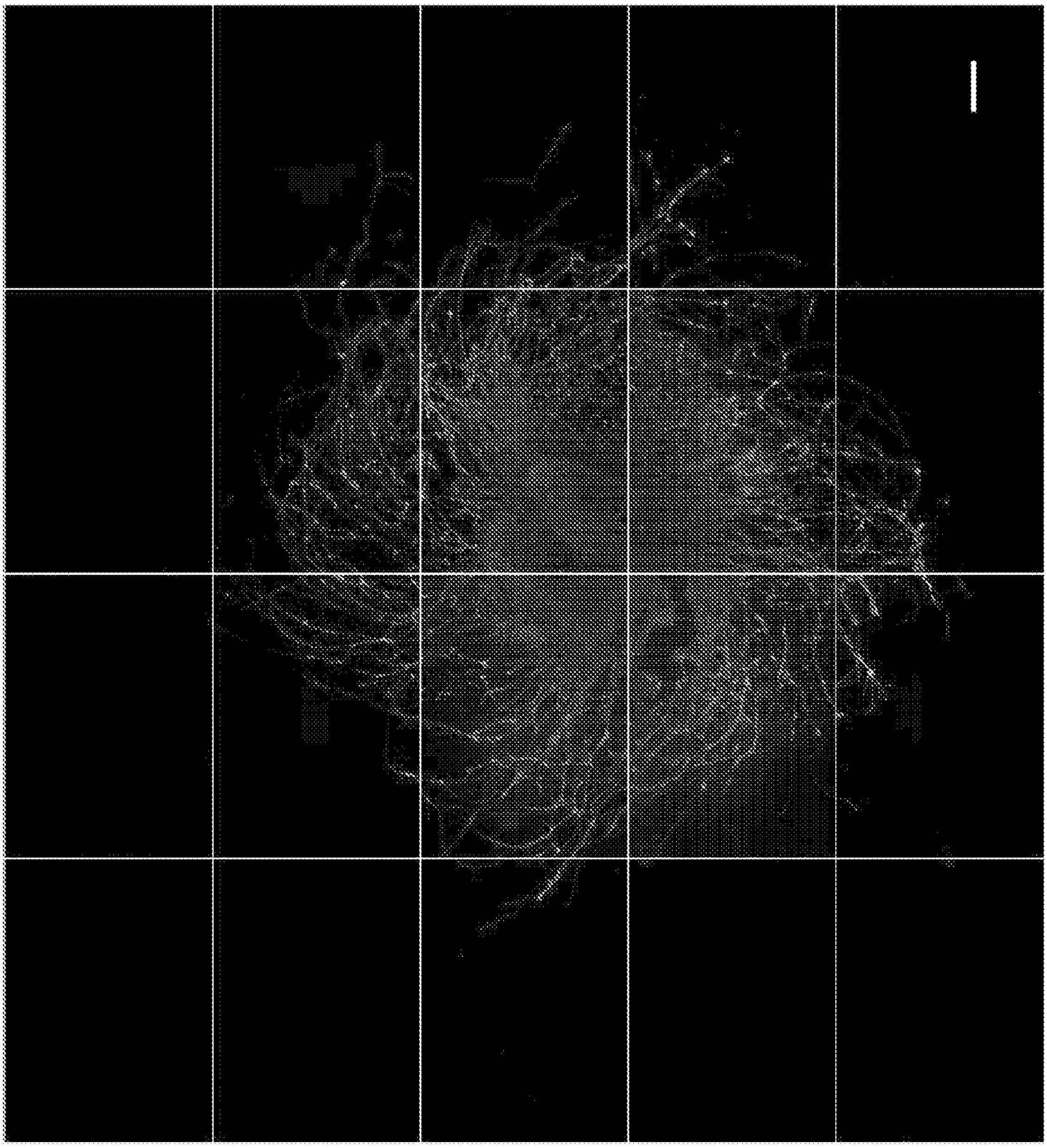

FIG. 22

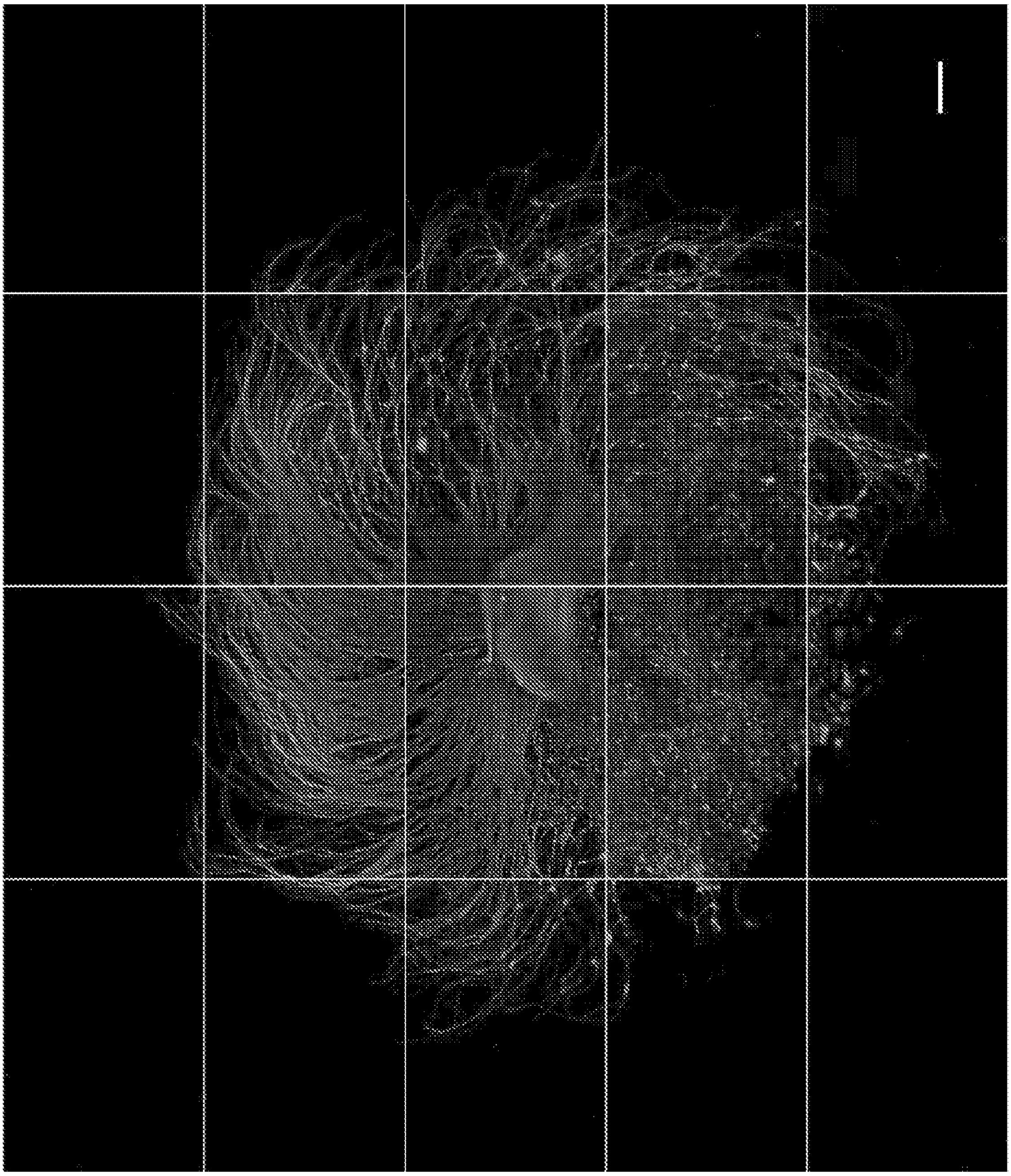

FIG. 23

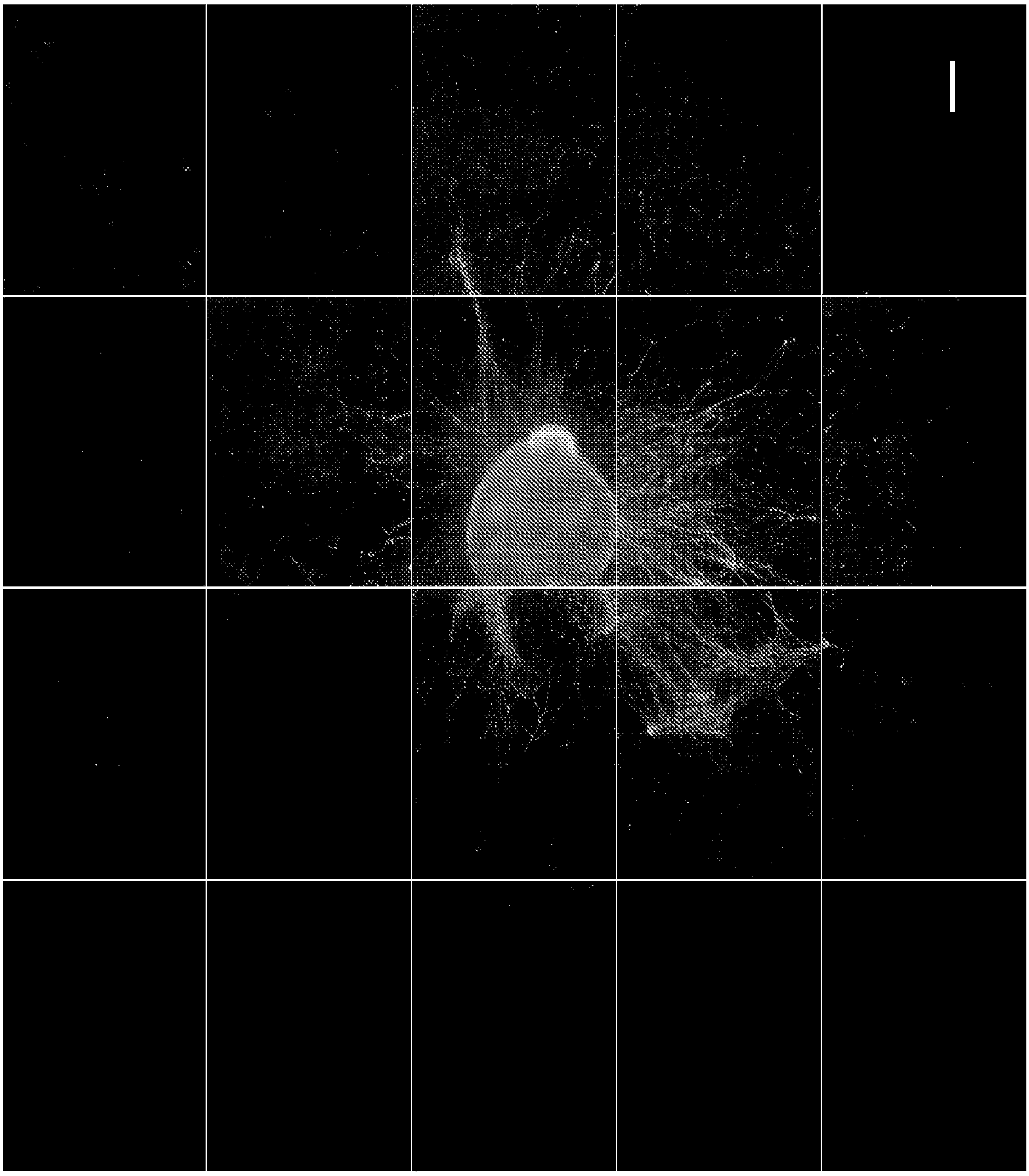

FIG. 24

FIG. 25A

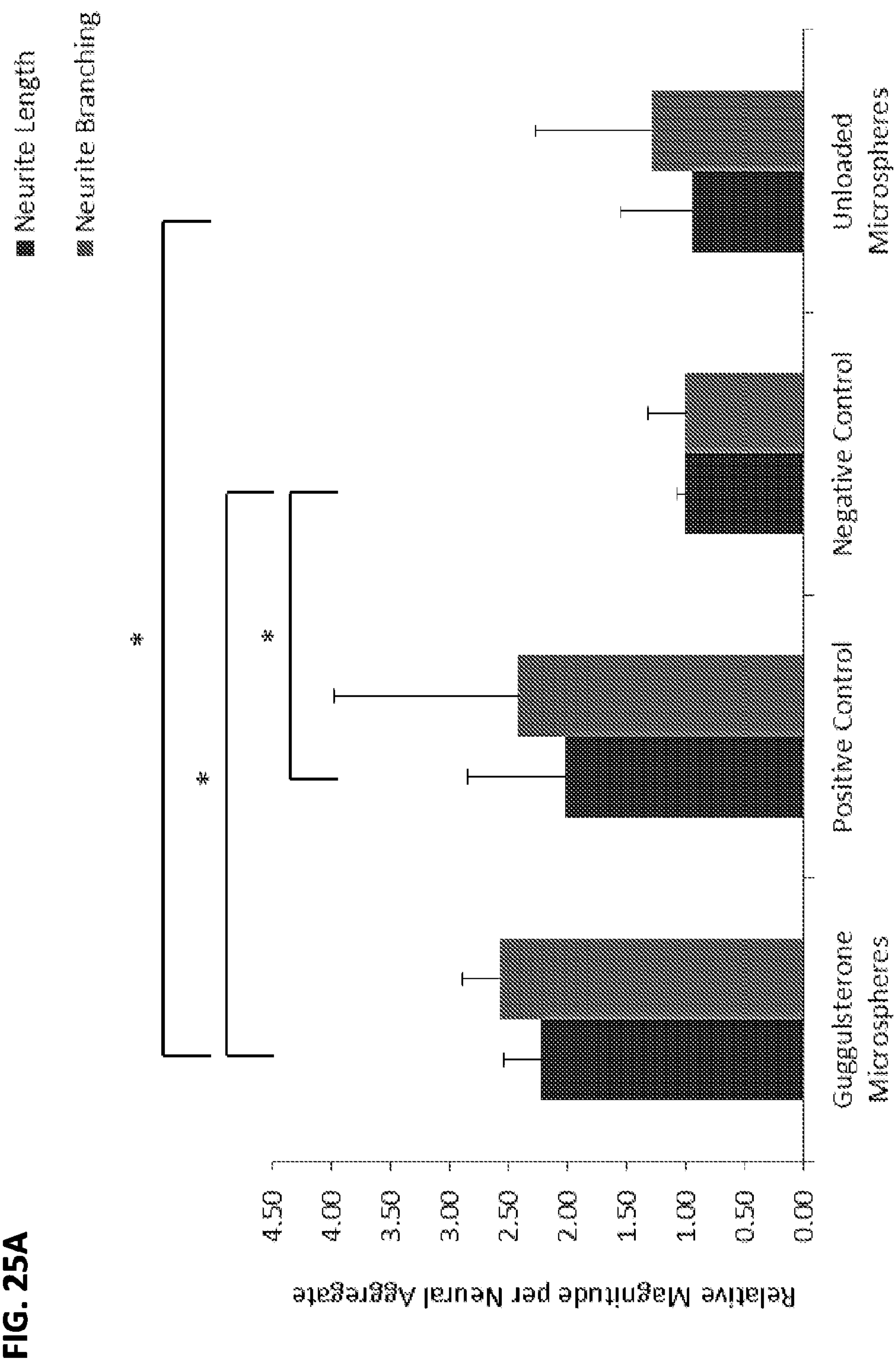
Neurite Length
Neurite Branching
Relative Magnitude per Neural Aggregate
4.50
4.00
3.50
3.00
2.50
2.00
1.50
1.00
0.50
0.00
*
*
*
Guggulsterone Microspheres
Positive Control
Negative Control
Unloaded Microspheres

FIG. 25B

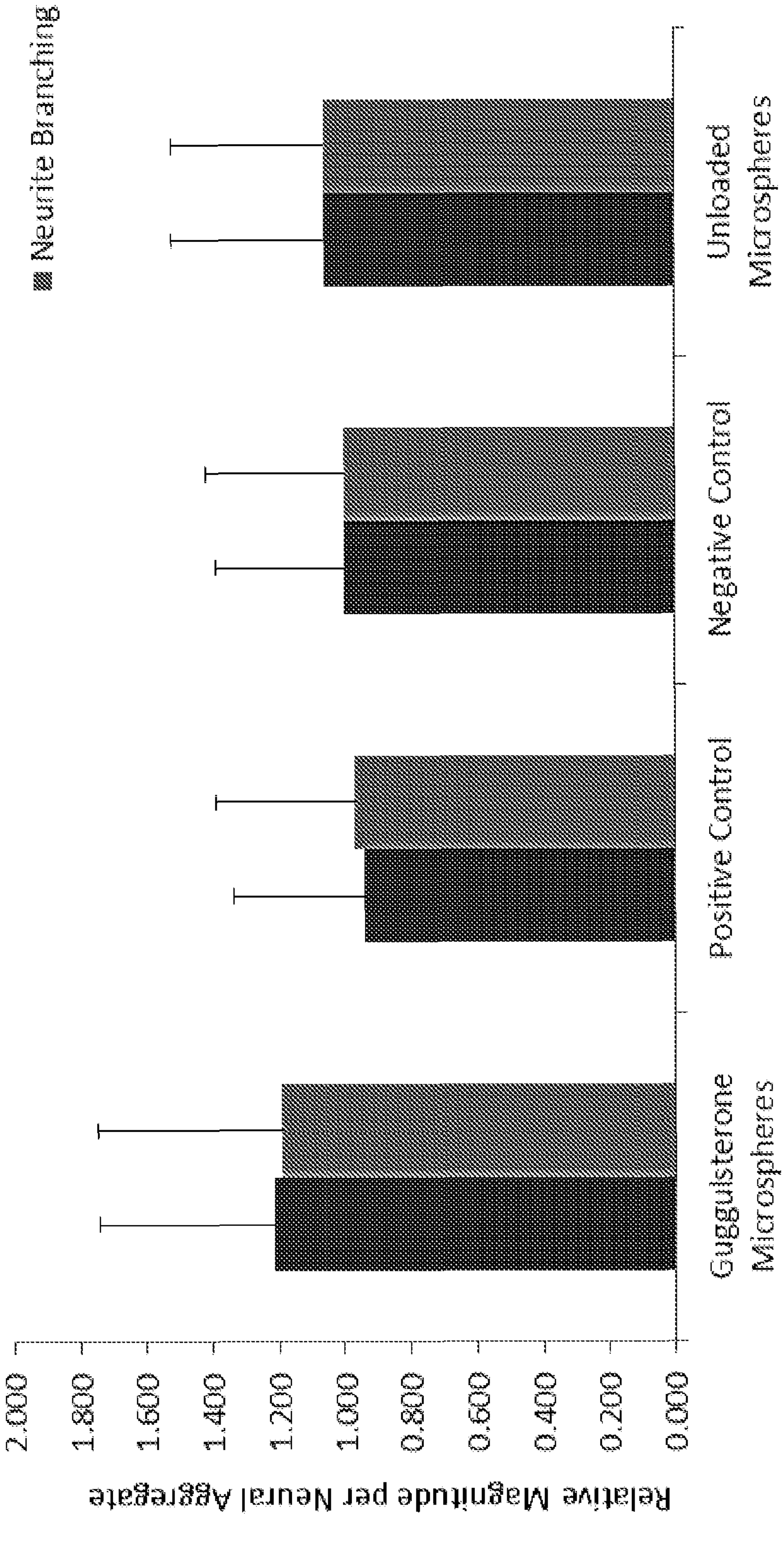
Relative Magnitude per Neural Aggregate
2.000
1.800
1.600
1.400
1.200
1.000
0.800
0.600
0.400
0.200
0.000
Guggulsterone Microspheres
Positive Control
Negative Control
Unloaded Microspheres
Neurite Length
Neurite Branching

FIG. 26A

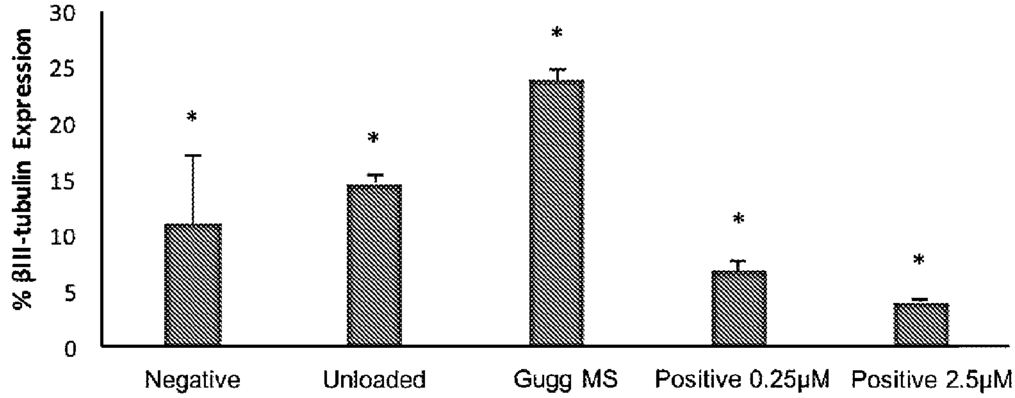


FIG. 26B

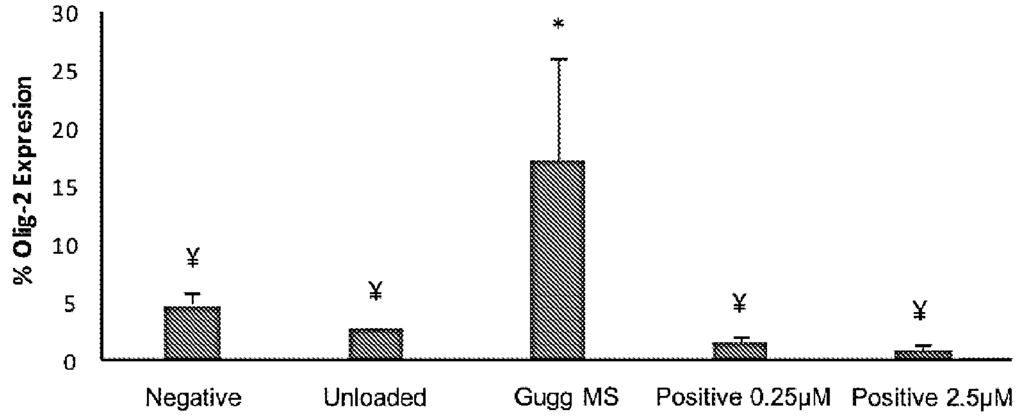


FIG. 26C

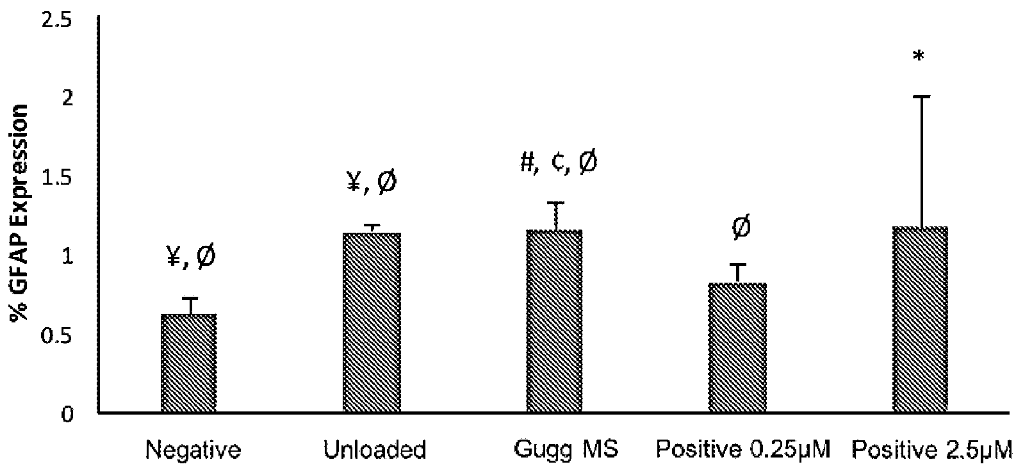

FIG. 27B

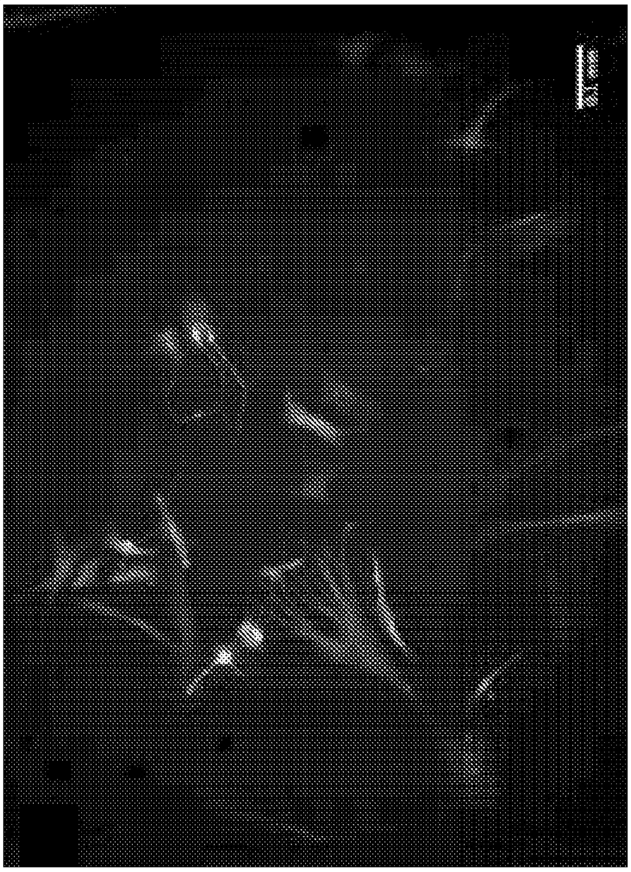

FIG. 27A

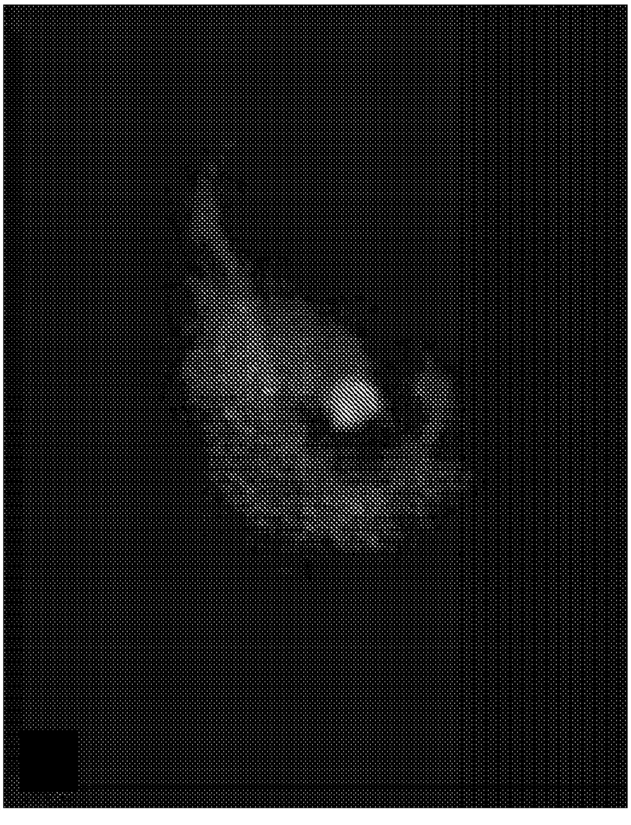

FIG. 28A

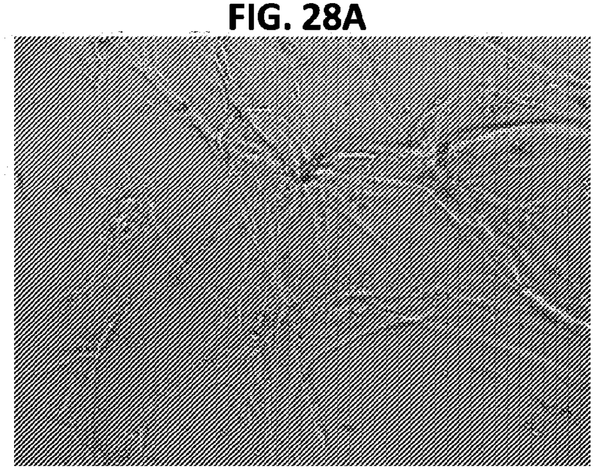

FIG. 28B

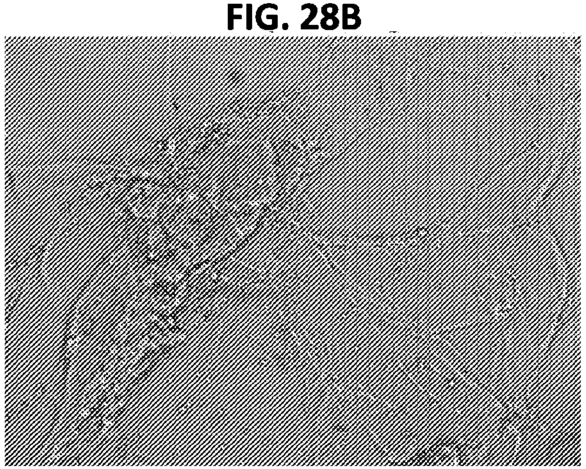

FIG. 28C

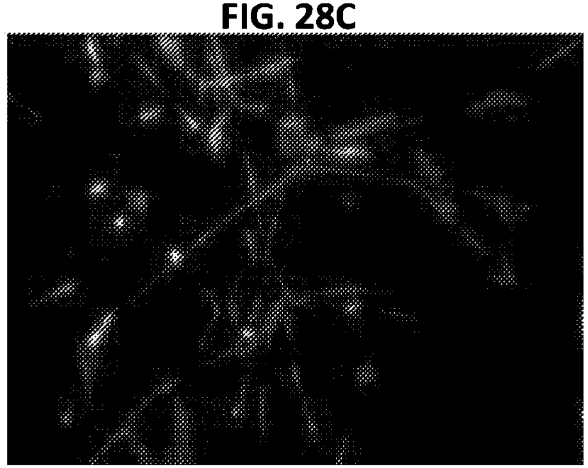

FIG. 28D

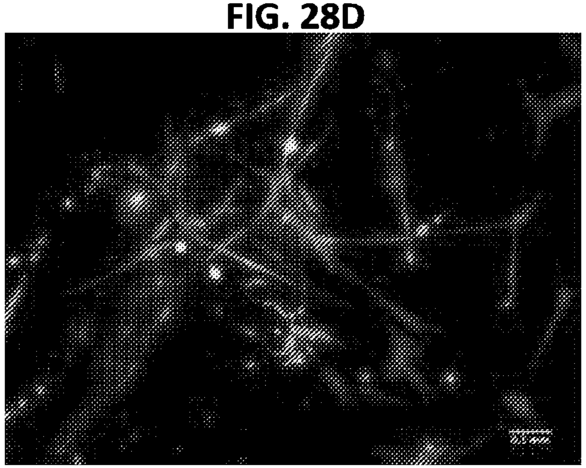

FIG. 29A

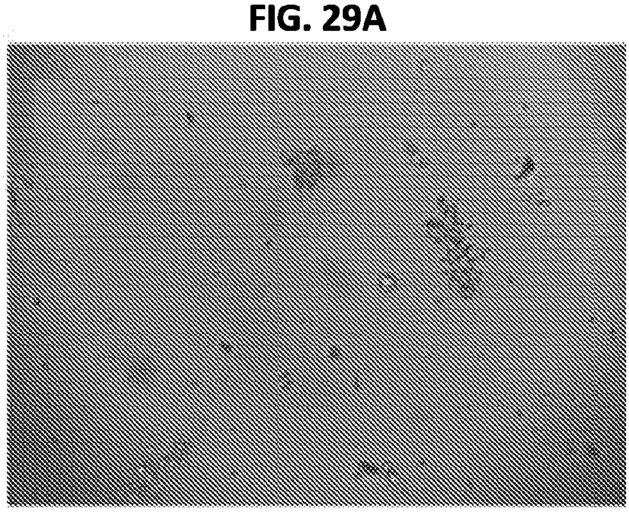

FIG. 29B

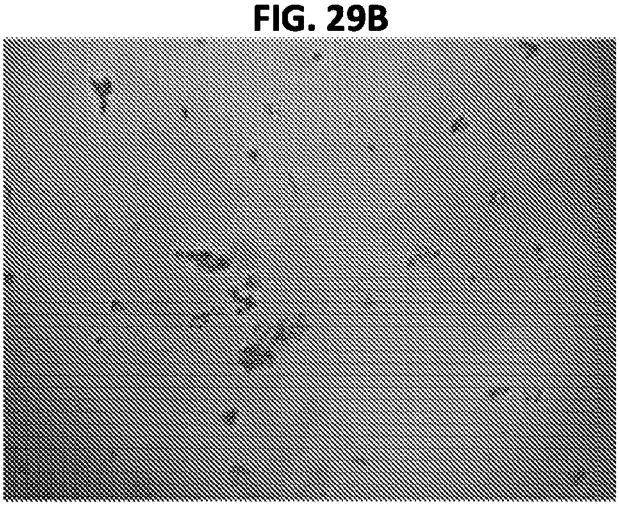

FIG. 29C

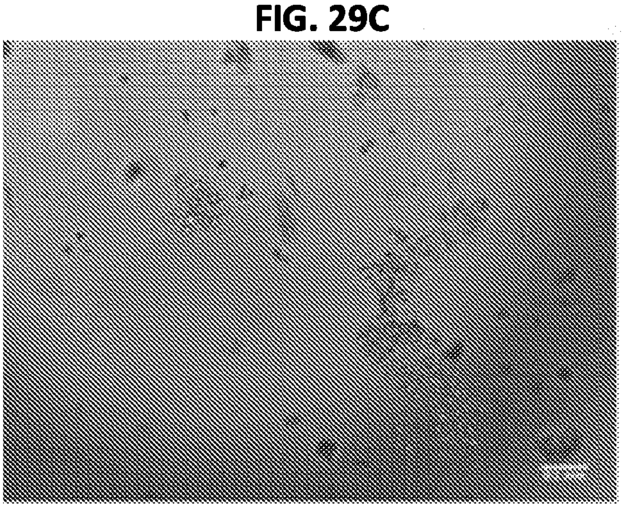

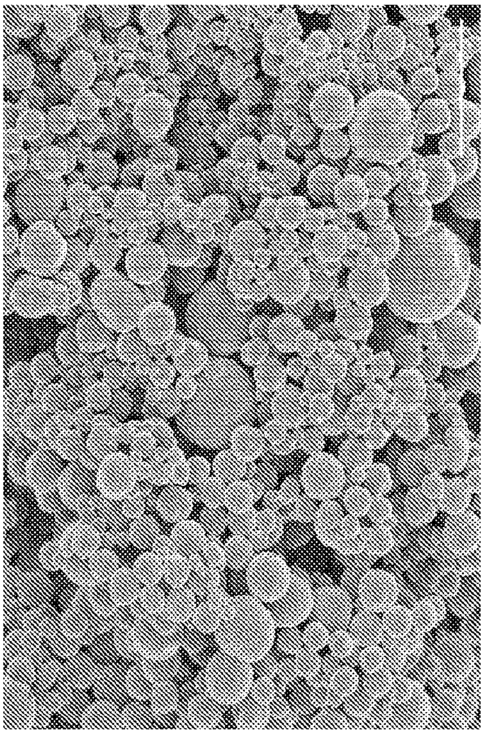

FIG. 30A

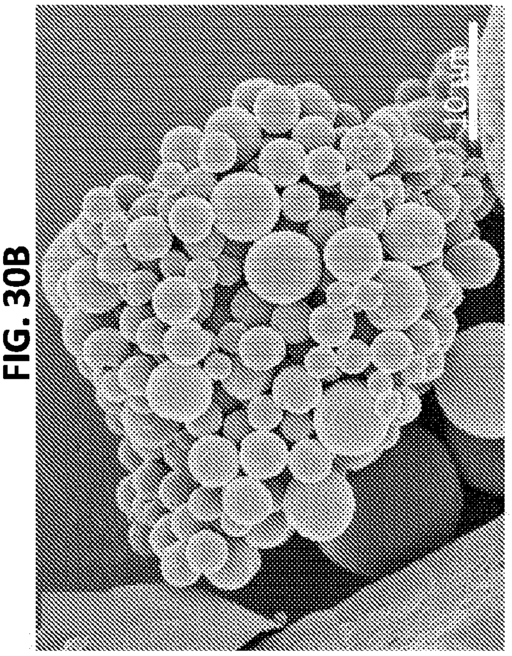

FIG. 30B

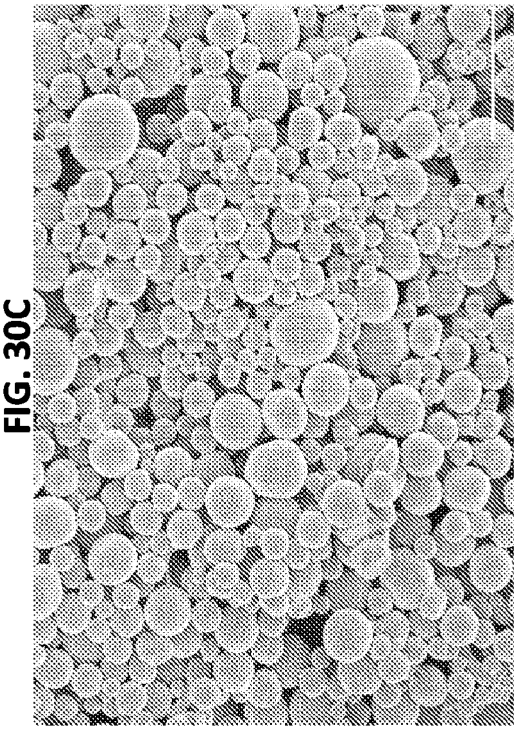

FIG. 30C

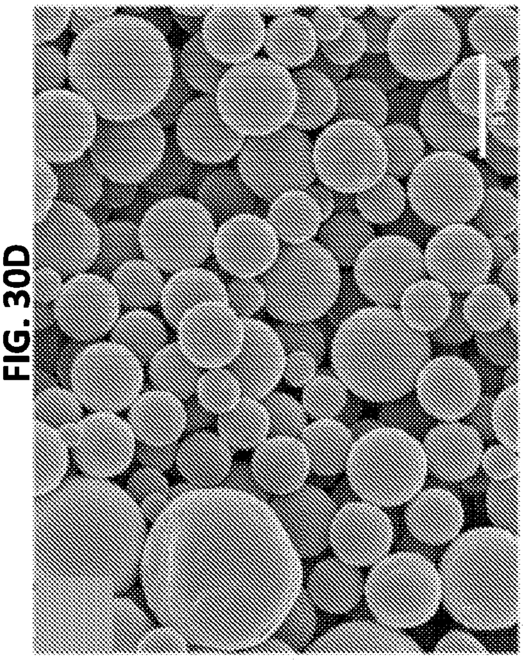

FIG. 30D

Soluble Puro/RA | Puro/RA microspheres

Day 15

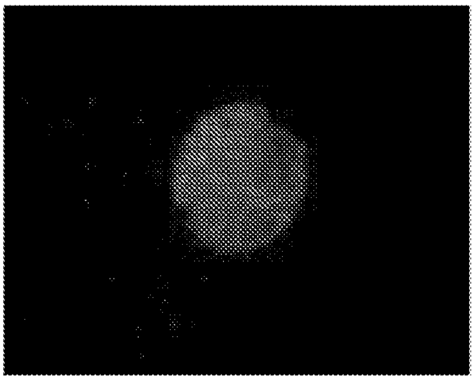

FIG. 31A

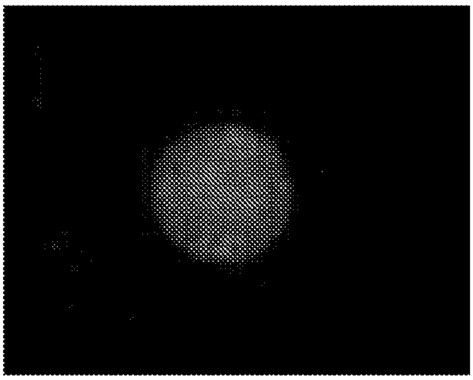

FIG. 31E

Day 28

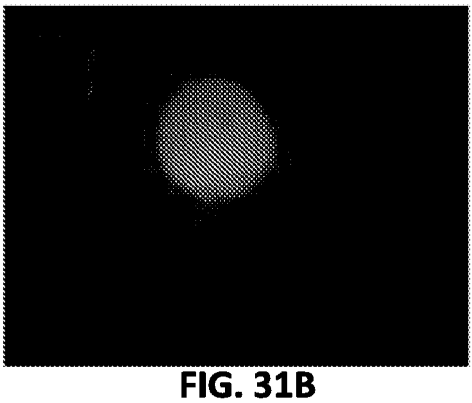

FIG. 31B

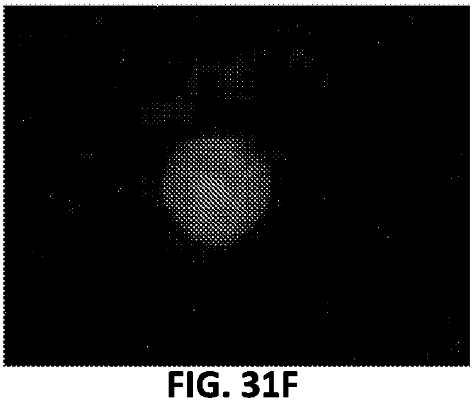

FIG. 31F

Day 35

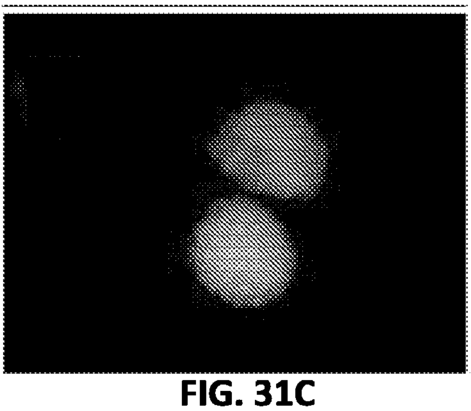

FIG. 31C

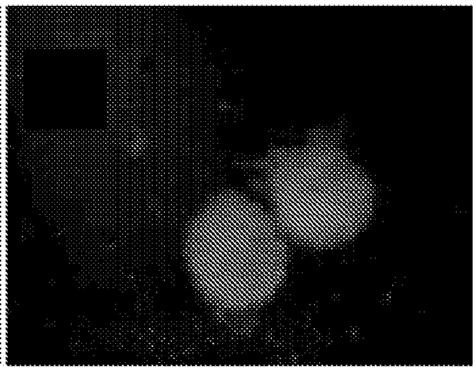

FIG. 31G

Day 56

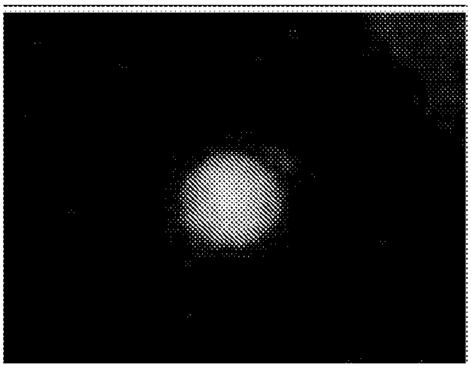

FIG. 31D

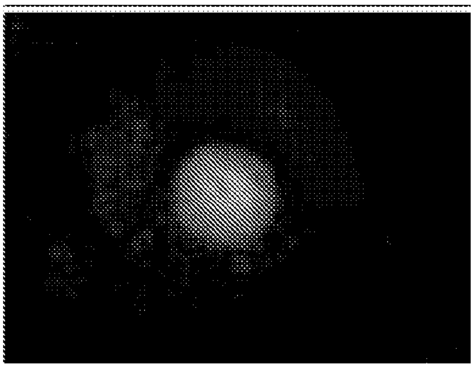

FIG. 31H

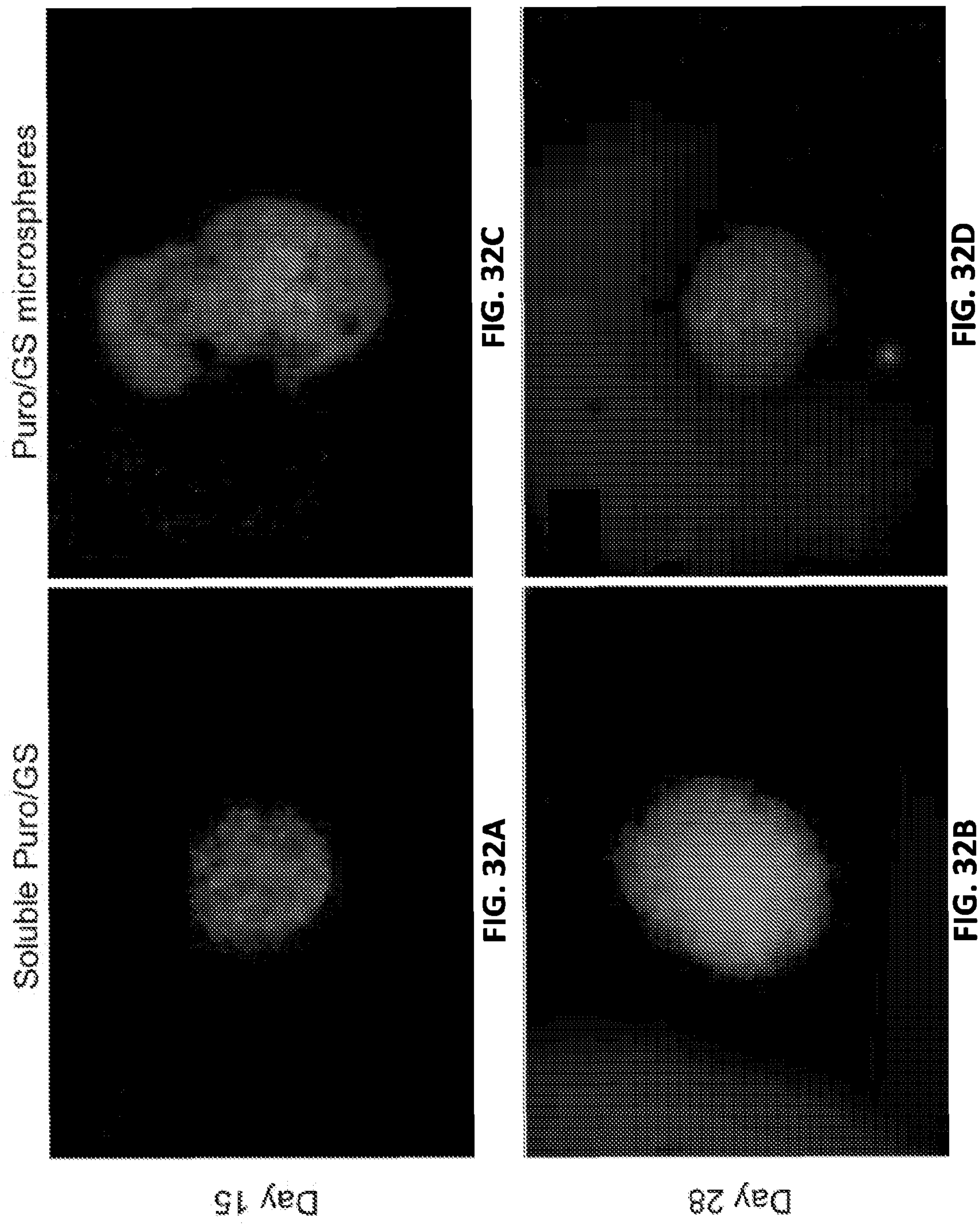


FIG. 32C

FIG. 32D

FIG. 32A

FIG. 32B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62634029
- US 2003175410 A1


### Non-patent literature cited in the description


- **KWON et al.** *J Endod,* 2015, vol. 41 (4), 501-7
- **KWON et al.** *J Endod.,* 2015, vol. 41 (4), 501-7
- **ISACSON et al.** *Stem Cells,* 2007, vol. 25 (9), 2257-2268
- **YUAN et al.** *Front Pharmacol,* 2017, vol. 8, 287
- **WALUS et al.** *Lab Chip,* 2016, vol. 16, 3351
- **DU, Z. W. et al.** BRAINPHYS™ products, STEMCELL™ TECHNOLOGIES, for example BRAINPHYS™ Neuronal Medium. *Nature communications,* 2015, vol. 6, 6626
- **DU, Z. W et al.** THERMOFISHER SCIENTIFIC. *Nature communications,* 2015, vol. 6, 6626
- **BRISCOE ; NOVITCH.** *Phil. Trans. R. Soc.,* 2008, vol. 363, 57-70
- **TAKAHASHI et al.** *Cell,* 2007, vol. 131, 861-872
- **KHAZAEI et al.** *Front Cell Dev Biol,* 2016, vol. 4, 152
- **BAHMAD et al.** *Front Mol Neurosci,* 2017, vol. 10, 50
- **NAGOSHI, H. ; OKANO.** *J Neurochem,* 2017, vol. 141, 848-860
- **LANCASTER et al.** *Nature,* 2013, vol. 501, 373-379
- **CONLEY et al.** *Int J Biochem Cell Biol,* 2004, vol. 36, 555-567
- **KUROSAWA.** *J Biosci Bioeng,* 2007, vol. 103, 389-398
- **B.Y. HU ; S.C. ZHANG.** *Nat Protoc,* 2009, vol. 4, 1295-1304
- **WILEM ; SAKIYAMA-ELBERT.** *Journal of Controlled Release,* 2015, vol. 213, 103-111
- **SILVA et al.** *Prog Neurobiol,* 2014, vol. 114, 25-57
- **P.K. STYS.** *Journal of Cerebral Blood Flow & Metabolism,* 1998, vol. 18, 2-25
- **YUAN ; HE.** *Neurosci Bull,* 2013, vol. 29, 421-435
- **ASSINCK et al.** *Nature Neuroscience,* 2017, vol. 20, 637
- **BENSADOUN et al.** *Journal of Controlled Release,* 2003, vol. 87, 107-115
- **NORI et al.** *Proc Natl Acad Sci USA,* 2011, vol. 108, 16825-16830
- **C.A. PRIEST et al.** *Regenerative Medicine,* 2015, vol. 10, 939-958
- Asterias Biotherapeutics, Asterias Biotherapeutics Announces Dosing of First Patient in New SCiSTAR Clinical Trial Cohort Testing AST-OPC1 in an Expanded Cervical Spinal Cord Injury Patient Population. *Asteriasbiotherapuetics.com,* 2016
- **SHIN et al.** *Stem Cells Dev.,* 2007, vol. 16, 131-141
- **B.S. JHA et al.** *Stem Cell Rev,* 2015, vol. 11, 194-204
- **N. BAHRAMI et al.** *Mol Neurobiol,* 2016
- **X. WU et al.** *Chem Biol,* 2004, vol. 11, 1229-1238
- **Y. YAN et al.** *Acta Biomater,* 2016, vol. 42, 114-126
- **K.D. MAGDALINI TSINTOU ; ALEXANDER MARCUS SEIFALIAN.** *Neural Regen Res,* 2015, vol. 10, 726-742
- **J. ZHANG et al.** *Cell Tissue Res,* 2016, vol. 366, 129-142
- **A. MONTGOMERY et al.** *Biomater Sci,* 2015, vol. 3, 401-413
- **C. LOPEZ-SERRANO et al.** *Cell Transplant,* 2016
- **C.S. AHUJA et al.** *Neuropathology,* 2009, vol. 29, 248-257
- **T.Z. HIROSHI NOMURA et al.** *Tissue engineering,* 2008, vol. 14, 649-665
- **J. EDGAR et al.** *Acta Biomater.,* 2017, vol. 51, 237-245
- **H. LI et al.** *Adv Healthc Mater,* 2016, vol. 5, 802-812
- **M. HIRAOKA et al.** *Bioconjugate chemistry,* 2009, vol. 20, 976-983
- **N.K. MOHTARAM et al.** *Journal of Biomedical Materials Research,* 2015, vol. 103, 2591-2601
- **C.D. JOHNSON et al.** *Cells Tissues Organs,* 2016, vol. 202, 116-135
- **A.M. BRATT-LEAL et al.** *Biotechnol Prog,* 2009, vol. 25, 43-51

- **A.P. VAN WINKLE et al.** *Cells Tissues Organs,* 2012, vol. 196, 34-47
- **A.M. BRATT-LEAL et al.** *Biomaterials,* 2013, vol. 34, 7227-7235
- **O. QUTACHI, K.M. ; SHAKESHEFF, L.D.** *J Control Release,* 2013, vol. 168, 18-27
- **MAKADIA ; SIEGEL.** *Polymers (Basel).,* 01 September 2011, vol. 3 (3), 1377-1397
- **J. GOMEZ et al.** *Cellular and Molecular Bioengineering,* 2015, vol. 8, 307-319
- **V.R. SINHA et al.** *Int J Pharm,* 2004, vol. 278, 1-23
- **AGBAY A et al.** *Biomed Mater,* 2018, vol. 13 (3), 034104
- **J.C. GOMEZ et al.** *Cellular and Molecular Bioengineering,* 2015, vol. 8, 307-319
- **A. AGBAY et al.** *Drug Delivery and Translational Research,* 2014, vol. 4, 159-170
- **M. ROBINSON et al.** *Biomarker Insights,* 2015, vol. 10, 1
- **B.-Y. HU et al.** *Proceedings of the National Academy of Sciences of the United States of America,* 2010, vol. 107, 4335-4340
- **B.-Y. HU ; S.-C. ZHANG.** *Nature protocols,* 2009, vol. 4, 1295-1304
- **S.Y.C. C. CHANGYONG ; N. JAE-WON.** *Polymer,* 2006, vol. 47, 4571
- **SHENOY ; AMIJI.** *Int J Pharm.,* 11 April 2005, vol. 293 (1-2), 261-70
- **R.L. CARPENEDO et al.** *J Biomed Mater Res A,* 2010, vol. 94, 466-475
- **S.R. HUTTON ; L.H. PEVNY.** *Developmental Biology,* 2011, vol. 352, 40-47
- **X. LIU et al.** *Cell Res,* 2008, vol. 18, 1177-1189
- **J. BRISCOE ; J. ERICSON.** *Current Opinion in Neurobiology,* 2001, vol. 11, 43-49
- **G. THIEL.** *Brain Pathology,* 1993, vol. 3, 87-95
- **DAUER, W. ; S. PRZEDBORSKI.** *Neuron,* 2003, vol. 39 (6), 889-909
- **HEGARTY et al.** *Dev Biol,* 2013, vol. 379 (2), 123-38
- **FAHN, S.** *Mov Disord,* 2015, vol. 30 (1), 4-18
- **JANKOVIC, J. ; L.G. AGUILAR.** *Neuropsychiatr Dis Treat,* 2008, vol. 4 (4), 743-57
- **MIYASAKI, J.M.** Movement Disorders. *Continuum (Minneap Minn),* 2016, vol. 22 (4), 1104-16
- **STOKER, T.B. ; R.A. BARKER.** *Regen Med,* 2016, vol. 11 (8), 777-786
- **WEINBERGER, L. et al.** *Nat Rev Mol Cell Biol,* 2016, vol. 17 (3), 155-69
- **TAKAHASHI, K. ; S. YAMANAKA.** *Cell,* 2006, vol. 126 (4), 663-76
- **TAKAHASHI, K. et al.** *Cell,* 2007, vol. 131 (5), 861-72
- **THOMSON, J.A. et al.** *Science,* 1998, vol. 282 (5391), 1145-7
- **CARPENEDO, R.L. et al.** *Biomaterials,* 2009, vol. 30 (13), 2507-15
- **LAKE, J. et al.** *J Cell Sci,* 2000, vol. 113, 555-66
- **MARTINEZ, S. et al.** *Development,* 1999, vol. 126 (6), 1189-200
- **CHI, C.L. et al.** *Development,* 2003, vol. 130 (12), 2633-44
- **FASANO, C.A. et al.** *Cell Stem Cell,* 2010, vol. 6 (4), 336-47
- **BASSON, M.A. et al.** *Development,* 2008, vol. 135 (5), 889-98
- **CROSSLEY et al.** *Nature,* 1996, vol. 380 (6569), 66-8
- **BRISCOE, J.** *Nat Chem Biol,* 2006, vol. 2 (1), 10-1
- **ARENAS et al.** *Development,* 2015, vol. 142 (11), 1918-36
- **KRIKS, S. et al.** *Nature,* 2011, vol. 480 (7378), 547-51
- **KIRKEBY, A. et al.** *Cell Rep,* 2012, vol. 1 (6), 703-14
- **GONZALEZ, R. et al.** *Sci Rep,* 2013, vol. 3, 1463
- **ROBINSON, M. et al.** *Biomarker Insights,* 2015, 61-70
- **BRATT-LEAL et al.** *Biotechnol Prog,* 2009, vol. 25 (1), 43-51
- **NIEDERREITHER, K. et al.** *Development,* 2000, vol. 127 (1), 75-85
- **CORSON, L.B. et al.** *Development,* 2003, vol. 130 (19), 4527-37
- **BRENNAN, J. et al.** *Nature,* 2001, vol. 411 (6840), 965-9
- **VAN WINKLE et al.** *Cells Tissues Organs,* 2012, vol. 196 (1), 34-47
- **SACHLOS, E. ; D.T. AUGUSTE.** *Biomaterials,* 2008, vol. 29 (34), 4471-80
- **BRATT-LEAL, A.M. et al.** *Biomaterials,* 2011, vol. 32 (1), 48-56
- **BRATT-LEAL, A.M. et al.** *Biomaterials,* 2013, vol. 34 (30), 7227-35
- **CARPENEDO et al.** *J Biomed Mater Res,* 2010, vol. 94 (2), 466-75
- **LIM, J.J. et al.** *Acta Biomater,* 2011, vol. 7 (3), 986-95
- **WANG, Y. et al.** *Acta Biomater,* 2016, vol. 29, 42-51
- **VARDE, N.K. ; D.W. PACK.** *Expert Opin Biol Ther,* 2004, vol. 4 (1), 35-51
- **YANG et al.** *Biomaterials,* 2001, vol. 22 (3), 231-241
- **COCCOLI et al.** *J Mater Sci Mater Med,* 2008, vol. 19 (4), 1703-11
- **SINHA, V.R. et al.** *Int J Pharm,* 2004, vol. 278 (1), 1-23
- **WOODRUFF ; HUTMACHER.** *Progress in Polymer Science,* 2010, vol. 35 (10), 1217-1256
- **GOMEZ, J.C. et al.** *Cellular and Molecular Bioengineering,* 2015, vol. 8 (3), 307-319

- **QUTACHI et al.** *J Control Release,* 2013, vol. 168 (1), 18-27
- **AGBAY et al.** *Drug Deliv Transl Res,* 2014, vol. 4 (2), 159-70
- **FREIBERG, S. ; X. ZHU.** *International Journal of Pharmaceutics,* 2004, vol. 282 (1-2), 1-18
- **GIUNCHEDI et al.** *J Microencapsul,* 1998, vol. 15 (2), 185-95
- **ZHAO.** *Adv Drug Deliv Rev,* 2013, vol. 65 (11-12), 1420-46
- **DUTTA, D. et al.** *J Biomed Mater Res,* 2015
- **JEONG, Y.I. et al.** *Int J Pharm,* 2003, vol. 259 (1-2), 79-91
- **BECK, L.R. et al.** *Adv Contracept,* 1985, vol. 1 (2), 119-29
- **FERREIRA, L. et al.** *Advanced Materials,* 2008, vol. 20 (12), 2285-2291
- **AGRAWAL, H. et al.** *J Pharm Biomed Anal,* 2004, vol. 36 (1), 23-31
- **ATYABI, S.M. et al.** *Cell Biochem Biophys,* 2016, vol. 74 (2), 181-90
- **CHUNG, T.W. et al.** *J Mater Sci Mater Med,* 2009, vol. 20 (1), 397-404
- **YILDIRIM, E.D. et al.** *Biofabrication,* 2010, vol. 2 (1), 014109
- **BRAMFELDT, H. ; P. VERMETTE.** *J Biomed Mater Res,* 2009, vol. 88 (2), 520-30
- **RECEK, N. et al.** *International Journal of Polymer Science,* 2016, 9
- **MOHTARAM, N.K. et al.** *Biomed Mater Res,* 2014
- **ABBASI, N. et al.** *J Biomed Mater Res,* 2015
- **JAKOBSSON, A. et al.** *Nanomedicine,* 2017, vol. 13 (4), 1563-1573
- **LI, B. et al.** *Biomaterials,* 2005, vol. 26 (24), 4956-63
- **WEN et al.** *Cell Adh Migr,* 2009, vol. 3 (1), 107-17
- **HONG, S. ; M.R. SONG.** *PLoS One,* 2014, vol. 9 (1), e86851
- **CHENG, P.Y. et al.** *PLoS One,* 2011, vol. 6 (7), e22018
- **FUKUDA et al.** *Cell Death Differ,* 2004, vol. 11 (2), 196-202
- **KAGEYAMA et al.** *Crit Rev Neurobiol,* 1995, vol. 9 (2-3), 177-88
- **CASTELO-BRANCO et al.** *J Cell Sci,* 2004, vol. 117, 5731-7
- **CASTELO-BRANCO et al.** *Proc Natl Acad Sci USA,* 2003, vol. 100 (22), 12747-52
- **BUETTNER et al.** *Clin Cancer Res,* 2002, vol. 8 (4), 945-54
- **AHN et al.** *Cancer Res,* 2008, vol. 68 (11), 4406-15
- **HOSPODIUK et al.** *Biotechnology Advances,* 2017, vol. 35 (2), 217-239
- **ABELSETH, L.** Cytotoxic response of neural progenitor cells in 3D fibrin scaffolds. 2017
- **HU, B.-Y. ; S.-C. ZHANG.** *Nature protocols,* 2009, vol. 4 (9), 1295-1304